# EUROPÄISCHE PATENTSCHRIFT

(11) **EP 3 803 998 B1**
(45) Veröffentlichungstag und Bekanntmachung des Hinweises auf die Patenterteilung: **30.07.2025**
(21) Anmeldenummer: 19729473.9
(22) Anmeldetag: 03.06.2019
(51) Int. Cl.: H10K 71/12, H10K 85/60, C07D 209/80, C07D 209/96, C07D 403/04, C07D 403/14, C07D 405/14, C07D 495/04, H10K 85/30, H10K 50/11

(54) **ORGANISCHE ELEKTROLUMINESZENZVORRICHTUNGEN**
ORGANIC ELECTROLUMINESCENCE DEVICES
DISPOSITIFS ELECTROLUMINESCENTS ORGANIQUES

(30) Priorität: 07.06.2018 EP 18176496
(43) Veröffentlichungstag der Anmeldung: 14.04.2021
(73) Patentinhaber: Merck Patent GmbH, 64293 Darmstadt (DE)
(72) Erfinder: PARHAM, Amir, 60486 FRANKFURT AM MAIN (DE); KROEBER, Jonas, 60311 FRANKFURT AM MAIN (DE); ENGELHART, Jens, 64285 DARMSTADT (DE); JATSCH, Anja, 60489 FRANKFURT AM MAIN (DE); EICKHOFF, Christian, 68259 MANNHEIM (DE); EHRENREICH, Christian, 64285 DARMSTADT (DE); JOOSTEN, Dominik, 64372 OBER-RAMSTADT (DE)
(74) Vertreter: Merck Patent Association
(86) Internationale Anmeldenummer: PCT/EP2019/064249
(87) Internationale Veröffentlichungsnummer: WO 2019/233904

(56) Entgegenhaltungen:
- KR-A- 20180 013 713
- US-A1- 2015 214 492
- US-A1- 2016 233 442

## Beschreibung

Die vorliegende Erfindung betrifft organische Elektrolumineszenzvorrichtungen, enthaltend Indenocarbazolderivate.

In organischen Elektrolumineszenzvorrichtungen (OLEDs) werden als emittierende Materialien häufig phosphoreszierende metallorganische Komplexe eingesetzt. Die Eigenschaften phosphoreszierender OLEDs werden nicht nur von den eingesetzten Triplettemittern bestimmt. Hier sind auch die anderen verwendeten Materialien, insbesondere die Matrixmaterialien, von besonderer Bedeutung. Verbesserungen dieser Materialien können somit auch zu deutlichen Verbesserungen der OLED-Eigenschaften führen.

Aufgabe der vorliegenden Erfindung ist die Bereitstellung phosphoreszierender organischer Elektrolumineszenzvorrichtungen, welche verbesserte Eigenschaften aufweisen, insbesondere eine verbesserte Lebensdauer bei gleichzeitig guter Effizienz und geringer Betriebsspannung. Eine weitere Aufgabe der Erfindung ist die Bereitstellung phosphoreszierender OLEDs mit hoher Lebensdauer, guter Effizienz und geringer Betriebsspannung, die nur ein Matrixmaterial und nicht eine Mischung aus zwei Matrixmaterialien enthalten. Der Vorteil einer solchen OLED ist die vereinfachte Prozessierung bei deren Herstellung.

Überraschend wurde gefunden, dass diese Aufgabe gelöst wird durch die Verwendung der unten näher beschriebenen Indenocarbazolderivate als Matrixmaterial für phosphoreszierende Emitter. Organische Elektrolumineszenzvorrichtungen, welche derartige Verbindungen als Matrixmaterial für phosphoreszierende Emitter enthalten, sind daher der Gegenstand der vorliegenden Erfindung. Aus WO 2010/136109 (A1), KR20180013713 (A), und US2015/214492 (A1) sind Indenocarbazolderi-vate als Matrixmaterialien für phosphoreszierende Emitter bekannt.

Matrixmaterialien gemäß der vorliegenden Erfindung sind nicht offenbart. Gegenstand der vorliegenden Erfindung ist eine organische Elektrolumineszenzvorrichtung enthaltend Anode, Kathode und mindestens eine emittierende Schicht, die mindestens eine phosphoreszierende Verbindung enthält, dadurch gekennzeichnet, dass die emittierende Schicht mindestens eine Verbindung gemäß Formel (1) enthält, 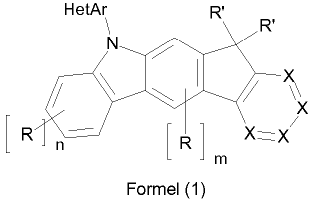 wobei für die verwendeten Symbole und Indizes gilt:
- X: zwei benachbarte X stehen für eine Gruppe der folgenden Formel (2), und die beiden anderen X stehen für CR, 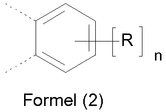 wobei die beiden gestrichelten Bindungen die Verknüpfung dieser Gruppe darstellen;

HetAr ist eine Gruppe gemäß einer der Formeln (HetAr-1d), (HetAr-2a), (HetAr-3a), (HetAr-4a), (HetAr-5a), (HetAr-6a), (HetAr-7a) und (HetAr-8c),

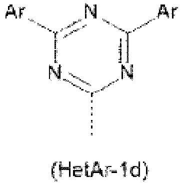

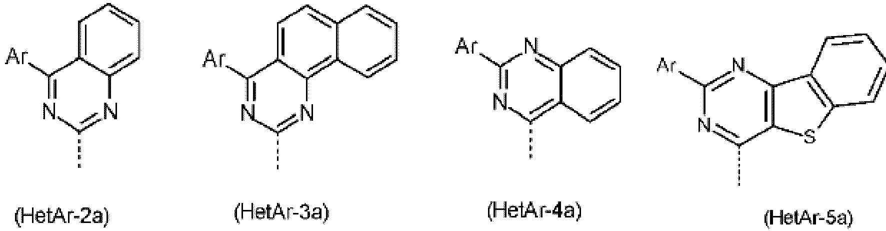

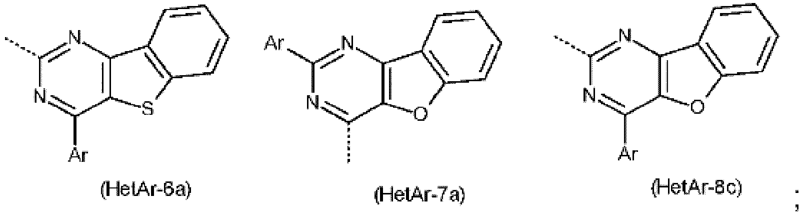

Ar ist gleich oder verschieden bei jedem Auftreten ein aromatisches oder heteroaromatisches Ringsystem mit 5 bis 40 aromatischen Ringatomen, welches mit einem oder mehreren Resten R¹ substituiert sein kann;
- R: ist bei jedem Auftreten gleich oder verschieden H, D, F Cl, Br, I, N(R¹)₂, N(Ar')₂, CN, NO₂, OR¹, SR¹, COOR¹, C(=O)N(R¹)₂, Si(R¹)₃, B(OR¹)₂, C(=O)R¹, P(=O)(R¹)₂, S(=O)R¹, S(=O)₂R¹, OSO₂R¹, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹ substituiert sein kann und wobei eine oder mehrere nicht benachbarte CH₂-Gruppen durch Si(R¹)₂, C=O, NR¹, O, S oder CONR¹ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹ substituiert sein kann;
- R': ist bei jedem Auftreten gleich oder verschieden eine geradkettige Alkylgruppe mit 1 bis 20 C-Atomen oder eine verzweigte oder cyclische Alkylgruppe mit 3 bis 20 C-Atomen, wobei die geradkettige, verzweigte oder cyclische Alkylgruppe jeweils mit einem oder mehreren Resten R¹ substituiert sein kann und wobei eine oder mehrere nicht benachbarte CH₂-Gruppen durch O ersetzt sein können, oder ein aromatisches oder heteroaromatisches Ringsystem mit 5 bis 40 aromatischen Ringatomen, das jeweils durch einen oder mehrere Reste R¹ substituiert sein kann; dabei können zwei Reste R' auch miteinander ein aromatisches, heteroaromatisches, aliphatisches oder heteroaliphatisches Ringsystem bilden;
- Ar': ist bei jedem Auftreten gleich oder verschieden ein aromatisches oder heteroaromatisches Ringsystem mit 5 bis 40 aromatischen Ringatomen, welches mit einem oder mehreren Resten R¹ substituiert sein kann;
- R¹: ist bei jedem Auftreten gleich oder verschieden H, D, F, Cl, Br, I, N(R²)₂, CN, NO₂, OR², SR², Si(R²)₃, B(OR²)₂, C(=O)R², P(=O)(R²)₂, S(=O)R², S(=O)₂R², OSO₂R², eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R² substituiert sein kann, wobei eine oder mehrere nicht benachbarte CH₂-Gruppen durch Si(R²)₂, C=O, NR², O, S oder CONR² ersetzt sein können, oder ein aromatisches oder heteroaromatisches Ringsystem mit 5 bis 40 aromatischen Ringatomen, das jeweils durch einen oder mehrere Reste R² substituiert sein kann;
- R²: ist bei jedem Auftreten gleich oder verschieden H, D, F oder ein aliphatischer, aromatischer oder heteroaromatischer organischer Rest, insbesondere ein Kohlenwasserstoffrest, mit 1 bis 20 C-Atomen, in dem auch ein oder mehrere H-Atome durch F ersetzt sein können;
- m: ist 0, 1 oder 2;
- n: ist bei jedem Auftreten gleich oder verschieden 0 oder 1.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Eine elektronenarme Heteroarylgruppe im Sinne der vorliegenden Erfindung ist eine Heteroarylgruppe, die mindestens einen heteroaromatischen Sechsring mit mindestens zwei Stickstoffatomen oder mindestens einen heteroaromatischen Fünfring mit mindestens zwei Heteroatomen aufweist, wobei mindestens ein Heteroatom in dem Fünfring Stickstoff ist und das andere Heteroatom in dem Fünfring ein substituierter Stickstoff oder Sauerstoff oder Schwefel ist. An diesen heteroaromatischen Fünf- bzw. Sechsring können noch weitere aromatische oder heteroaromatische Gruppen ankondensiert sein. In einer bevorzugten Ausführungsform der Erfindung enthält die elektronenarme Heteroarylgruppe mindestens einen heteroaromatischen Sechsring mit mindestens zwei Stickstoffatomen. Beispiele für elektronenarme Heteroarylgruppen sind Pyrimidin, Pyrazin, Pyridazin, Triazin, Chinazolin, Chinoxalin, Benzochinazolin oder Benzimidazol. Weitere elektronenarme Heteroarylgruppen werden in der folgenden Beschreibung genauer ausgeführt.

Ein aromatisches Ringsystem im Sinne dieser Erfindung enthält 6 bis 60 C-Atome im Ringsystem, bevorzugt 6 bis 40 C-Atome. Ein heteroaromatisches Ringsystem im Sinne dieser Erfindung enthält 2 bis 60 C-Atome, bevorzugt 2 bis 40 C-Atome, und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Bevorzugt ist das aromatische Ringsystem gewählt aus Fluoren, 9,9'-Spirobifluoren, 9,9-Diarylamin oder Gruppen, in denen zwei oder mehr Aryl- und/oder Heteroarylgruppen durch Einfachbindungen miteinander verknüpft sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 bis 60 aromatischen Ringatomen, welches noch jeweils mit den oben genannten Resten substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombinationen dieser Systeme.

Wenn zwei Reste R' miteinander ein Ringsystem bilden, so kann dieses mono- oder polycyclisch sein. Dabei sind die Reste, die miteinander ein Ringsystem bilden, bevorzugt benachbart, d. h. dass diese Reste an dasselbe Kohlenstoffatom oder an Kohlenstoffatome gebunden sind, die direkt aneinander gebunden sind. Wenn zwei Reste R' miteinander ein Ringsystem bilden, entsteht dadurch ein Spirosystem.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

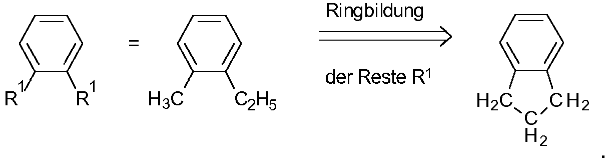

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

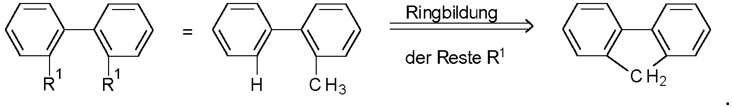

Je nach Position, in der die Gruppe der Formel (2) ankondensiert ist, umfasst die erfindungsgemäße organische Elektrolumineszenzvorrichtung mindestens eine Verbindung gemäß einer der folgenden Formeln (3), (4) oder (5), 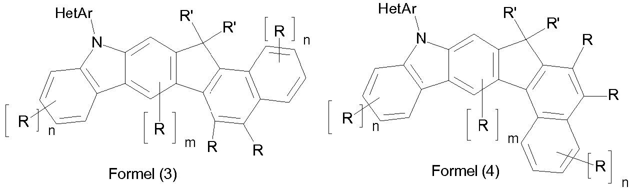 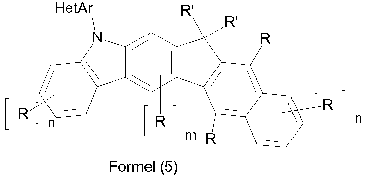 wobei die verwendeten Symbole und Indizes die oben genannten Bedeutungen aufweisen.

In einer bevorzugten Ausführungsform der Erfindung sind die Verbindungen der Formeln (3), (4) und (5) gewählt aus den Verbindungen der folgenden Formeln (3a-1), (3a-2), (4a-1), (4a-2), (5a-1) und (5a-2), 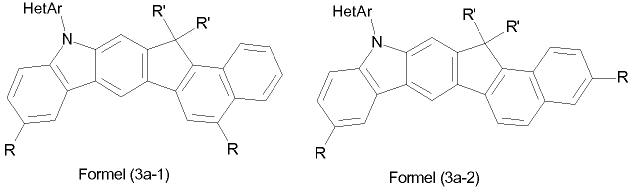 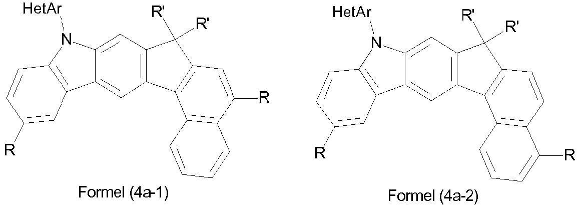 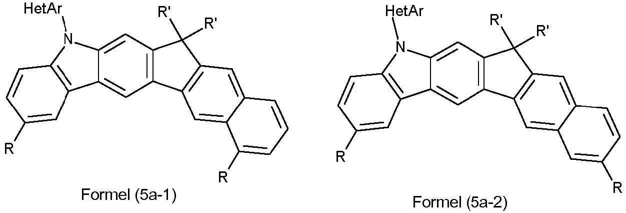 wobei HetAr, R und R' die oben genannten Bedeutungen aufweisen.

Dabei steht der Rest R an der Gruppe der Formel (2) für bevorzugt H, so dass es sich bei den Verbindungen der Formeln (3), (4) und (5) um Verbindungen der folgenden Formeln (3b), (4b) bzw. (5b) handelt, 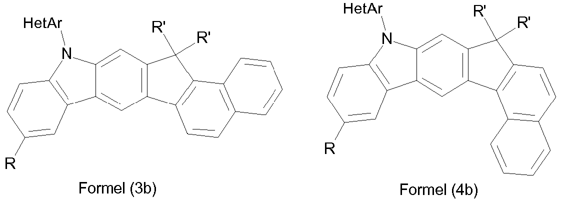 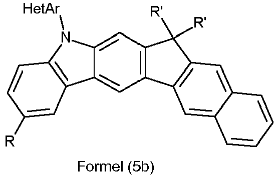 wobei HetAr, R und R' die oben genannten Bedeutungen aufweisen.

In einer bevorzugten Ausführungsform der Erfindung steht R in den Formeln (3b), (4b) und (5b) für Carbazolyl, welches am N-Atom mit einem Rest R¹ substituiert ist, wobei R¹ für ein aromatisches oder heteroaromatisches Ringsystem steht. Dabei ist der Carbazolylrest bevorzugt über die 3-Position gebunden. In einer weiteren bevorzugten Ausführungsform der Erfindung steht der Rest R in den Formeln (3b), (4b) und (5b) für H, so dass es sich bevorzugt um Verbindungen der folgenden Formeln (3c), (4c) bzw. (5c) handelt, 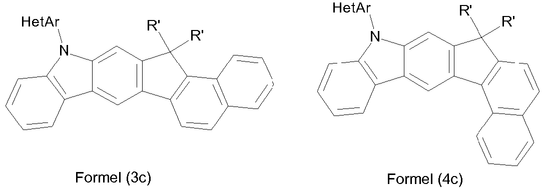 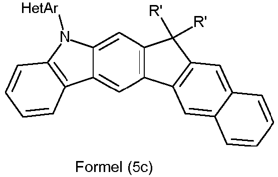 wobei HetAr und R' die oben genannten Bedeutungen aufweisen.

Im Folgenden werden bevorzugte Gruppen HetAr beschrieben.

Besonders bevorzugt sind die Gruppen (HetAr-1d) und (HetAr-2a), insbesondere (HetAr-2a).

Geeignete aromatische bzw. heteroaromatische Ringsysteme Ar sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R¹ substituiert sein können.

Dabei sind die Gruppen Ar unabhängig voneinander bevorzugt gewählt aus den Gruppen der folgenden Formeln Ar-1 bis Ar-75, 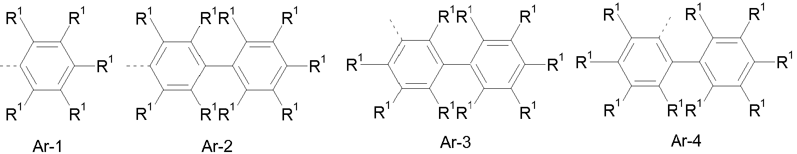 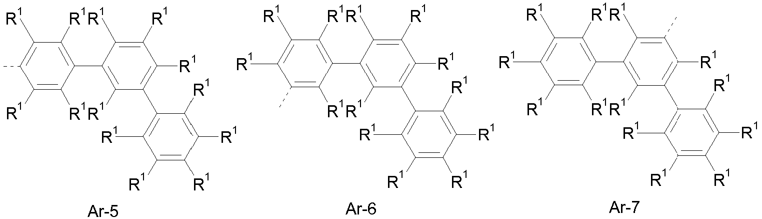 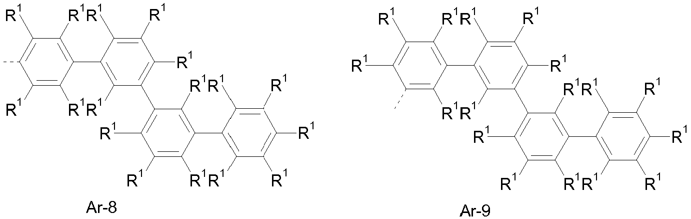 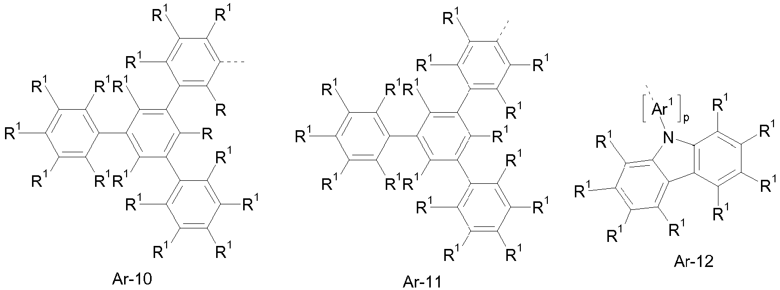 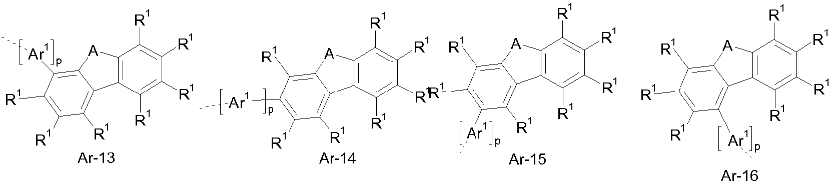 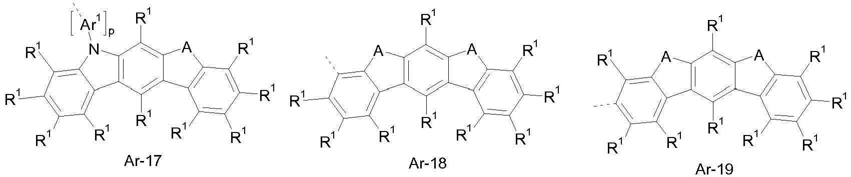 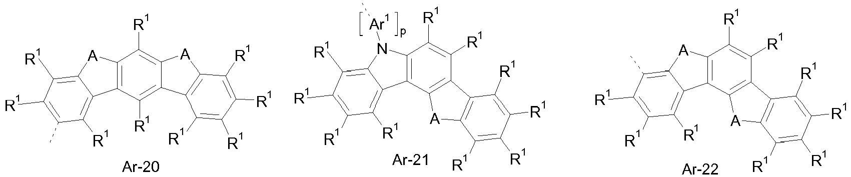 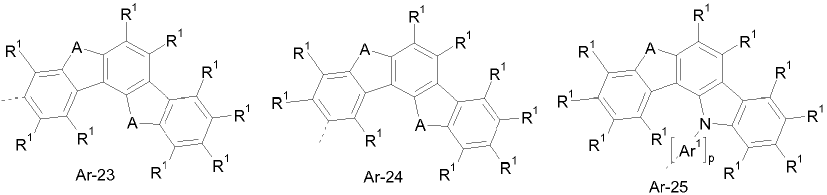 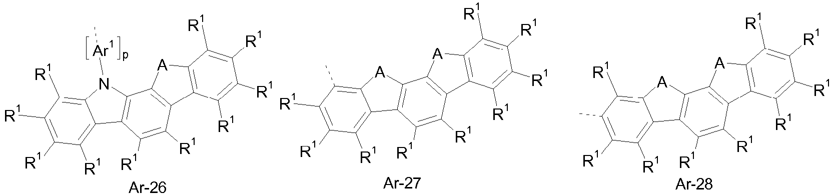 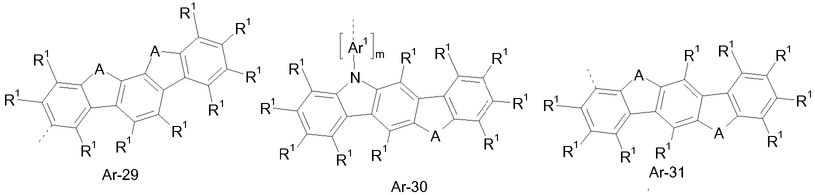 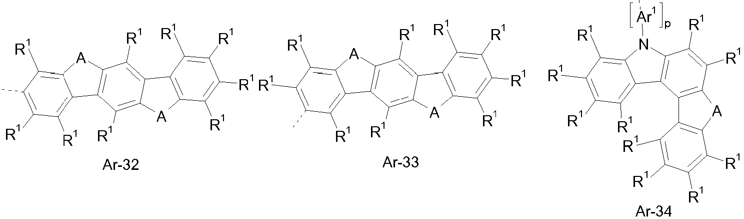 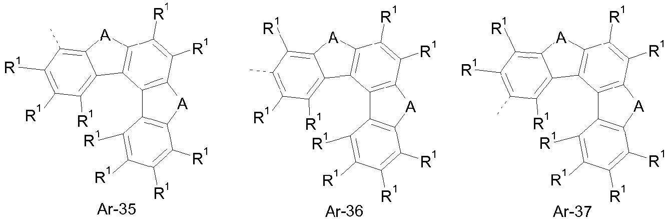 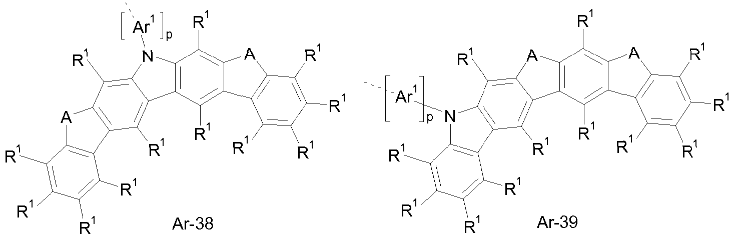 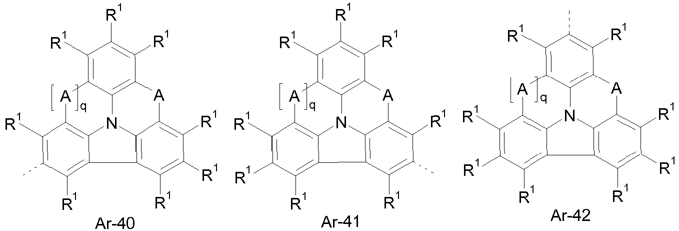 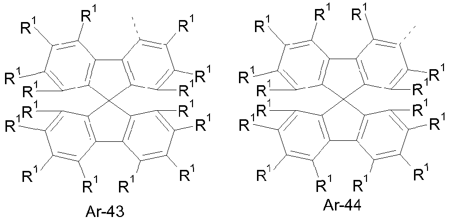 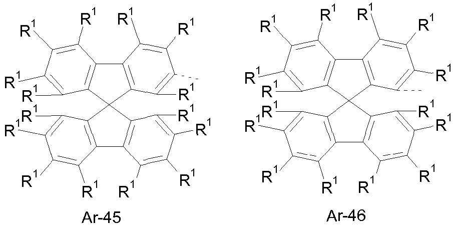 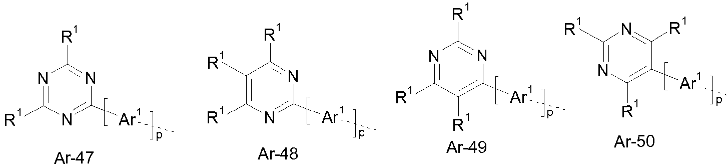 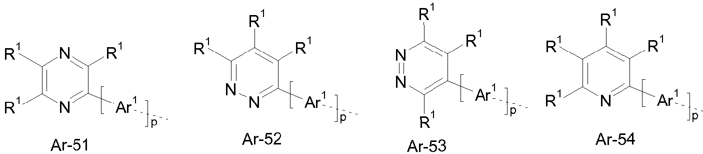 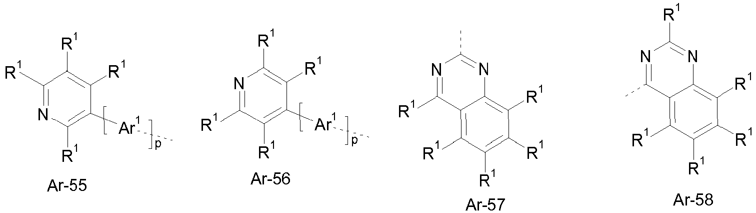 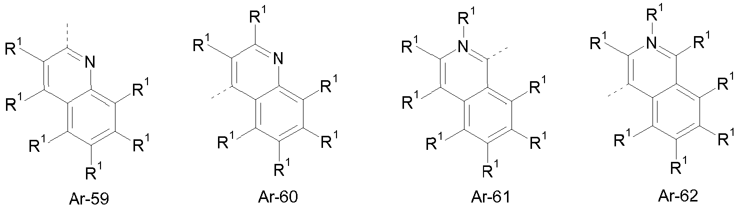 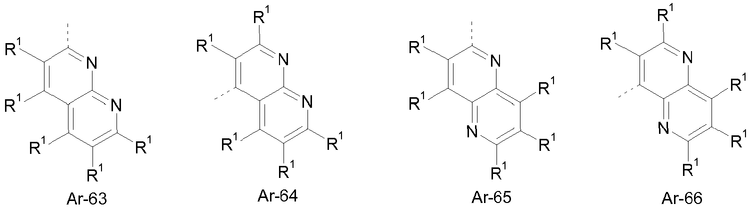 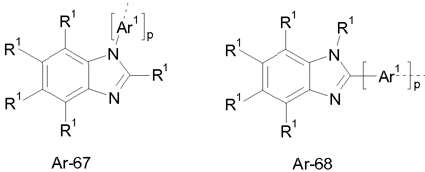 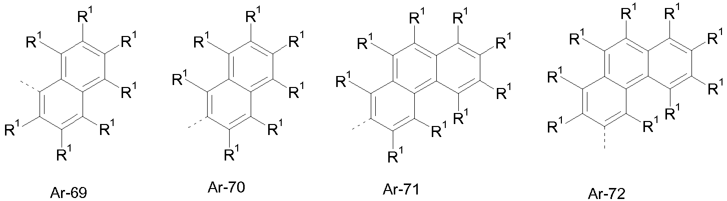 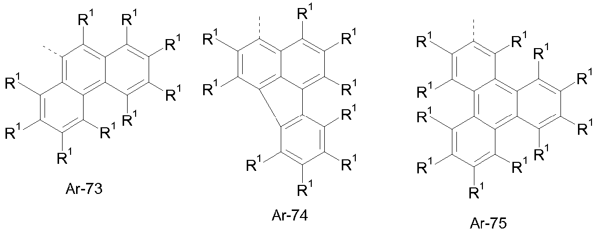 wobei R¹ die oben genannten Bedeutungen aufweist, die gestrichelte Bindung die Bindung an HetAr darstellt und weiterhin gilt:
- Ar¹: ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R¹ substituiert sein kann;
- A: ist bei jedem Auftreten gleich oder verschieden C(R¹)₂, NR¹, O oder S;
- p: ist 0 oder 1, wobei p = 0 bedeutet, dass die Gruppe Ar¹ nicht vorhanden ist und dass die entsprechende aromatische bzw. heteroaromatische Gruppe direkt an HetAr gebunden ist;
- q: ist 0 oder 1, wobei q = 0 bedeutet, dass an dieser Position keine Gruppe A gebunden ist und an die entsprechenden Kohlenstoffatome statt dessen Reste R¹ gebunden sind.

Wenn die oben genannten Gruppen für Ar mehrere Gruppen A aufweisen, so kommen hierfür alle Kombinationen aus der Definition von A in Frage. Bevorzugte Ausführungsformen sind dann solche, in denen eine Gruppe A für NR¹ und die andere Gruppe A für C(R¹)₂ steht oder in denen beide Gruppen A für NR¹ stehen oder in denen beide Gruppen A für O stehen.

Wenn A für NR¹ steht, steht der Substituent R¹, der an das Stickstoffatom gebunden ist, bevorzugt für ein aromatisches oder heteroaromatisches Ringsystem mit 5 bis 24 aromatischen Ringatomen, welches auch durch einen oder mehrere Reste R² substituiert sein kann. In einer besonders bevorzugten Ausführungsform steht dieser Substituent R¹ gleich oder verschieden bei jedem Auftreten für ein aromatisches oder heteroaromatisches Ringsystem mit 6 bis 24 aromatischen Ringatomen, insbesondere mit 6 bis 18 aromatischen Ringatomen, welches keine kondensierten Arylgruppen aufweist und welches keine kondensierten Heteroarylgruppen, in denen zwei oder mehr aromatische bzw. heteroaromatische 6-RingGruppen direkt aneinander ankondensiert sind, aufweist, und welches jeweils auch durch einen oder mehrere Reste R² substituiert sein kann. Bevorzugt sind Phenyl, Biphenyl, Terphenyl und Quaterphenyl mit Verknüpfungsmustern, wie vorne für Ar-1 bis Ar-11 aufgeführt, wobei diese Strukturen statt durch R¹ durch einen oder mehrere Reste R² substituiert sein können, bevorzugt aber unsubstituiert sind. Bevorzugt sind weiterhin Triazin, Pyrimidin und Chinazolin, wie vorne für Ar-47 bis Ar-50, Ar-57 und Ar-58 aufgeführt, wobei diese Strukturen statt durch R¹ durch einen oder mehrere Reste R² substituiert sein können.

Wenn A für C(R¹)₂ steht, stehen die Substituenten R¹, die an dieses Kohlenstoffatom gebunden sind, bevorzugt gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24 aromatischen Ringatomen, welches auch durch einen oder mehrere Reste R² substituiert sein kann. Ganz besonders bevorzugt steht R¹ für eine Methylgruppe oder für eine Phenylgruppe. Dabei können die Reste R¹ auch miteinander ein Ringsystem bilden, was zu einem Spirosystem führt.

Im Folgenden werden bevorzugte Substituenten R und R' beschrieben, die an das Grundgerüst des Benzoindenocarbazols gebunden sind.

In einer bevorzugten Ausführungsform der Erfindung ist R gleich oder verschieden bei jedem Auftreten ausgewählt aus der Gruppe bestehend aus H, D, einem aromatischen oder heteroaromatischen Ringsystem mit 6 bis 30 aromatischen Ringatomen, welches mit einem oder mehreren Resten R¹ substituiert sein kann, oder einer Gruppe N(Ar')₂. Besonders bevorzugt ist R gleich oder verschieden bei jedem Auftreten ausgewählt aus der Gruppe bestehend aus H oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R¹ substituiert sein kann.

Geeignete aromatische bzw. heteroaromatische Ringsystem R bzw. Ar' sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3- oder 4-Position verknüpft sein kann oder welches für R auch über das Stickstoffatom verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R¹ substituiert sein können. Besonders bevorzugt sind die oben aufgeführten Strukturen Ar-1 bis Ar-75.

Weitere geeignete Gruppen R sind Gruppen der Formel -Ar⁴-N(Ar²)(Ar³), wobei Ar², Ar³ und Ar⁴ gleich oder verschieden bei jedem Auftreten für ein aromatisches oder heteroaromatisches Ringsystem mit 5 bis 24 aromatischen Ringatomen stehen, welches jeweils mit einem oder mehreren Resten R¹ substituiert sein kann. Dabei beträgt die Gesamtzahl der aromatischen Ringatome von Ar², Ar³ und Ar⁴ maximal 60 und bevorzugt maximal 40.

Dabei können Ar⁴ und Ar² miteinander und/oder Ar² und Ar³ miteinander auch durch eine Gruppe ausgewählt aus C(R¹)₂, NR¹, O oder S verbunden sein. Bevorzugt erfolgt die Verknüpfung von Ar⁴ und Ar² miteinander bzw. von Ar² und Ar³ miteinander jeweils ortho zur Position der Verknüpfung mit dem Stickstoffatom. In einer weiteren Ausführungsform der Erfindung sind keine der Gruppen Ar², Ar³ bzw. Ar⁴ miteinander verbunden.

Bevorzugt ist Ar⁴ ein aromatisches oder heteroaromatisches Ringsystem mit 6 bis 24 aromatischen Ringatomen, bevorzugt mit 6 bis 12 aromatischen Ringatomen, das jeweils mit einem oder mehreren Resten R¹ substituiert sein kann. Besonders bevorzugt ist Ar⁴ ausgewählt aus der Gruppe bestehend aus ortho-, meta- oder para-Phenylen oder ortho-, meta- oder para-Biphenyl, welche jeweils durch einen oder mehrere Reste R¹ substituiert sein können, bevorzugt aber unsubstituiert sind. Ganz besonders bevorzugt ist Ar⁴ eine unsubstituierte Phenylengruppe.

Bevorzugt sind Ar² und Ar³ gleich oder verschieden bei jedem Auftreten ein aromatisches oder heteroaromatisches Ringsystem mit 6 bis 24 aromatischen Ringatomen, das jeweils mit einem oder mehreren Resten R¹ substituiert sein kann. Besonders bevorzugte Gruppen Ar² bzw. Ar³ sind gleich oder verschieden bei jedem Auftreten ausgewählt aus der Gruppe bestehend aus Benzol, ortho-, meta- oder para-Biphenyl, ortho-, meta-, para- oder verzweigtem Terphenyl, ortho-, meta-, para- oder verzweigtem Quaterphenyl, 1-, 2-, 3- oder 4-Fluorenyl, 1-, 2-, 3- oder 4-Spirobifluorenyl, 1- oder 2-Naphthyl, Indol, Benzofuran, Benzothiophen, 1-, 2-, 3- oder 4-Carbazol, 1-, 2-, 3- oder 4-Dibenzofuran, 1-, 2-, 3- oder 4-Dibenzothiophen, Indenocarbazol, Indolocarbazol, 2-, 3- oder 4-Pyridin, 2-, 4- oder 5-Pyrimidin, Pyrazin, Pyridazin, Triazin, Phenanthren oder Triphenylen, welche jeweils mit einem oder mehreren Resten R¹ substituiert sein können. Ganz besonders bevorzugt sind Ar² und Ar³ gleich oder verschieden bei jedem Auftreten ausgewählt aus der Gruppe bestehend aus Benzol, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, insbesondere 1-, 2-, 3- oder 4-Fluoren, oder Spirobifluoren, insbesondere 1-, 2-, 3- oder 4-Spirobifluoren.

In einer bevorzugten Ausführungsform der Erfindung ist R' bei jedem Auftreten gleich oder verschieden ausgewählt aus der Gruppe bestehend aus einer geradkettigen Alkylgruppe mit 1 bis 6 C-Atomen oder einer cyclischen Alkylgruppe mit 3 bis 6 C-Atomen, wobei die Alkylgruppe jeweils mit einem oder mehreren Resten R¹ substituiert sein kann, oder einem aromatischen oder heteroaromatischen Ringsystem mit 6 bis 24 aromatischen Ringatomen, das jeweils durch einen oder mehrere Reste R¹ substituiert sein kann; dabei können zwei Reste R' auch miteinander ein Ringsystem bilden, wodurch ein Spirosystem entsteht. Besonders bevorzugt ist R' bei jedem Auftreten gleich oder verschieden ausgewählt aus der Gruppe bestehend aus einer geradkettigen Alkylgruppe mit 1, 2, 3 oder 4 C-Atomen oder einer verzweigten oder cyclischen Alkylgruppe mit 3 bis 6 C-Atomen, wobei die Alkylgruppe jeweils mit einem oder mehreren Resten R¹ substituiert sein kann, bevorzugt aber unsubstituiert ist, oder einem aromatischen Ringsystem mit 6 bis 12 aromatischen Ringatomen, insbesondere mit 6 aromatischen Ringatomen, das jeweils durch einen oder mehrere, bevorzugt nicht-aromatische Reste R¹ substituiert sein kann, bevorzugt aber unsubstituiert ist; dabei können zwei Reste R' miteinander ein Ringsystem bilden. Wenn zwei Reste R' miteinander ein Ringsystem bilden, so handelt es sich dabei bevorzugt um die Bildung einer Fluorenstruktur. Ganz besonders bevorzugt ist R' bei jedem Auftreten gleich oder verschieden ausgewählt aus der Gruppe bestehend aus einer geradkettigen Alkylgruppe mit 1, 2, 3 oder 4 C-Atomen, oder einer verzweigten Alkylgruppe mit 3 bis 6 C-Atomen.

In einer weiteren bevorzugten Ausführungsform der Erfindung ist R¹ gleich oder verschieden bei jedem Auftreten ausgewählt aus der Gruppe bestehend aus H, D, F, CN, einer geradkettigen Alkylgruppe mit 1 bis 10 C-Atomen oder einer verzweigten oder cyclischen Alkylgruppe mit 3 bis 10 C-Atomen, wobei die Alkylgruppe jeweils mit einem oder mehreren Resten R² substituiert sein kann, oder einem aromatischen oder heteroaromatischen Ringsystem mit 6 bis 24 aromatischen Ringatomen, das jeweils durch einen oder mehrere Reste R² substituiert sein kann. In einer besonders bevorzugten Ausführungsform der Erfindung ist R¹ gleich oder verschieden bei jedem Auftreten ausgewählt aus der Gruppe bestehend aus H, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe jeweils mit einem oder mehreren Resten R² substituiert sein kann, bevorzugt aber unsubstituiert ist, oder einem aromatischen oder heteroaromatischen Ringsystem mit 6 bis 13 aromatischen Ringatomen, das jeweils durch einen oder mehrere Reste R² substituiert sein kann, bevorzugt aber unsubstituiert ist.

In einer weiteren bevorzugten Ausführungsform der Erfindung ist R² gleich oder verschieden bei jedem Auftreten H, eine Alkylgruppe mit 1 bis 4 C-Atomen oder eine Arylgruppe mit 6 bis 10 C-Atomen, welche mit einer Alkylgruppe mit 1 bis 4 C-Atomen substituiert sein kann, bevorzugt aber unsubstituiert ist.

Dabei haben in erfindungsgemäßen Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Weiterhin ist es bevorzugt, wenn die Verbindung keine kondensierten Aryl- bzw. Heteroarylgruppen enthält, in denen mehr als zwei Sechsringe direkt aneinander ankondensiert sind. Eine Ausnahme hiervon bilden Phenanthren, Triphenylen sowie die oben aufgeführte Gruppe (HetAr-3), die aufgrund ihrer hohen Triplettenergie trotz der Anwesenheit kondensierter aromatischer Sechsringe bevorzugt sein können.

Die oben genannten bevorzugten Ausführungsformen können beliebig innerhalb der in Anspruch 1 definierten Einschränkungen miteinander kombiniert werden. In einer besonders bevorzugten Ausführungsform der Erfindung treten die oben genannten Bevorzugungen gleichzeitig auf.

Beispiele für bevorzugte Verbindungen gemäß den oben aufgeführten Ausführungsformen sind die in der folgenden Tabelle aufgeführten Verbindungen.

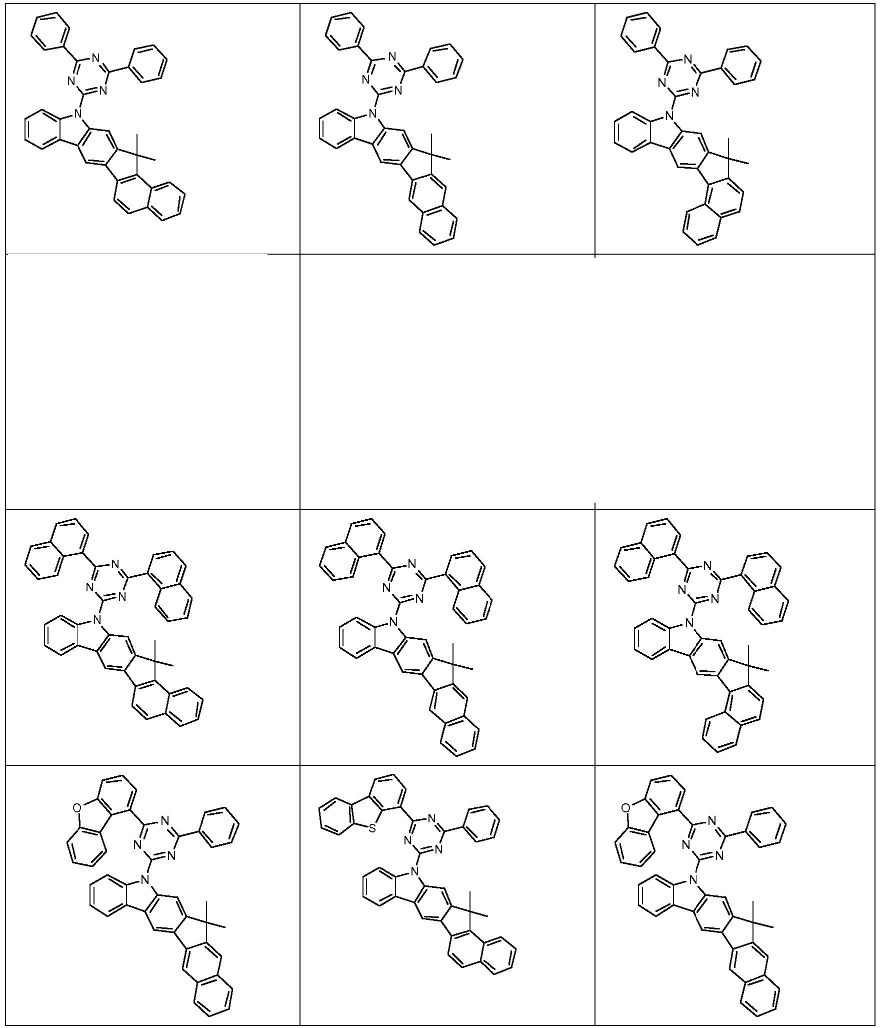

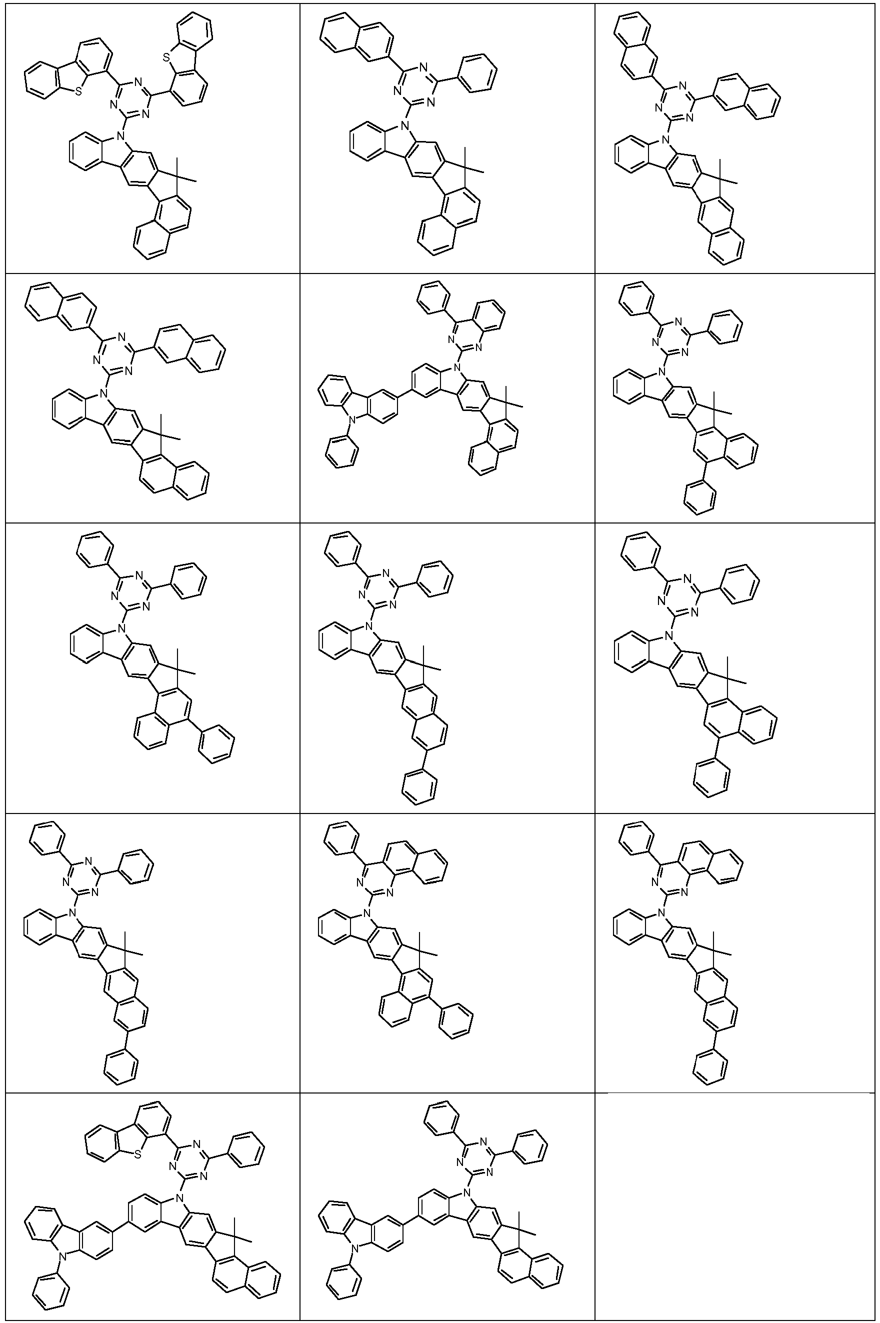

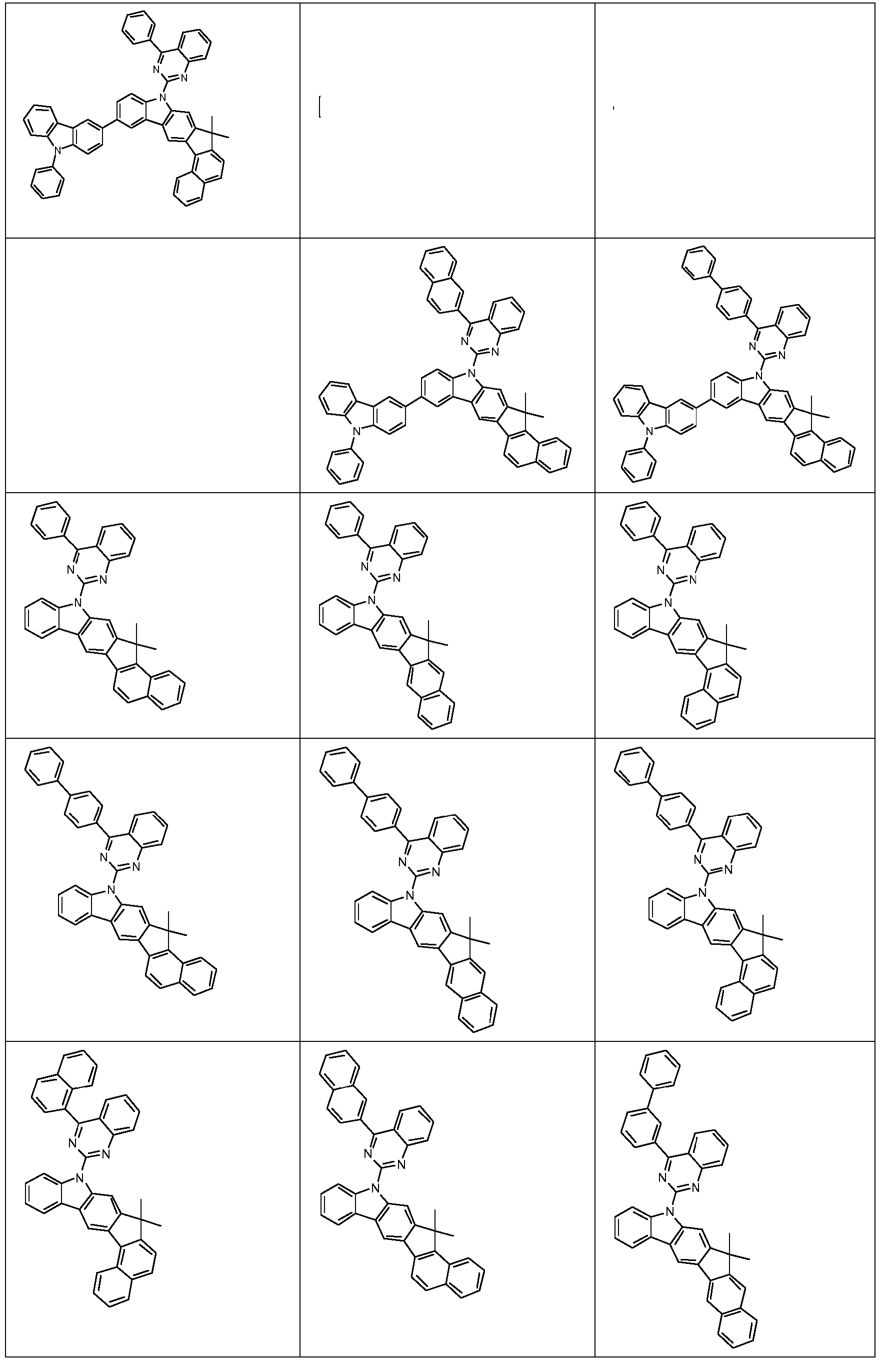

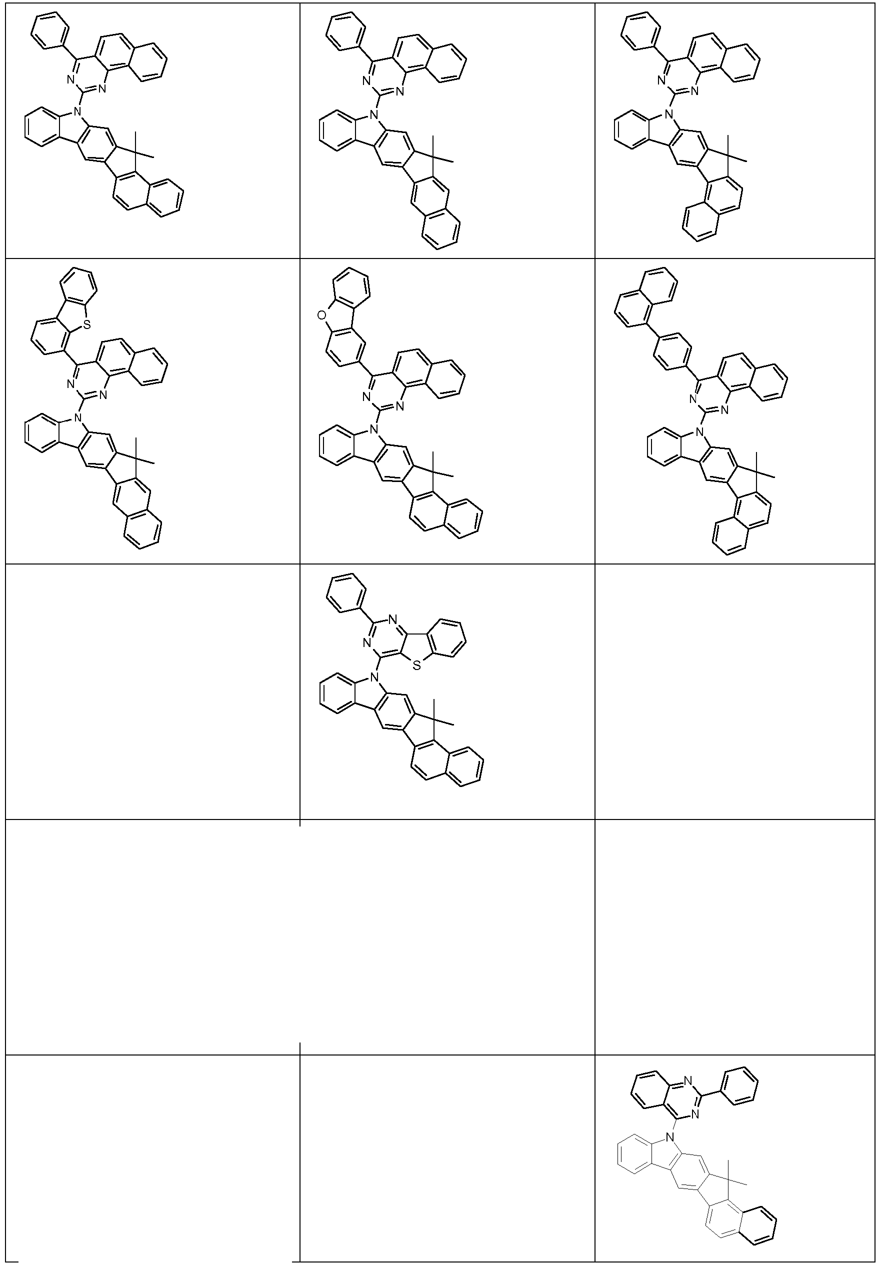

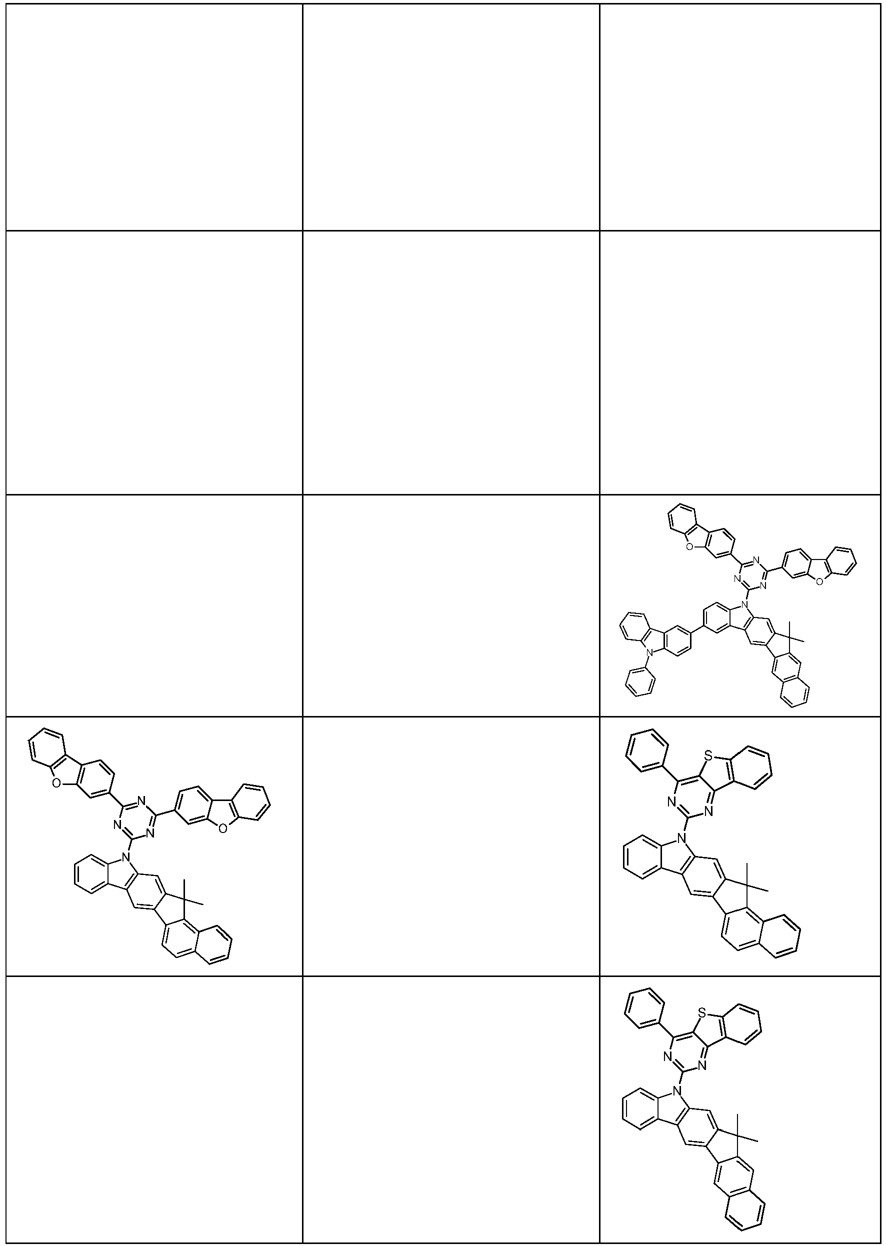

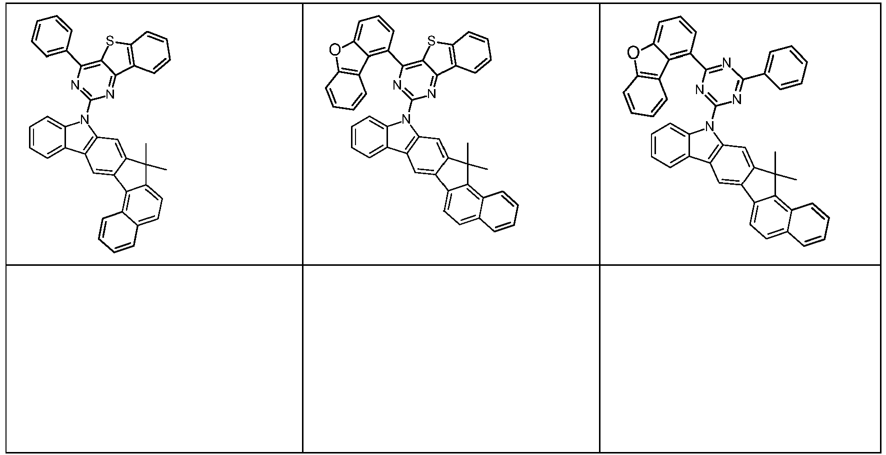

Die Grundstruktur der Verbindungen gemäß Formel (1) kann nach den in den nachfolgenden Schemata skizzierten Wegen dargestellt werden. Dabei sind die einzelnen Syntheseschritte, wie beispielsweise C-C-Kupplungsreaktionen gemäß Suzuki, C-N-Kupplungsreaktionen gemäß Hartwig-Buchwald oder Cyclisierungsreaktionen, dem Fachmann prinzipiell bekannt. Weitere Informationen zur Synthese der Verbindungen können den Synthesebeispielen entnommen werden. Die Synthese der Grundstruktur ist in Schema 1 dargestellt. Diese kann durch Kupplung eines Benzfluorens, welches mit einer reaktiven Abgangsgruppe, beispielsweise Brom, substituiert ist, mit einer gegebenenfalls substituierten 2-Nitrobenzolboronsäure, gefolgt von einer Ringschlussreaktion erfolgen. Alternativ kann die Kupplung mit der Aminogruppe eines gegebenenfalls substituierten 2-Aminochlorbenzols, gefolgt von einer Ringschlussreaktion erfolgen. In Schema 2 wird die Einführung der Gruppe HetAr am Stickstoffatom des Grundgerüsts dargestellt. Dabei kann eine mit einer geeigneten Abgangsgruppe, beispielweise Chlor, substituierte Gruppe HetAr in einer nukleophilen aromatischen Substitution oder in einer Palladiumkatalysierten Kupplungsreaktion eingeführt werden.

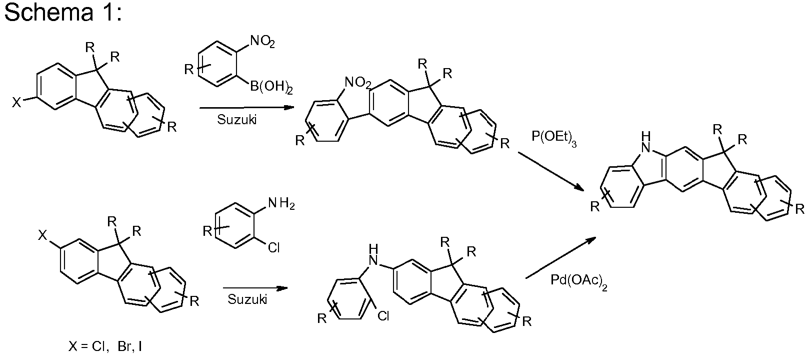

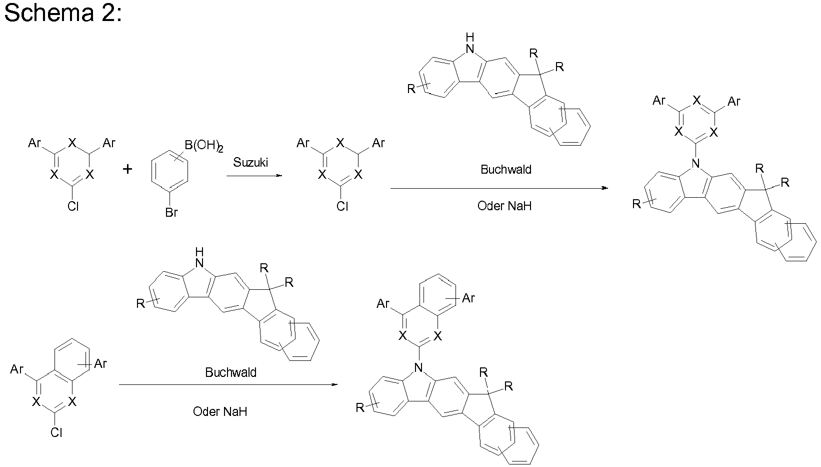

Die Verbindungen der Formel (1) können zur Herstellung der organischen Elektrolumineszenzvorrichtung zusammen mit der phosphoreszierenden Verbindung auch aus flüssiger Phase, beispielsweise durch Spin-Coating oder durch Druckverfahren, verarbeitet werden. Hierfür sind Formulierungen der Verbindungen erforderlich, beispielsweise Lösungen, Dispersionen oder Emulsionen. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthyl-isovalerat, Cyclohexylhexanoat oder Mischungen dieser Lösemittel.

Ein weiterer Gegenstand der vorliegenden Erfindung ist daher eine Formulierung bzw. eine Zusammensetzung, enthaltend mindestens eine Verbindung gemäß Formel (1), mindestens eine phosphoreszierende Verbindung und mindestens ein Lösemittel, insbesondere eines der oben genannten Lösemittel oder eine Mischung dieser Lösemittel. Die Formulierung kann auch weitere organische oder anorganische Verbindung enthalten, beispielsweise ein oder mehrere weitere Matrixmaterialien. Geeignete phosphoreszierende Verbindungen und weitere Matrixmaterialien sind hinten genauer ausgeführt.

Ein weiterer Gegenstand der vorliegenden Erfindung ist die Verwendung einer erfindungsgemäßen Formulierung zur Herstellung einer organischen Elektrolumineszenzvorrichtung.

Die erfindungsgemäße organische Elektrolumineszenzvorrichtung enthält, wie oben definiert, Kathode, Anode und mindestens eine emittierende Schicht, welche mindestens eine phosphoreszierende Verbindung und mindestens eine Verbindung gemäß Formel (1) enthält. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen. Es kann sich bei der erfindungsgemäßen organischen Elektrolumineszenzvorrichtung auch um eine Tandem-OLED handeln, insbesondere für weiß emittierende OLEDs.

Bevorzugt enthält die erfindungsgemäße Elektrolumineszenzvorrichtung als phosphoreszierende Verbindung eine rot, orange oder gelb phosphoreszierende Verbindung, insbesondere eine rot phosphoreszierende Verbindung.

Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der Verbindung gemäß Formel (1) und der phosphoreszierenden Verbindung in der emittierenden Schicht der Elektrolumineszenzvorrichtung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der Verbindung gemäß Formel (1) bezogen auf die Gesamtmischung aus phosphoreszierender Verbindung und Verbindung gemäß Formel (1). Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% der phosphoreszierenden Verbindung bezogen auf die Gesamtmischung aus phosphoreszierender Verbindung und Verbindung gemäß Formel (1). Wird die Elektrolumineszenzvorrichtung aus Lösung hergestellt, gelten die gleichen bevorzugten Anteile, wobei dann statt Vol.-% die entsprechenden Gew.% als bevorzugte Anteile eingesetzt werden.

In einer Ausführungsform der Erfindung wird die Verbindung gemäß Formel (1) als einziges Matrixmaterial ("single host") für die phosphoreszierende Verbindung eingesetzt, d. h. die emittierende Schicht der OLED enthält nur die Verbindung der Formel (1) und die phosphoreszierende Verbindung, aber keine weiteren Verbindungen. Dies stellt einen deutlich Vorteil gegenüber Elektrolumineszenzvorrichtungen dar, die zwei oder mehr Matrixmaterialien in der emittierenden Schicht enthalten, da derartige Elektrolumineszenzvorrichtungen deutlich einfacher gefertigt werden können als solche, die Mischungen mehrerer Matrixmaterialien enthalten. Überraschend wurde gefunden, dass gerade Verbindungen der Formel (1) zu sehr guten Ergebnissen führen, wenn diese Materialien als einziges Matrixmaterial für phosphoreszierende Verbindungen eingesetzt werden. Es ist daher eine bevorzugte Ausführungsform der Erfindung, dass die emittierende Schicht aus genau einer Verbindung der Formel (1) und einer oder mehreren phosphoreszierenden Verbindungen, bevorzugt genau einer phosphoreszierenden Verbindung, besteht.

In einer weiteren Ausführungsform der Erfindung enthält die emittierende Schicht außer der Verbindung der Formel (1) und der phosphoreszierenden Verbindung mindestens ein weiteres Matrixmaterial. Geeignete Matrixmaterialien, welche in Kombination mit einer Verbindung gemäß Formel (1) eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Bis-carbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, Triphenylenderivate, z. B. gemäß WO 2012/048781, Dibenzofuranderivate, z. B. gemäß WO 2015/169412, WO 2016/015810, WO 2016/023608, WO 2017/148564 oder WO 2017/148565 oder Biscarbazole, z. B. gemäß JP 3139321 B2.

Ebenso kann eine weitere phosphoreszierende Verbindung, welche kürzerwellig als der eigentliche Emitter emittiert, als Co-Host in der emittierenden Schicht vorhanden sein. Besonders gute Ergebnisse werden erzielt, wenn als Emitter eine rot phoshoreszierende Verbindung eingesetzt wird und als Co-Host in Kombination mit der Verbindung gemäß Formel (1) eine gelb phosphoreszierende Verbindung verwendet wird.

Weiterhin kann als Co-Host eine Verbindung verwendet werden, die nicht oder nicht in wesentlichem Umfang am Ladungstransport teilnimmt, wie beispielsweise in WO 2010/108579 beschrieben. Insbesondere eignen sich in Kombination mit der Verbindung gemäß Formel (1) als Co-MatrixMaterial Verbindungen, welche eine große Bandlücke aufweisen und welche selber nicht oder zumindest nicht in wesentlichem Maße am Ladungstransport der emittierenden Schicht teilnehmen. Es handelt sich bei solchen Materialien bevorzugt um reine Kohlenwasserstoffe. Beispiele für solche Materialien finden sich beispielsweise in der WO 2009/124627 oder in der WO 2010/006680.

Besonders bevorzugte Co-Host-Materialien, welche in Kombination mit den Verbindungen gemäß Formel (1) eingesetzt werden können, sind Biscarbazol- bzw. Indenocarbazol-Derivate gemäß einer der Formeln (6), (7) oder (8), 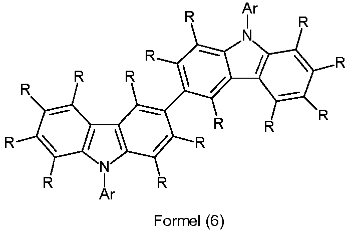 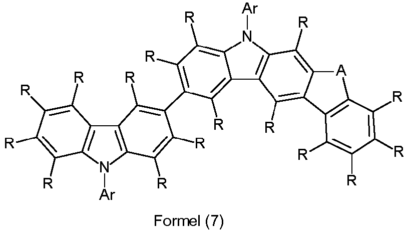 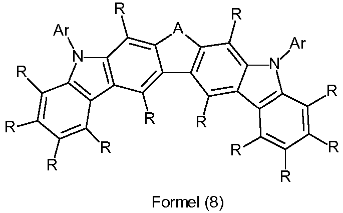 wobei Ar und A die oben genannten Bedeutungen aufweisen und R die oben genannten Bedeutungen aufweist, jedoch Reste R hier auch miteinander ein aromatisches oder heteroaromatisches Ringsystem bilden können. In einer bevorzugten Ausführungsform der Erfindung steht A für C(R')₂.

Bevorzugte Ausführungsformen der Verbindungen der Formeln (6), (7) bzw. (8) sind die Verbindungen der folgenden Formeln (6a), (7a) bzw. (8a), 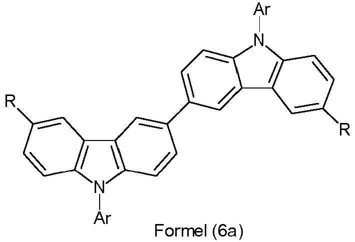 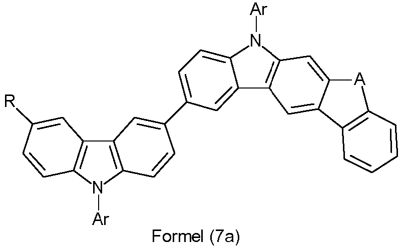 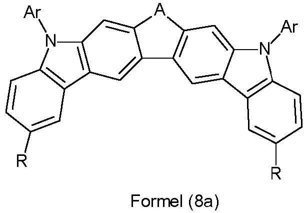 wobei die verwendeten Symbole die oben genannten Bedeutungen aufweisen.

Beispiele für geeignete Verbindungen gemäß Formel (6) sind die nachfolgend abgebildeten Verbindungen.

| Struktur | CAS-Nummer | Struktur | CAS-Nummer |
|---|---|---|---|
| 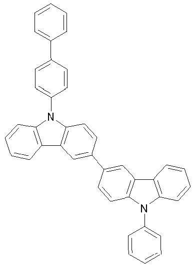 | CAS-1454567-05-5 | 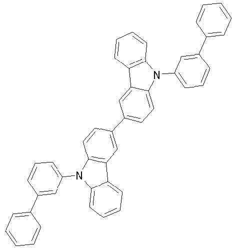 | CAS-1352040-89-1 |
| 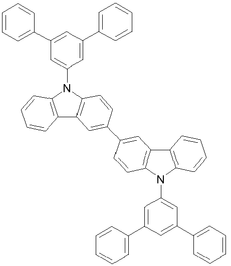 | CAS-1336889-25-8 | 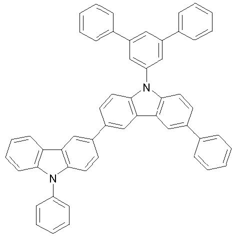 | CAS-1800544-05-1 |
| 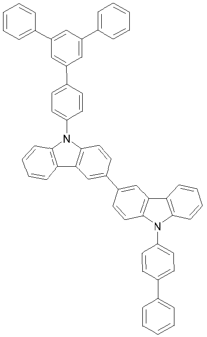 | CAS-1800544-08-4 | 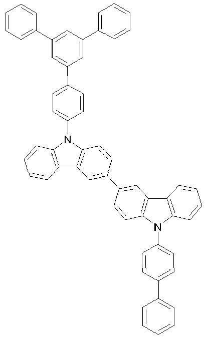 | CAS-1800544-08-4 |
| 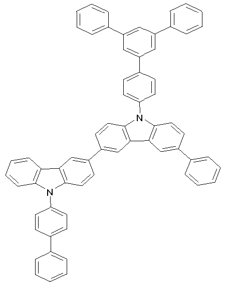 | CAS-1800544-09-5 | 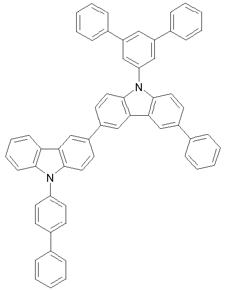 | CAS-1800544-10-8 |
| 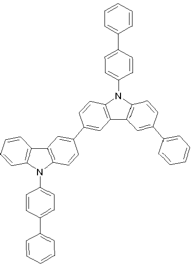 | CAS-1800544-11-9 | 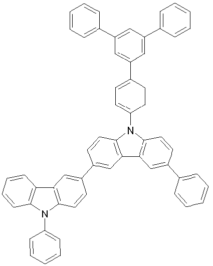 | CAS-1800544-04-0 |
| 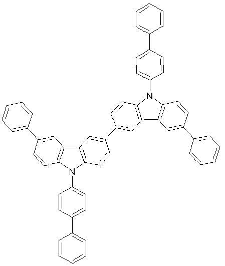 | CAS-1842320-52-8 | 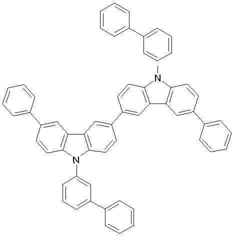 | CAS-1842320-53-9 |
| 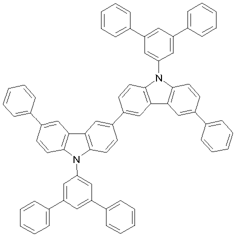 | CAS-1842320-54-0 | 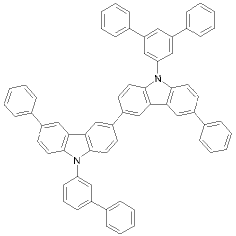 | CAS-1842320-55-1 |
| 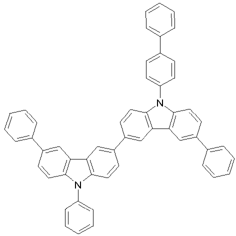 | CAS-1842320-56-2 | 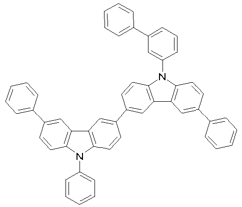 | CAS-1842320-57-3 |
| 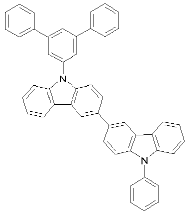 | CAS-1410876-33-3 | 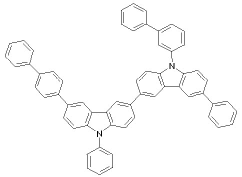 | CAS-1842320-58-4 |
| 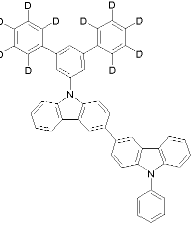 | CAS-1410876-47-9 | 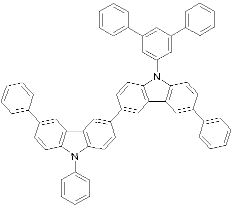 | CAS-1842320-59-5 |
| 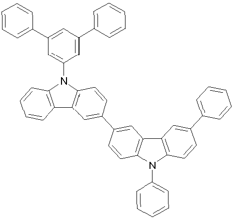 | CAS-1848256-38-1 | 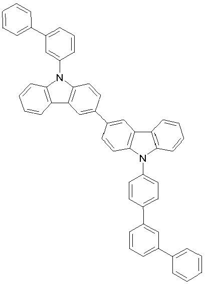 | CAS-1865661-14-8 |
| 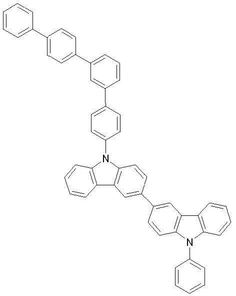 | CAS-1870867-25-6 | 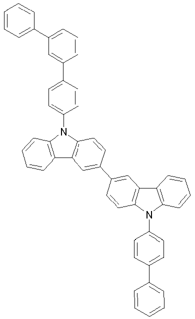 | CAS-1884707-32-7 |
| 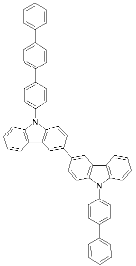 | CAS-1889262-88-7 | 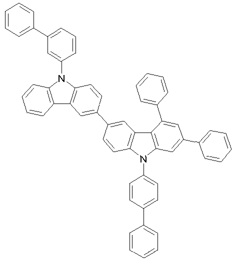 | CAS-2018307-89-4 |
| 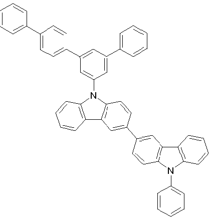 | CAS-1454655-29-8 | 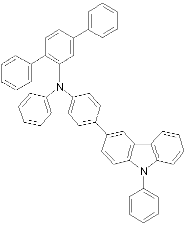 | CAS-1454655-33-4 |
| 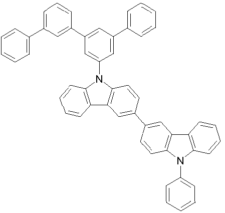 | CAS-1454660-22-0 | 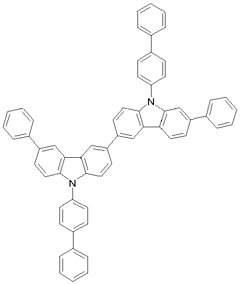 | CAS-1907663-27-7 |
| 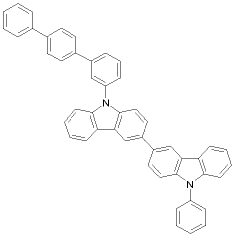 | CAS-1548581-24-3 | 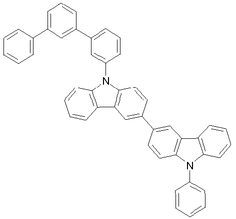 | CAS-1548581-27-6 |
| 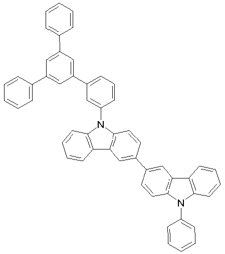 | CAS-1548581-29-8 | 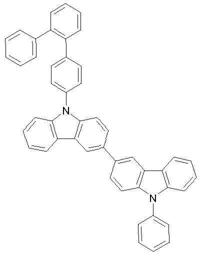 | CAS-1548581-37-8 |
| 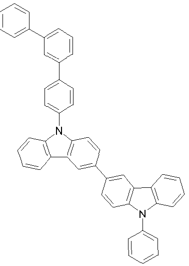 | CAS-1548581-40-3 | 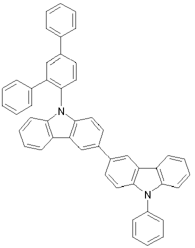 | CAS-1943719-62-7 |
| 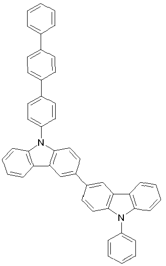 | CAS-1548581-42-5 | 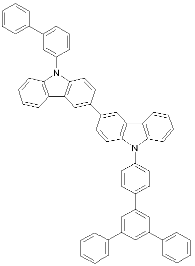 | CAS-1942079-50-6 |
| 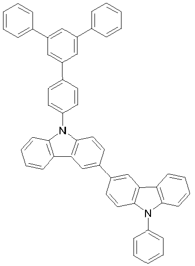 | CAS-1548581-44-7 | 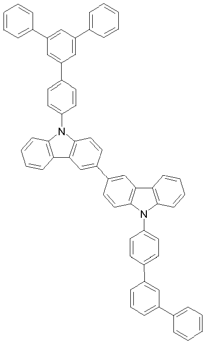 | CAS-1942079-51-7 |
| 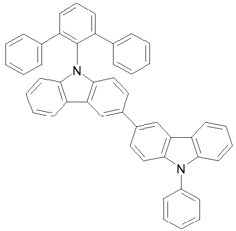 | CAS-1943719-63-8 | 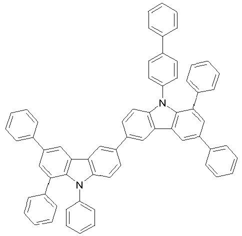 | CAS-1955476-12-6 |
| 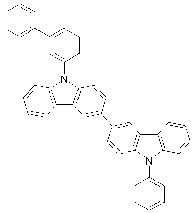 | CAS-1619966-75-4 | 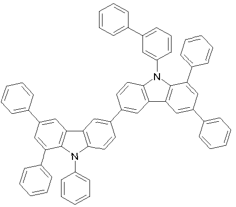 | CAS-1955476-13-7 |
| 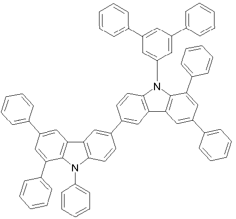 | CAS-1955476-15-9 | 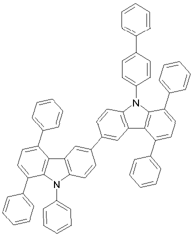 | CAS-1955476-28-4 |
| 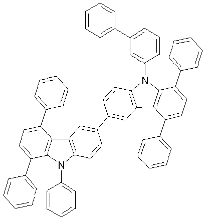 | CAS-1955476-30-8 | 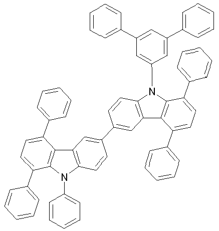 | CAS-1955476-32-0 |
| 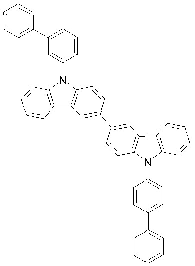 | CAS-1643479-47-3 | 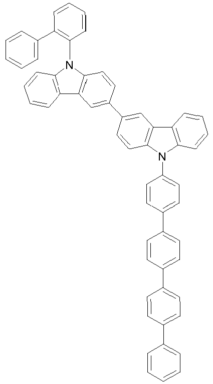 | CAS-1973498-04-2 |
| 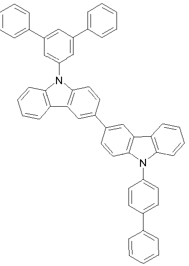 | CAS-1643479-49-5 | 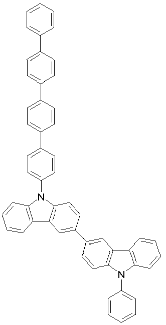 | CAS-1973498-03-1 |
| 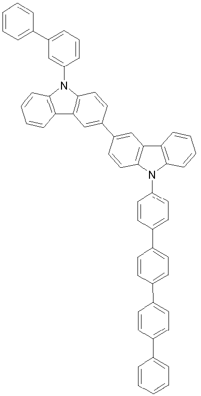 | CAS-1973498-05-3 | 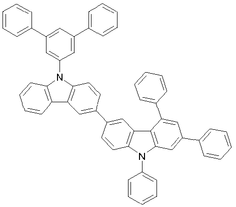 | CAS-2018307-36-1 |
| 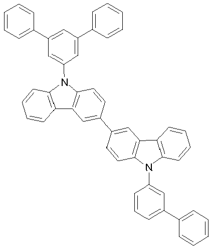 | CAS-1643479-56-4 | 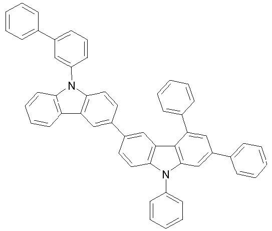 | CAS-2018307-35-0 |
| 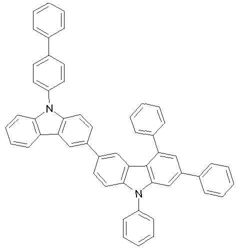 | CAS-2018307-37-2 | 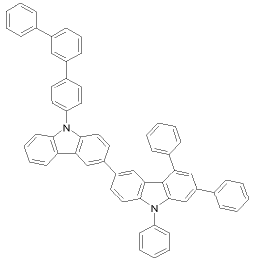 | CAS-2018307-38-3 |
| 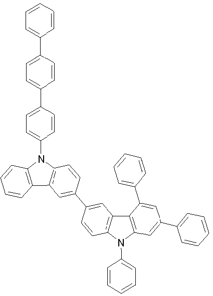 | CAS-2018307-39-4 | 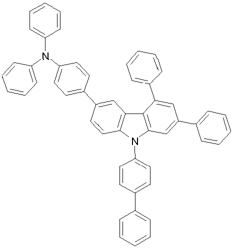 | CAS-2018307-77-0 |
| 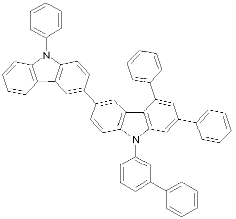 | CAS-2018307-78-1 | 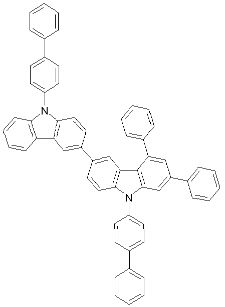 | CAS-2018307-90-7 |
| 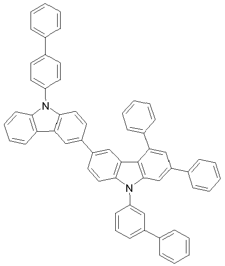 | CAS-2018307-91-8 | 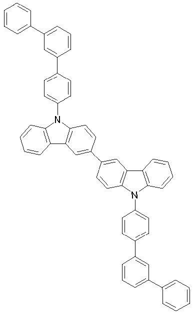 | CAS-1799958-74-9 |
| 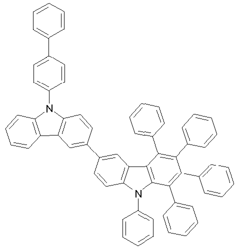 | CAS-2052160-86-6 | 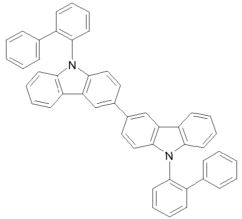 | CAS-1799958-79-4 |
| 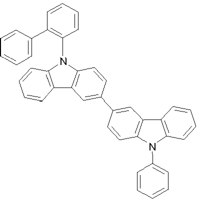 | CAS-1799958-76-1 | 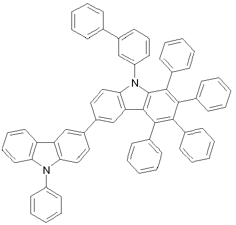 | CAS-2052160-91-3 |
| 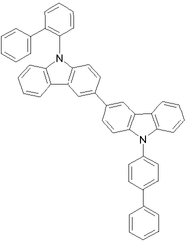 | CAS-1799958-77-2 | 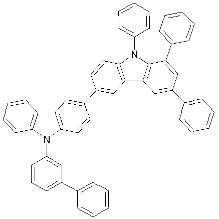 | CAS-2055848-40-1 |
| 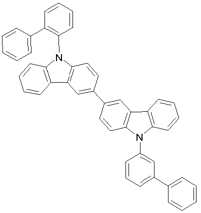 | CAS-1799958-78-3 | 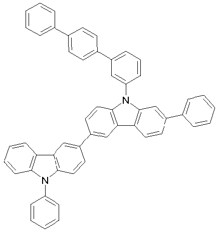 | CAS-2057418-19-4 |
| 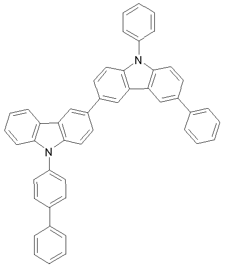 | CAS-1799958-99-8 | 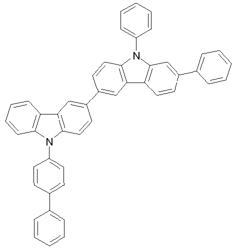 | CAS-1799959-01-5 |
| 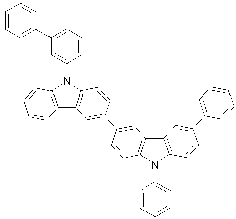 | CAS-1799959-03-7 | 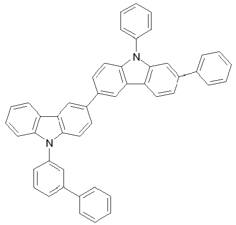 | CAS-1799959-05-9 |
| 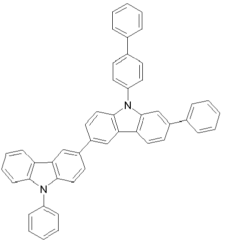 | CAS-1799959-07-1 | 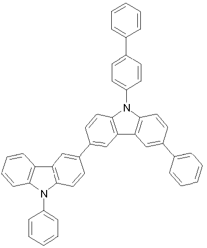 | CAS-1799959-09-3 |
| 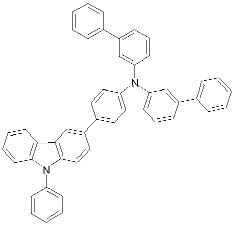 | CAS-1799959-11-7 | 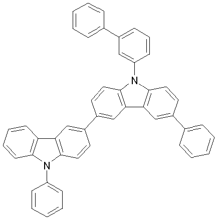 | CAS-1799959-13-9 |
| 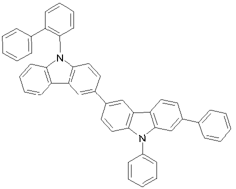 | CAS-2085318-61-0 | 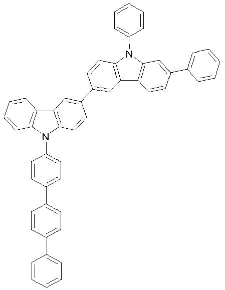 | CAS-2085318-62-1 |
| 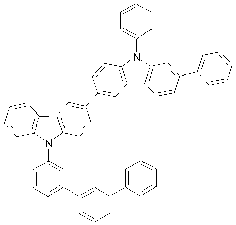 | CAS-2085318-64-3 | 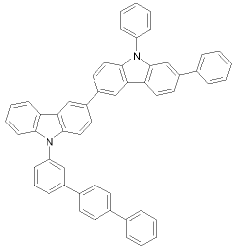 | CAS-2085318-63-2 |
| 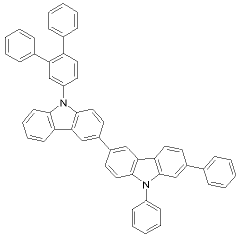 | CAS-2085318-66-5 | 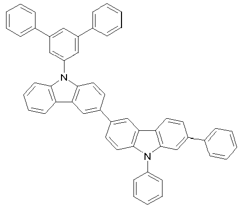 | CAS-2085318-65-4 |
| 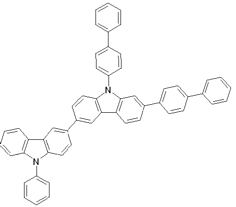 | CAS-2085318-77-8 | 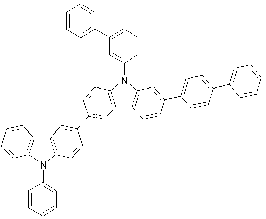 | CAS-2085318-78-9 |
| 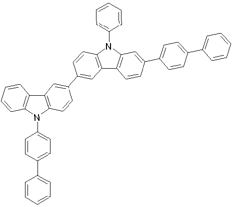 | CAS-2085318-79-0 | 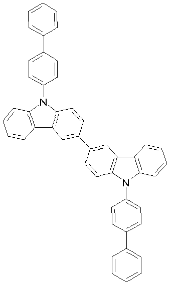 | CAS-57102-51-9 |
| 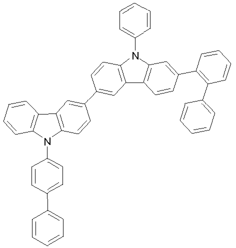 | CAS-2085318-81-4 | 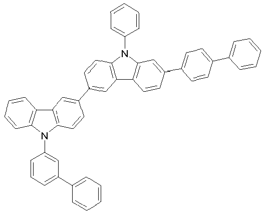 | CAS-2085318-80-3 |
| 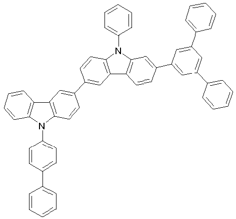 | CAS-2085318-83-6 | 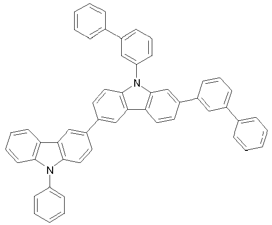 | CAS-2085318-82-5 |
| 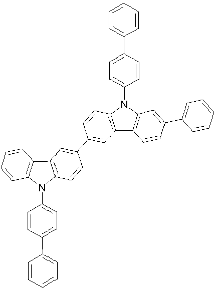 | CAS-2085318-88-1 | 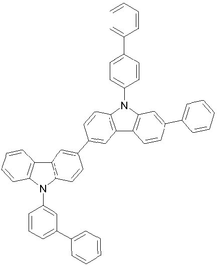 | CAS-2085318-87-0 |
| 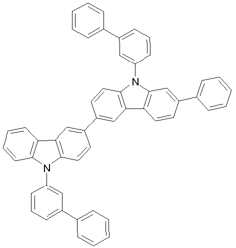 | CAS-2085318-92-7 | 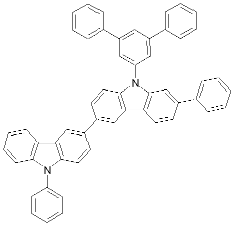 | CAS-2085318-89-2 |
| 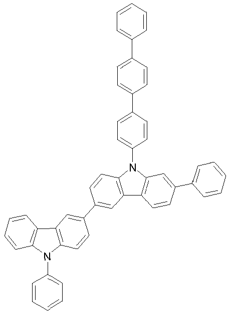 | CAS-2085318-94-9 | 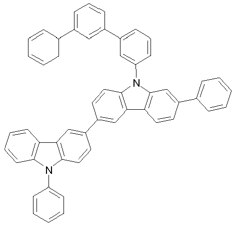 | CAS-2085318-93-8 |
| 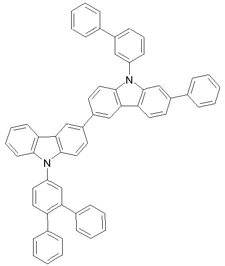 | CAS-2085318-98-3 | 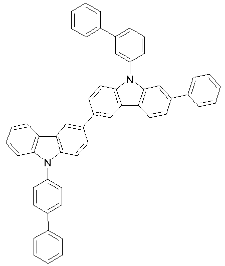 | CAS-2085318-97-2 |
| 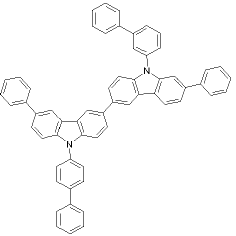 | CAS-2085319-00-0 | 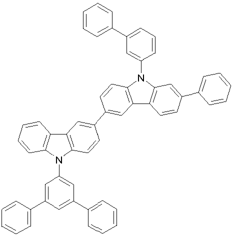 | CAS-2085318-99-4 |
| 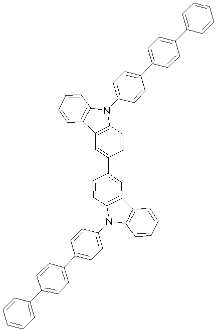 | CAS-251316-80-0 | 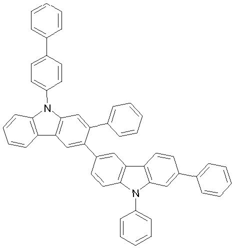 | CAS- 2085319-17-9 |
| 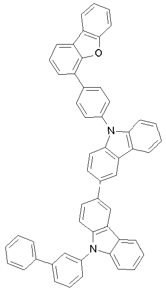 | | 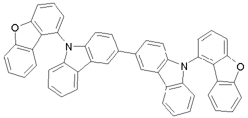 | CAS-1427160-09-5 |
| 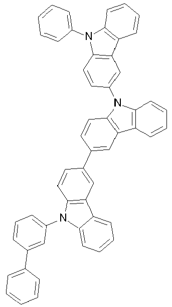 | CAS-1643479-72-4 | 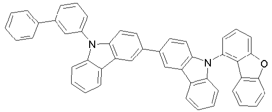 | |
| 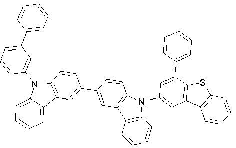 | | 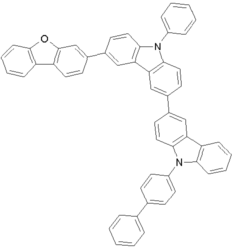 | CAS-1799959-65-1 |
| 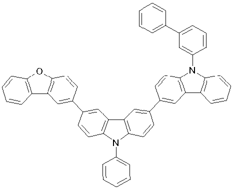 | CAS-1799959-74-2 | 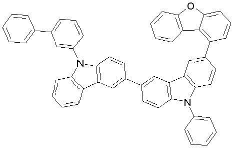 | CAS-1799959-75-3 |
| 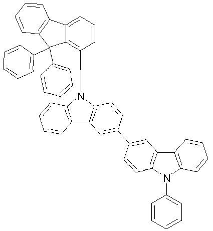 | CAS-1799960-24-9 | 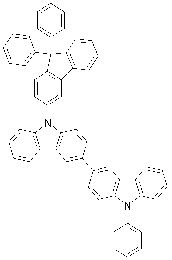 | CAS-1799960-25-0 |
| 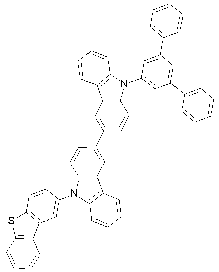 | CAS-1340668-17-8 | 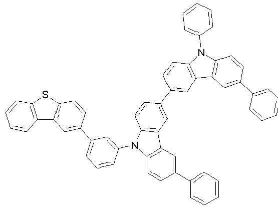 | CAS-1340668-19-0 |
| 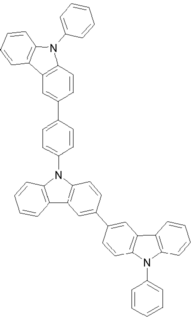 | CAS-1289556-24-6 | 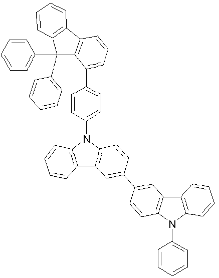 | CAS-1799960-56-7 |
| 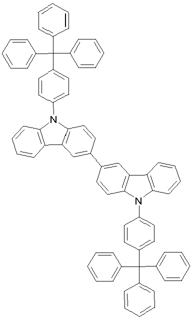 | CAS-1336889-27-0 | 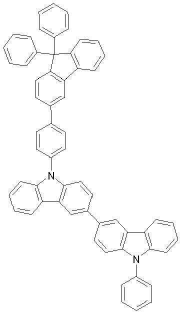 | CAS-1799960-58-9 |
| 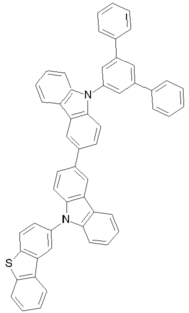 | CAS-1340668-17-8 | 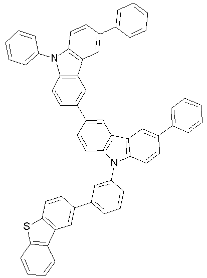 | CAS-1340668-19-0 |
| 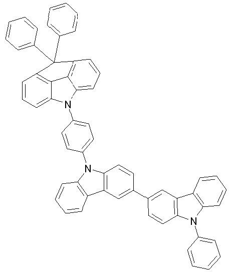 | CAS-1812208-18-6 | 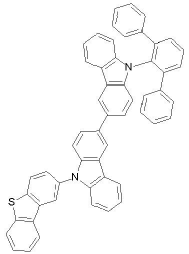 | CAS-1340668-35-0 |
| 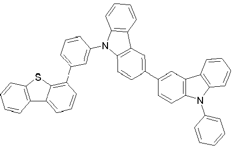 | CAS-1340668-37-2 | 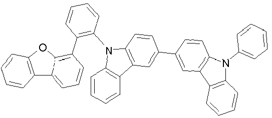 | CAS-1830334-82-1 |
| 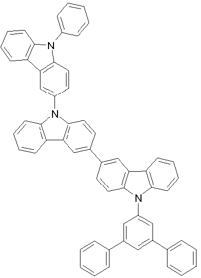 | CAS-1340669-19-3 | 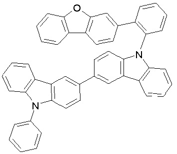 | CAS-1830334-85-4 |
| 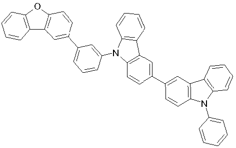 | CAS-1830334-94-5 | 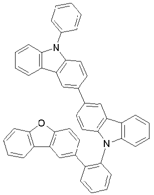 | CAS-1830334-88-7 |
| 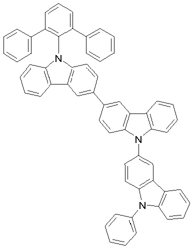 | CAS-1340669-32-0 | 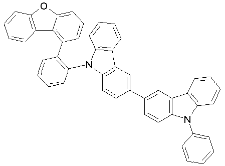 | CAS-1830334-90-1 |
| 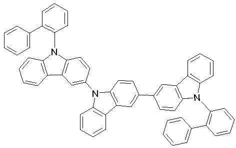 | CAS-1340669-33-1 | 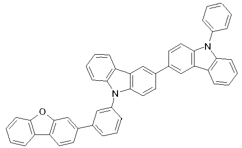 | CAS-1830334-91-2 |
| 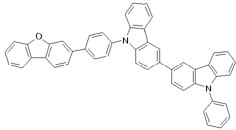 | CAS-1830335-02-8 | 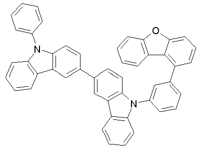 | CAS-1830334-97-8 |
| 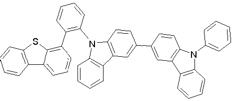 | CAS-1830335-71-1 | 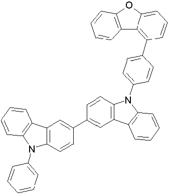 | CAS-1830335-07-3 |
| 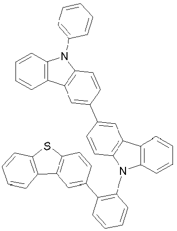 | CAS-1830335-76-6 | 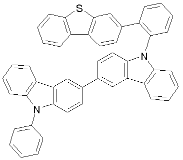 | CAS-1830335-72-2 |
| 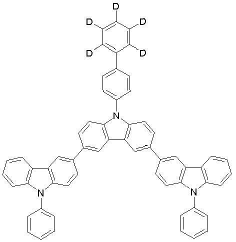 | CAS-1354054-11-7 | 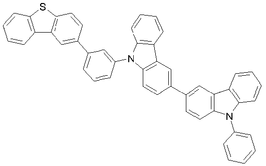 | CAS-1830335-85-7 |
| 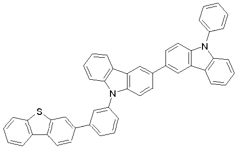 | CAS-1830335-82-4 | 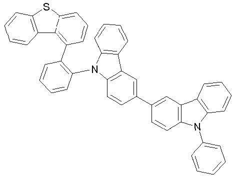 | CAS-1830335-79-9 |
| 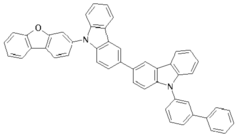 | CAS-1830339-40-6 | 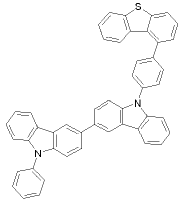 | CAS-1830335-95-9 |
| 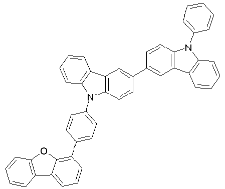 | CAS-1377150-35-0 | 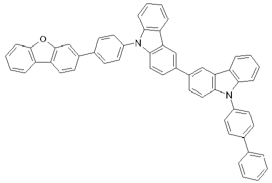 | CAS-1830339-41-7 |
| 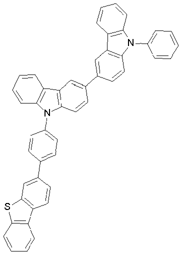 | CAS-1830335-90-4 | 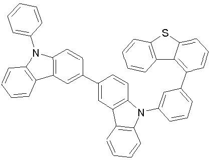 | CAS-1830335-87-9 |
| 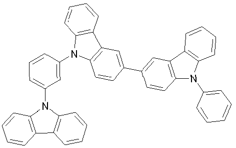 | CAS-1399855-37-8 | 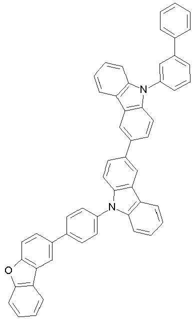 | CAS-1830339-42-8 |
| 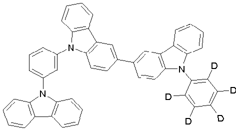 | CAS-1399855-38-9 | 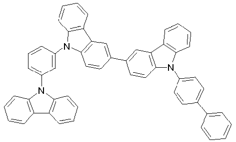 | CAS-1399855-39-0 |
| 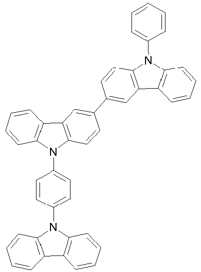 | CAS-1399855-46-9 | 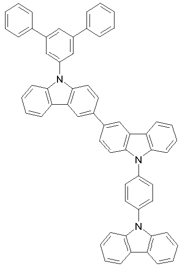 | CAS-1399855-47-0 |
| 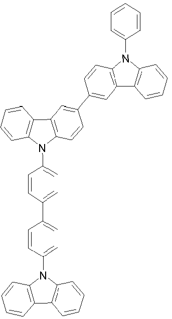 | CAS-1413936-92-1 | 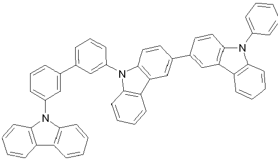 | CAS-1413936-95-4 |
| 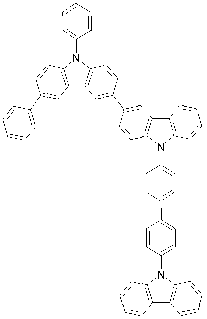 | CAS-1413936-96-5 | 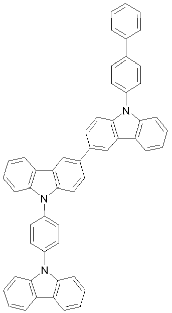 | CAS-1413936-97-6 |
| 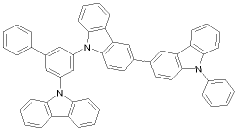 | CAS-1413937-08-2 | 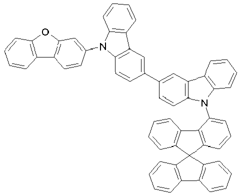 | CAS-1890157-92-2 |
| 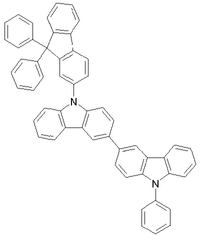 | CAS-1415348-93-4 | 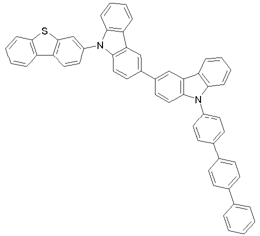 | CAS-1889262-89-8 |
| 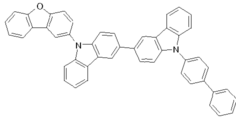 | CAS-1415348-99-0 | 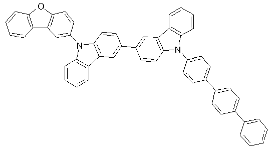 | CAS-1890156-90-7 |
| 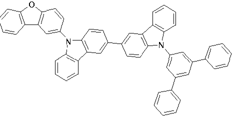 | CAS-1415349-00-6 | 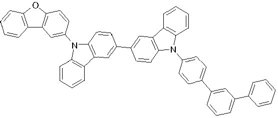 | CAS-1890156-91-8 |
| 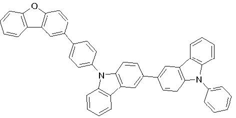 | CAS-1415349-01-7 | 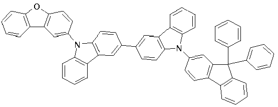 | CAS-1890157-12-6 |
| 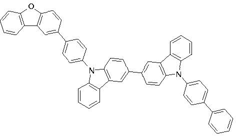 | CAS-1415349-02-8 | 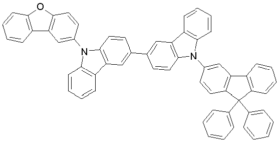 | CAS-1890157-13-7 |
| 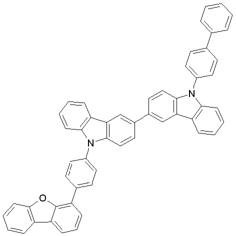 | CAS-1415349-03-9 | 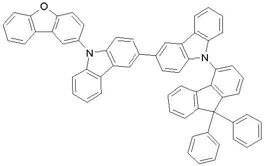 | CAS-1890157-14-8 |
| 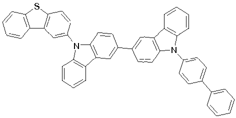 | CAS-1415349-04-0 | 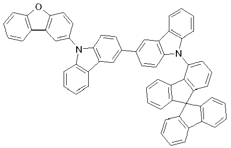 | CAS-1890157-37-5 |
| 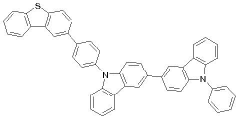 | CAS-1415349-05-1 | 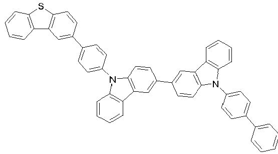 | CAS-1415349-06-2 |
| 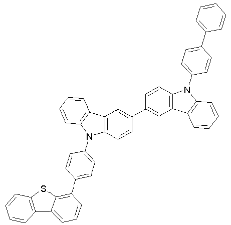 | CAS-1415349-07-3 | 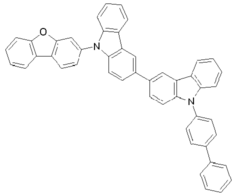 | CAS-1890157-41-1 |
| 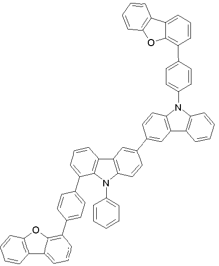 | CAS-1415422-76-2 | 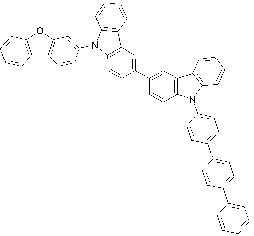 | CAS-1890157-42-2 |
| 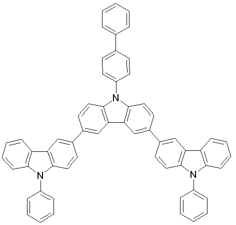 | CAS-1422451-46-4 | 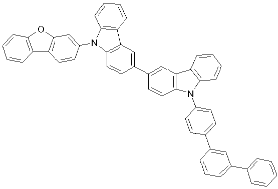 | CAS-1890157-43-3 |
| 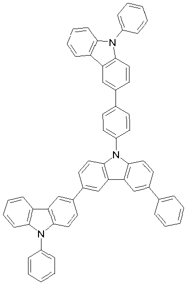 | CAS-1422451-48-6 | 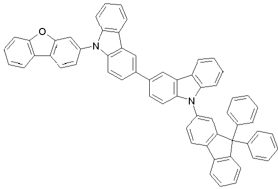 | CAS-1890157-64-8 |
| 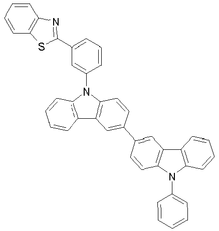 | CAS-1445952-53-3 | 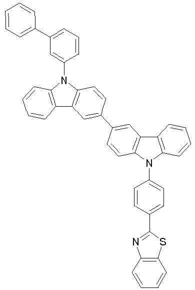 | CAS-1445952-58-8 |
| 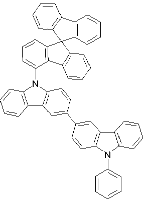 | CAS-1450933-86-4 | 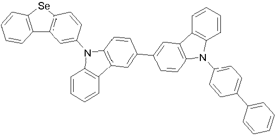 | CAS-1894194-07-0 |
| 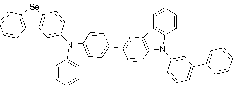 | CAS-1894194-09-2 | 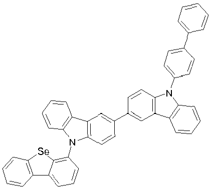 | CAS-1894194-08-1 |
| 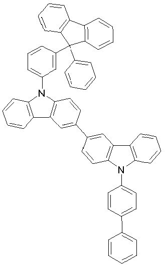 | CAS-1919031-93-8 | 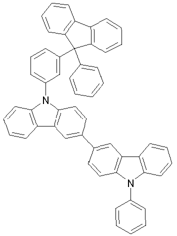 | CAS-1919031-92-7 |
| 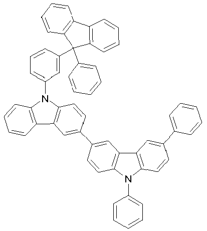 | CAS-1919031-95-0 | 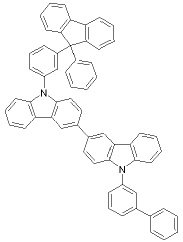 | CAS-1919031-94-9 |
| 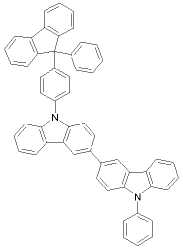 | CAS-1919031-97-2 | 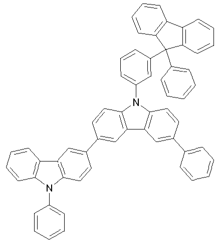 | CAS-1919031-96-1 |
| 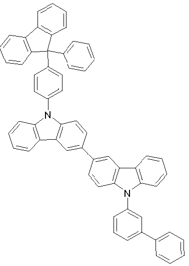 | CAS-1919031-99-4 | 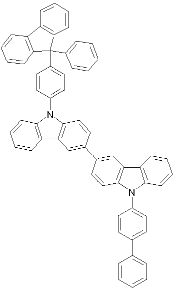 | CAS-1919031-98-3 |
| 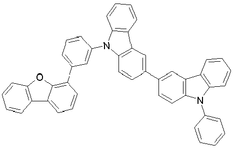 | CAS-1598389-98-0 | 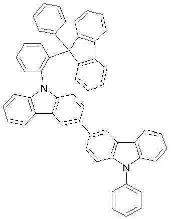 | CAS-1919032-02-2 |
| 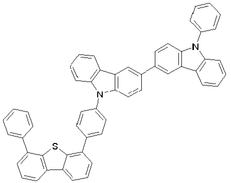 | CAS-1604034-14-1 | 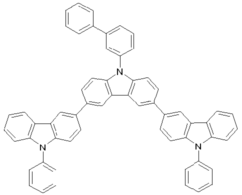 | CAS-1943719-67-2 |
| 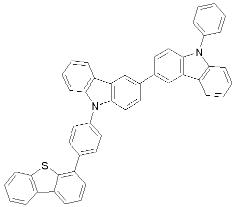 | CAS-1604034-02-7 | 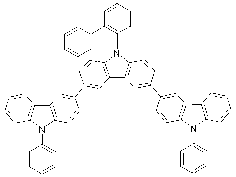 | CAS-1943719-70-7 |
| 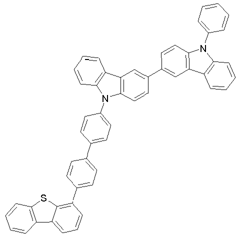 | CAS-1604034-07-2 | 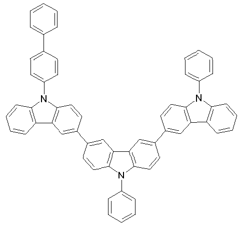 | CAS-1943719-71-8 |
| 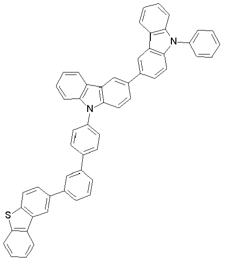 | CAS-1604034-12-9 | 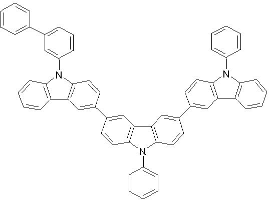 | CAS-1943719-72-9 |
| 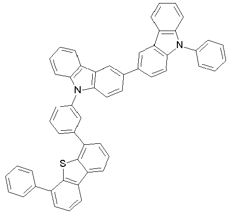 | CAS-1622931-00-3 | 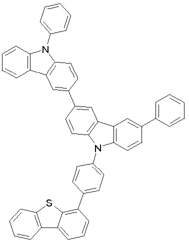 | CAS-1604034-15-2 |
| 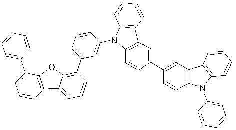 | CAS-1622931-01-4 | 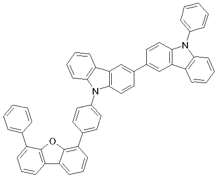 | CAS-1622931-04-7 |
| 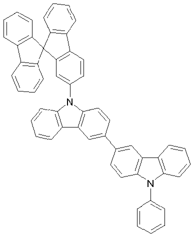 | CAS-1630029-28-5 | 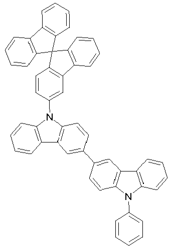 | CAS-1630029-29-6 |
| 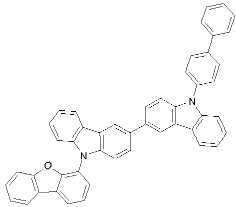 | CAS-1643479-51-9 | 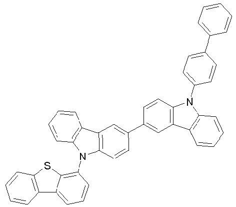 | CAS-1643479-52-0 |
| 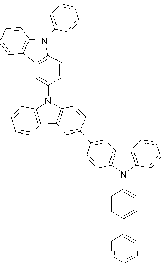 | CAS-1643479-54-2 | 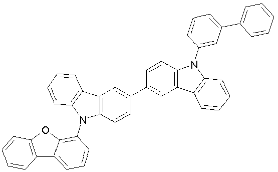 | CAS-1643479-59-7 |
| 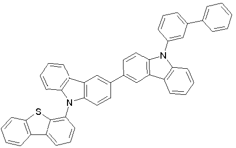 | CAS-1643479-62-2 | 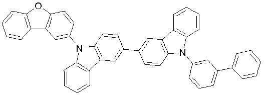 | CAS-1643479-68-8 |
| 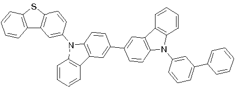 | CAS-1643479-69-9 | 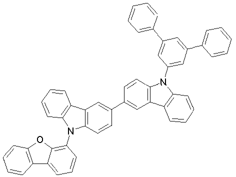 | CAS-1643479-74-6 |
| 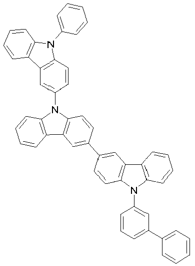 | CAS-1643479-72-4 | 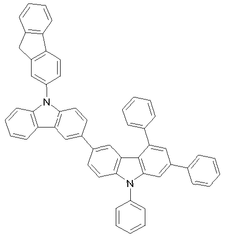 | CAS-2018307-43-0 |
| 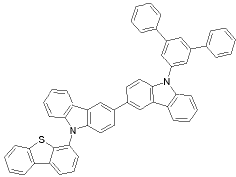 | CAS-1643479-75-7 | 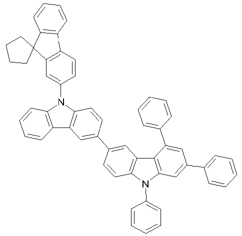 | CAS-2018307-47-4 |
| 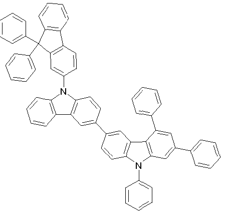 | CAS-2018307-50-9 | 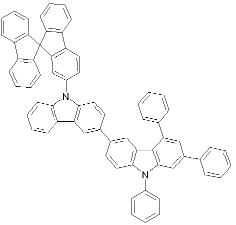 | CAS- 2018307-49-6 |
| 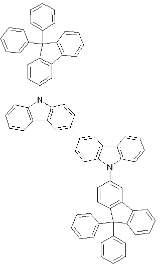 | CAS-1656982-30-7 | 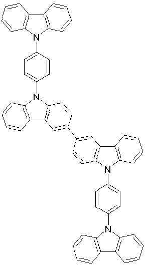 | CAS-1680184-58-0 |
| 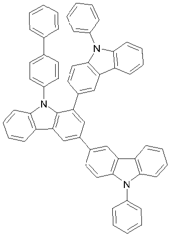 | CAS-1704071-12-4 | 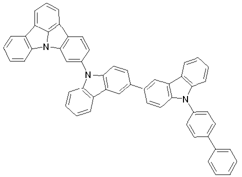 | CAS-1799483-56-9 |
| 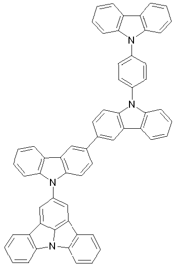 | CAS-1799519-35-9 | 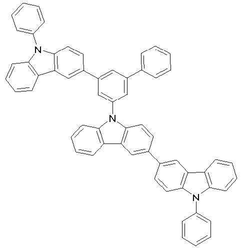 | CAS-1799678-37-7 |
| 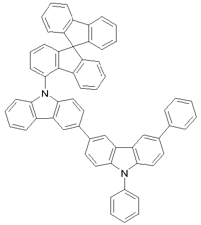 | CAS-2073116-97-7 | 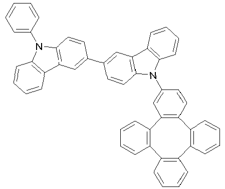 | CAS-2048236-10-6 |
| 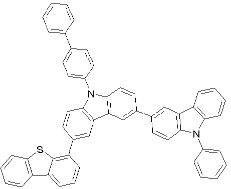 | CAS-1799959-20-8 | 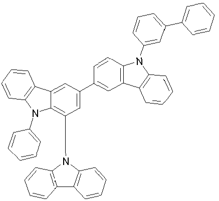 | CAS-1704071-30-6 |
| 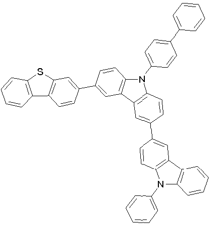 | CAS-1799959-21-9 | 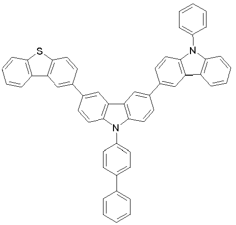 | CAS-1799959-22-0 |
| 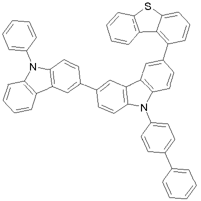 | CAS-1799959-23-1 | 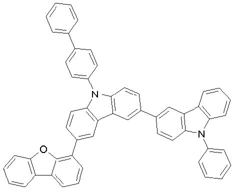 | CAS-1799959-24-2 |
| 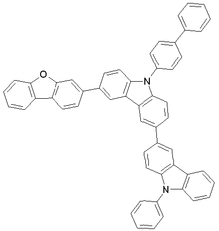 | CAS-1799959-25-3 | 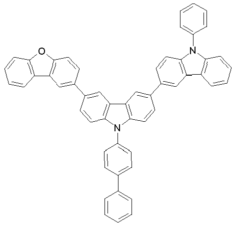 | CAS-1799959-26-4 |
| 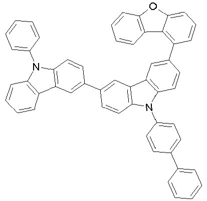 | CAS-1799959-27-5 | 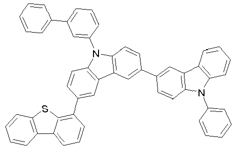 | CAS-1799959-28-6 |
| 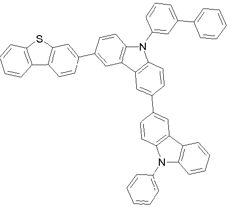 | CAS-1799959-29-7 | 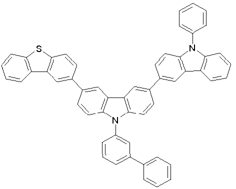 | CAS-1799959-30-0 |
| 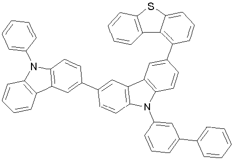 | CAS-1799959-31-1 | 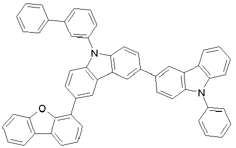 | CAS-1799959-32-2 |
| 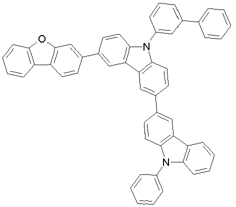 | CAS-1799959-33-3 | 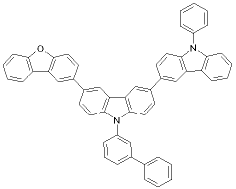 | CAS-1799959-34-4 |
| 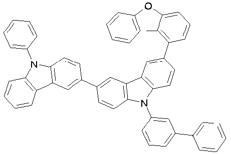 | CAS-1799959-35-5 | 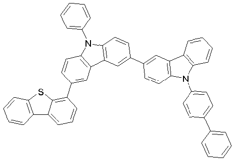 | CAS-1799959-60-6 |
| 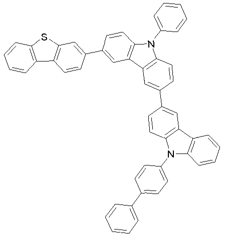 | CAS-1799959-61-7 | 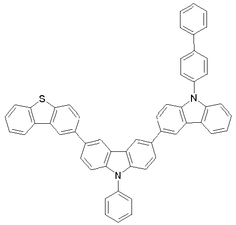 | CAS-1799959-62-8 |
| 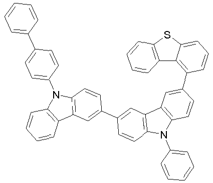 | CAS-1799959-63-9 | 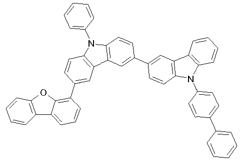 | CAS-1799959-64-0 |
| 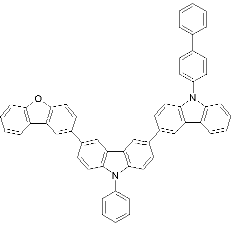 | CAS-1799959-66-2 | 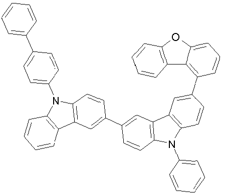 | CAS-1799959-67-3 |
| 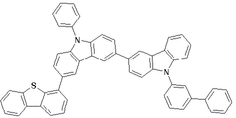 | CAS-1799959-68-4 | 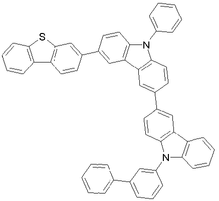 | CAS-1799959-69-5 |
| 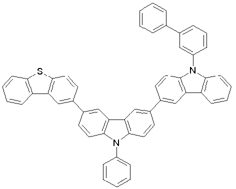 | CAS-1799959-70-8 | 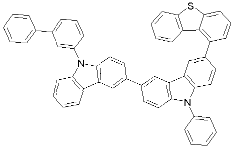 | CAS-1799959-71-9 |
| 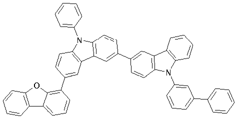 | CAS-1799959-72-0 | 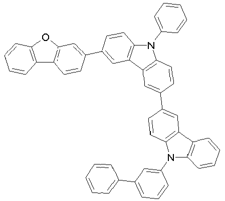 | CAS-1799959-73-1 |
| 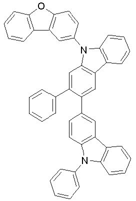 | CAS-1428635-33-9 | 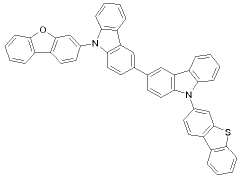 | CAS-1890157-93-3 |
| 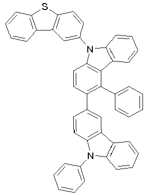 | CAS-1428635-40-8 | 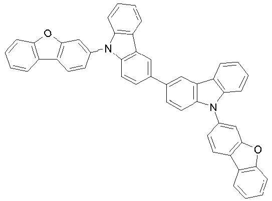 | CAS-1890157-94-4 |
| 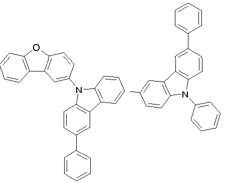 | CAS-1431151-34-6 | 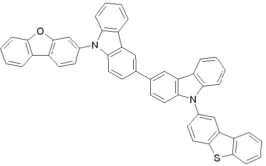 | CAS-1890157-95-5 |
| 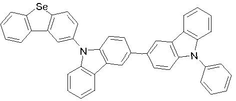 | CAS-1894193-99-7 | 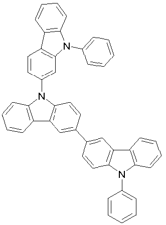 | CAS-1894193-97-5 |
| 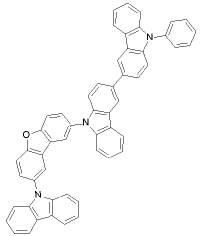 | CAS-1446411-07-9 | 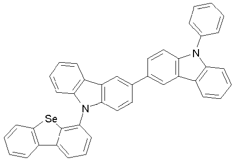 | CAS-1894194-03-6 |
| 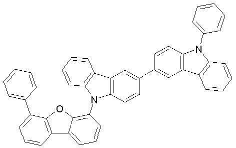 | CAS-1894194-10-5 | 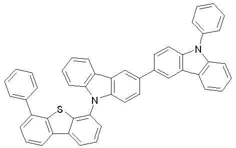 | CAS-1894194-11-6 |
| 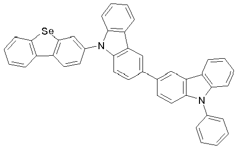 | CAS-1894194-16-1 | 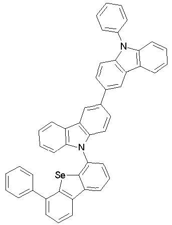 | CAS-1894194-12-7 |
| 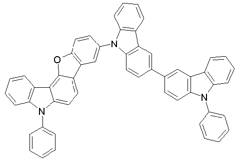 | CAS-1497337-43-5 | 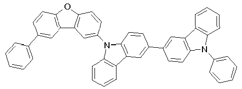 | CAS-1499917-70-2 |
| 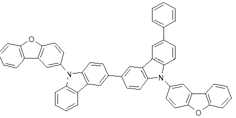 | CAS-1588866-10-7 | 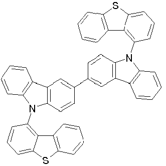 | CAS-1934252-94-4 |
| 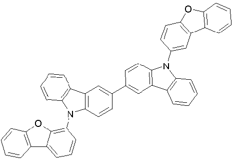 | CAS-1598389-99-1 | 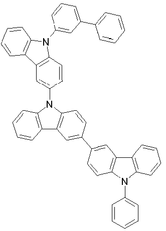 | CAS-1943719-77-4 |
| 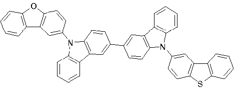 | CAS-1613752-14-9 | 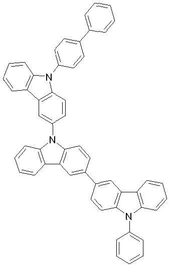 | CAS- 1943719-78-5 |
| 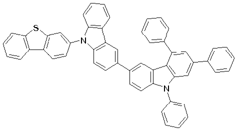 | CAS-2018307-45-2 | 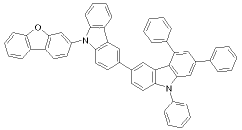 | CAS-2018307-44-1 |
| 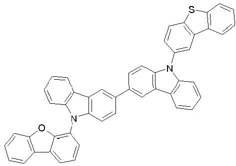 | CAS-1643479-80-4 | 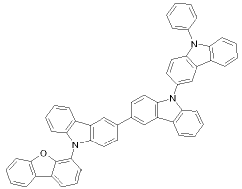 | CAS-1643479-84-8 |
| 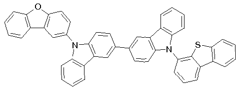 | CAS-1643479-88-2 | 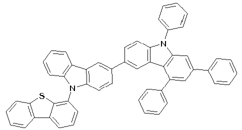 | CAS-2018307-52-1 |
| 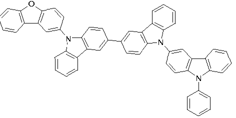 | CAS-1643480-02-7 | 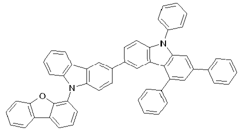 | CAS-2018307-51-0 |
| 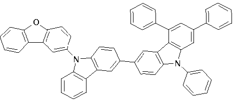 | CAS-2018307-53-2 | 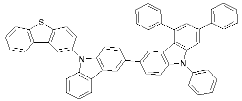 | CAS-2018307-54-3 |
| 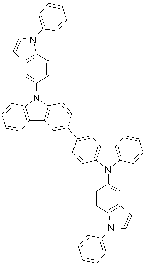 | CAS-1656982-32-9 | 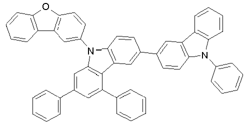 | CAS-2018307-80-5 |
| 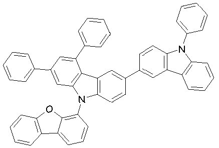 | CAS-2018307-79-2 | 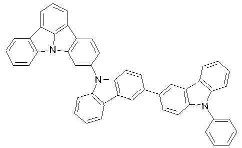 | CAS- 1799483-31-0 |
| 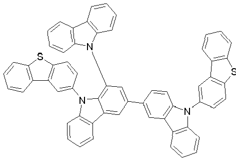 | CAS-1704071-33-9 | 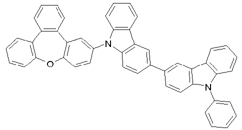 | CAS-1792238-01-7 |
| 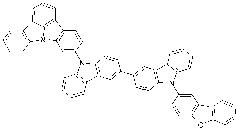 | CAS-1799483-43-4 | 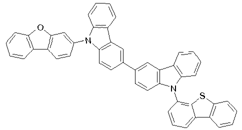 | CAS-2020391-63-1 |
| 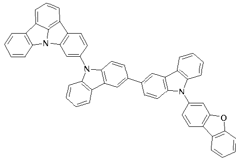 | CAS-1799483-44-5 | 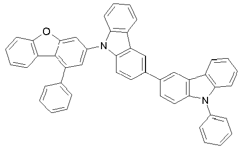 | CAS-2020391-71-1 |
| 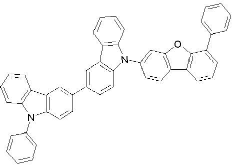 | CAS-2020391-73-3 | 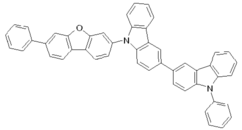 | CAS-2020391-72-2 |
| 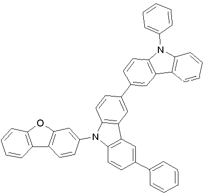 | CAS-2020391-75-5 | 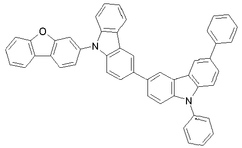 | CAS-2020391-74-4 |
| 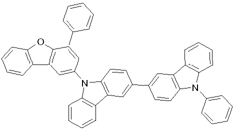 | CAS-2079874-13-6 | 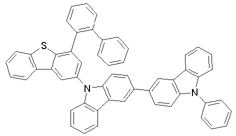 | CAS-2075738-96-2 |
| 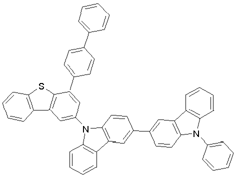 | CAS-2075738-98-4 | 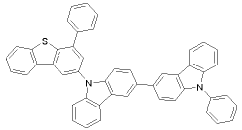 | CAS-2075738-97-3 |
| 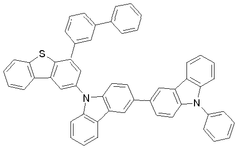 | CAS-2075738-99-5 | 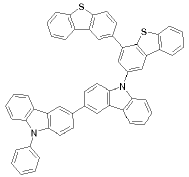 | CAS-2075739-04-5 |
| 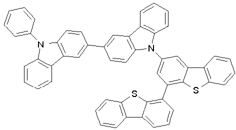 | CAS-2075739-05-6 | 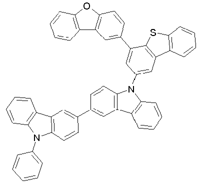 | CAS-2075739-06-7 |
| 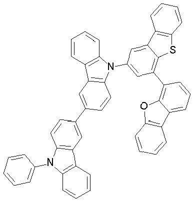 | CAS-2075739-07-8 | 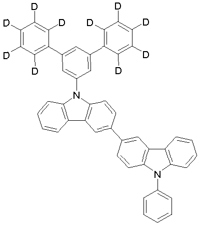 | |
| 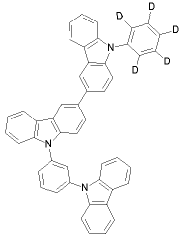 | | 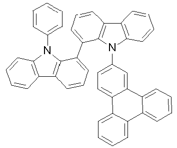 | |
| 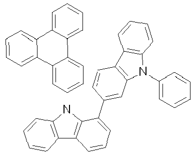 | | 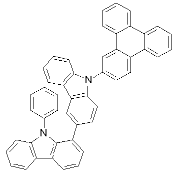 | |
| 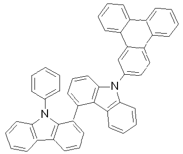 | | 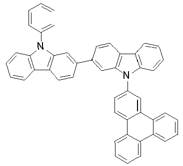 | |
| 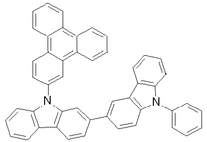 | | 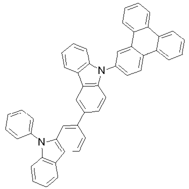 | |
| 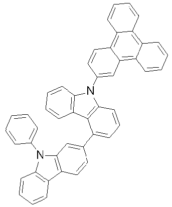 | | 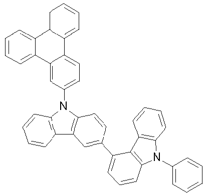 | |
| 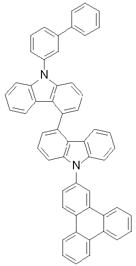 | | 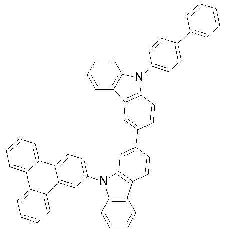 | |
| 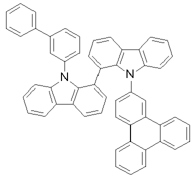 | | | |

Weitere geeignete Verbindungen, die als Co-Host zusammen mit den Verbindungen der Formel (1) eingesetzt werden können, sind die Verbindungen der folgenden Formel (9), 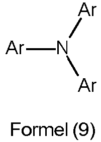 wobei Ar gleich oder verschieden bei jedem Auftreten die oben genannten Bedeutungen aufweist. Dabei ist es bevorzugt, wenn mindestens eine Gruppe Ar für eine 4-Fluorenylgruppe, 4-Spirobifluorenylgruppe, 1-Dibenzofuranylgruppe oder 1-Dibenzothienylgruppe steht, welche jeweils optional durch einen oder mehrere Reste R substituiert sein kann. Beispiele für geeignete Gruppen Ar in der Verbindung der Formel (9) sind die Gruppen der oben abgebildeten Formeln Ar-1 bis Ar-75.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele für phosphoreszierende Verbindungen können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439, WO 2018/011186 und WO 2018/041769 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Beispiele für phosphoreszierende Dotanden sind nachfolgend aufgeführt.

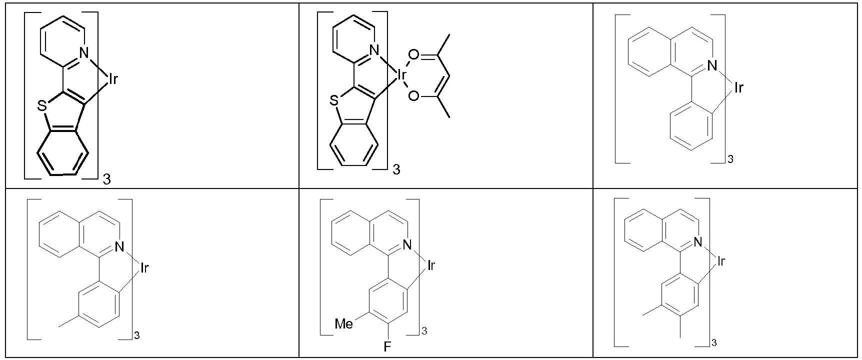

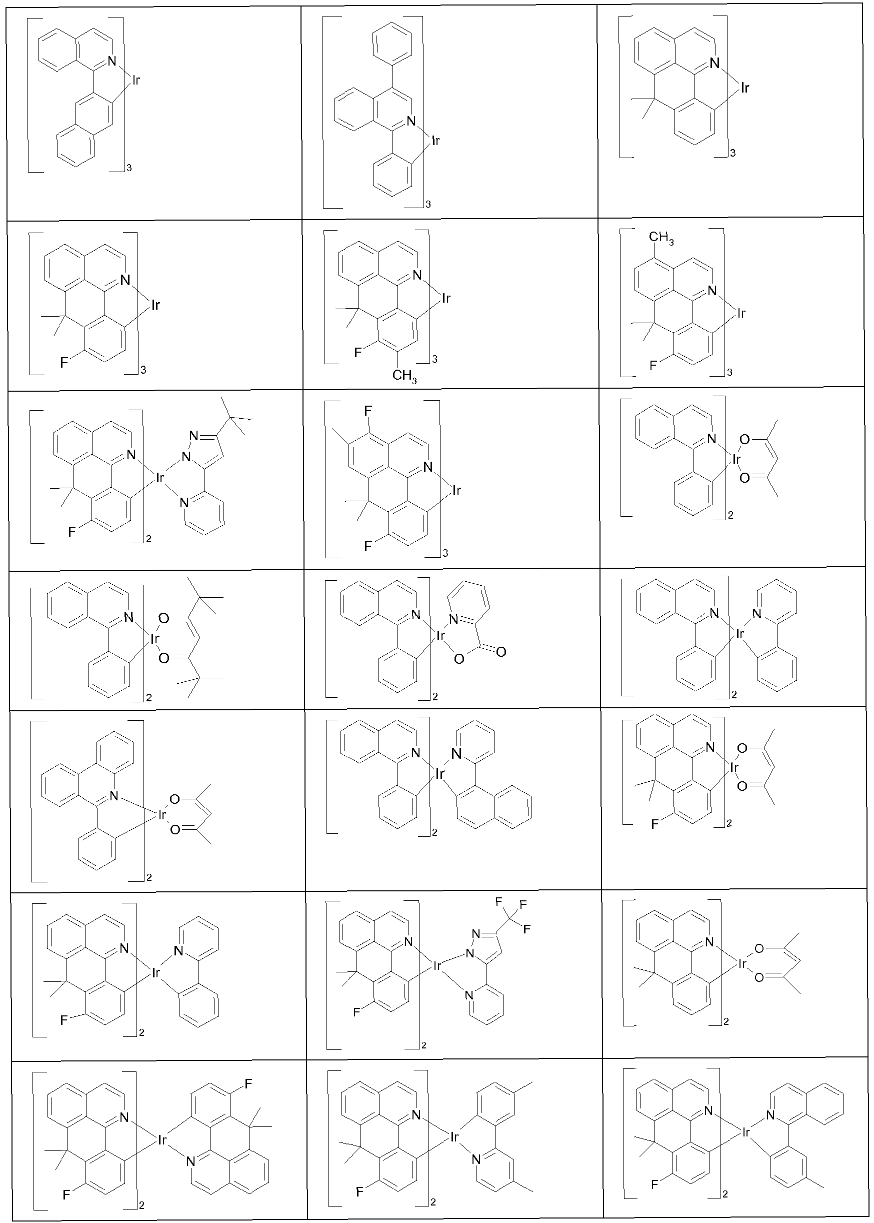

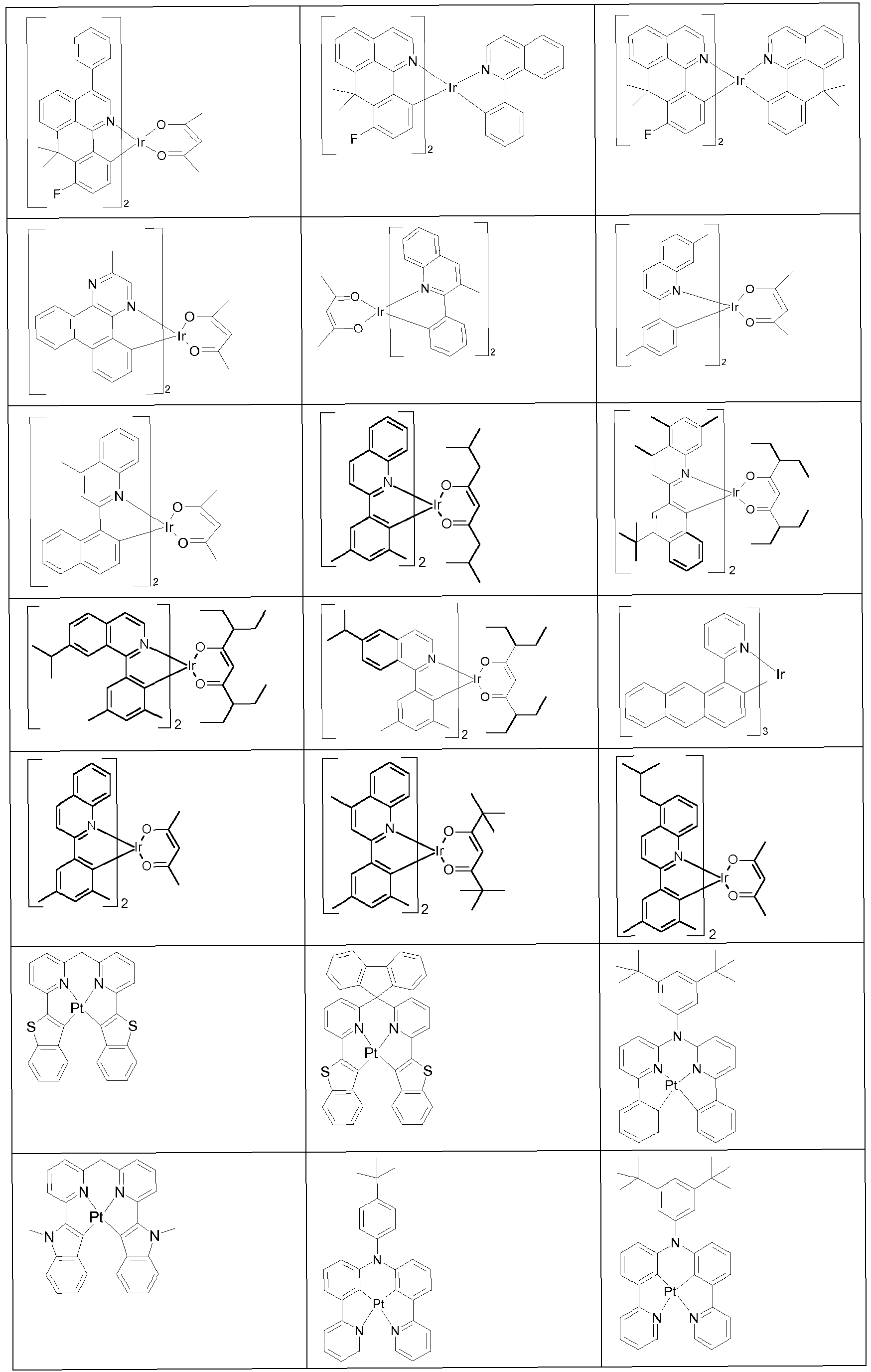

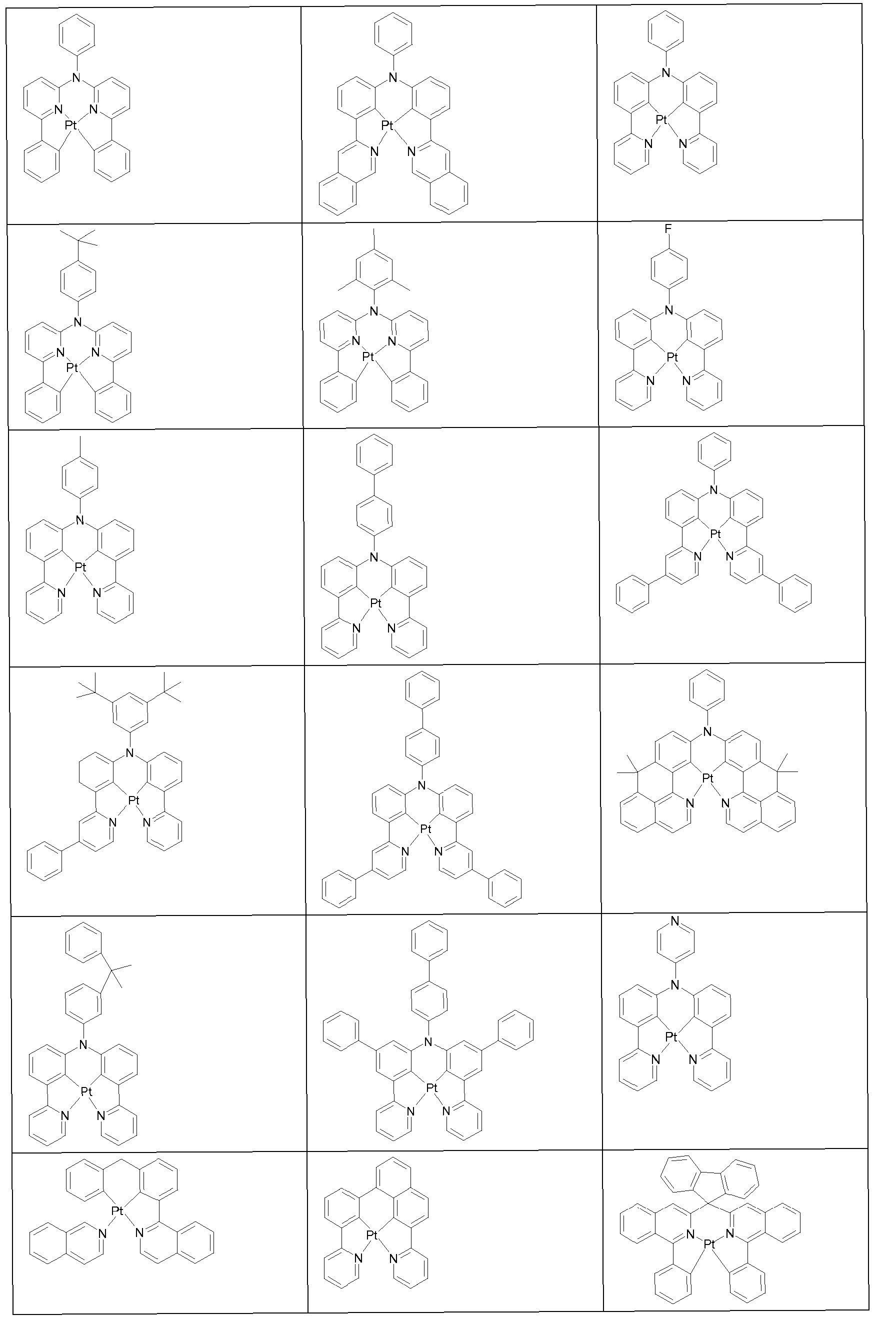

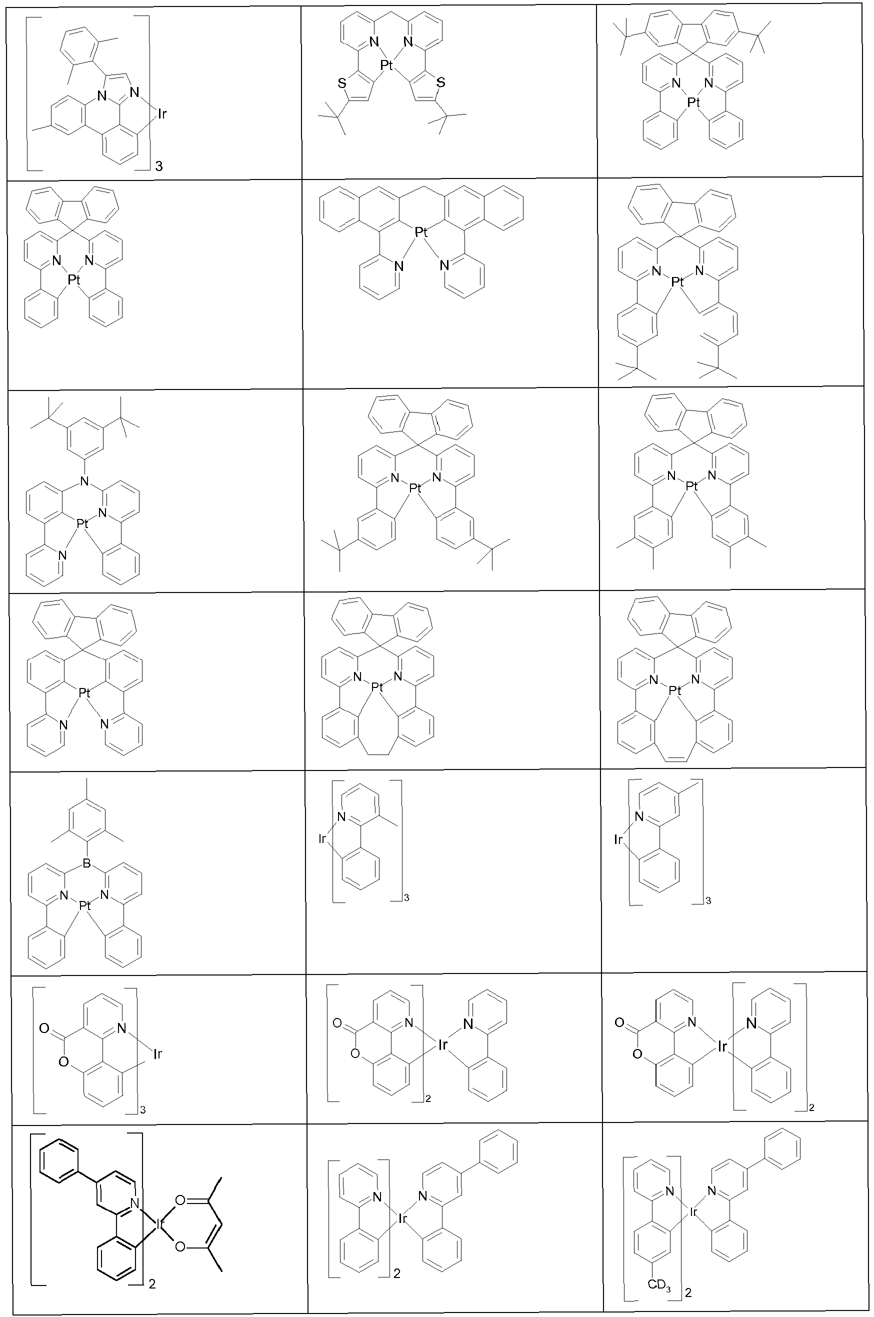

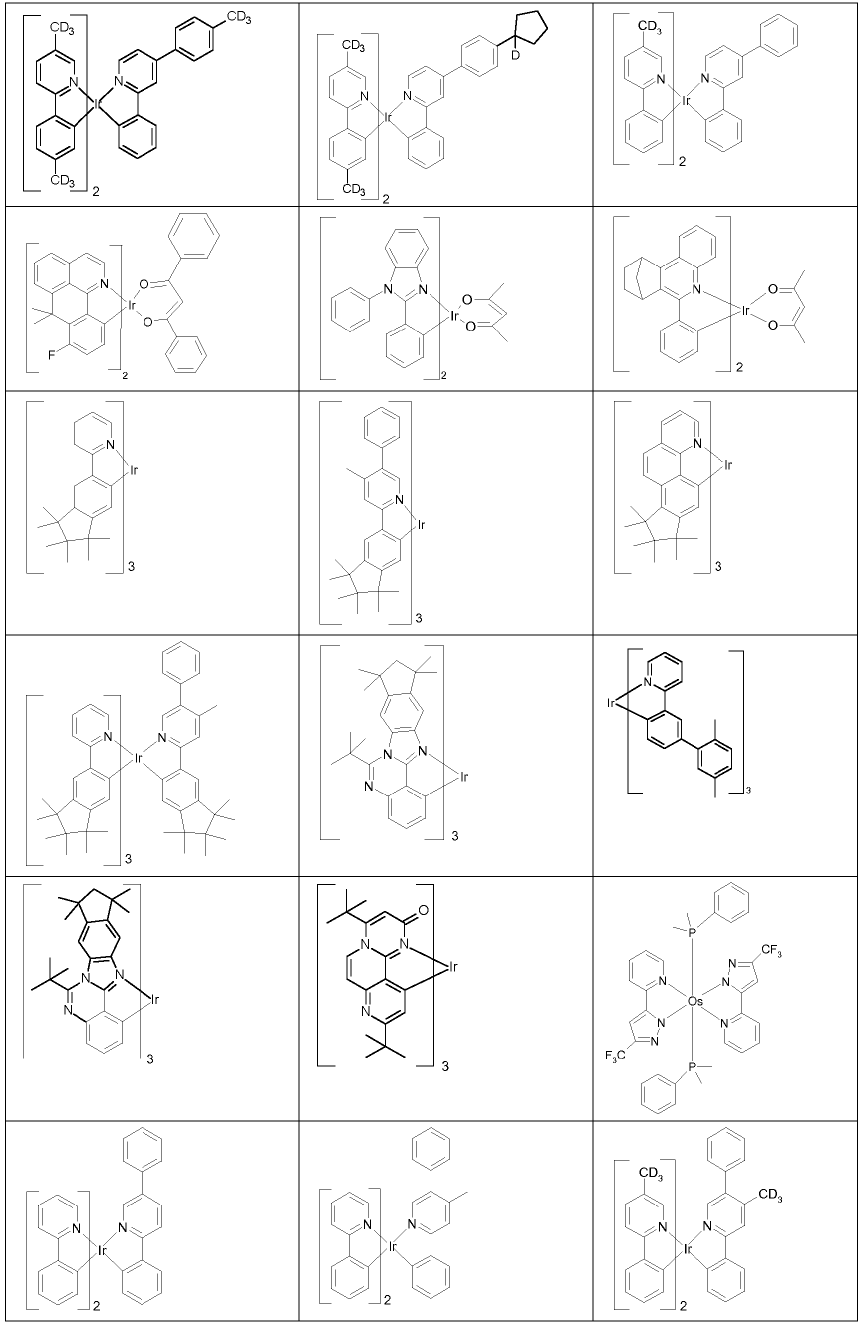

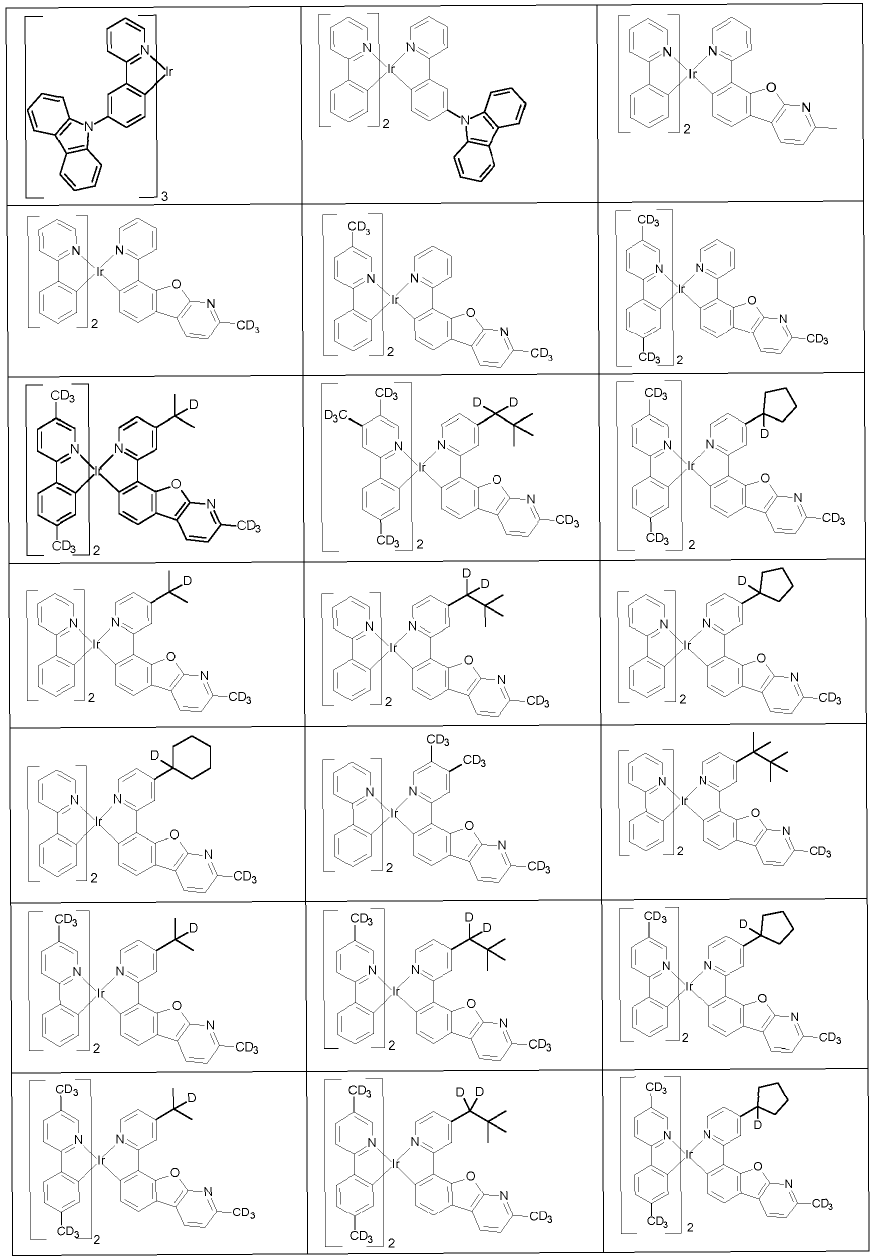

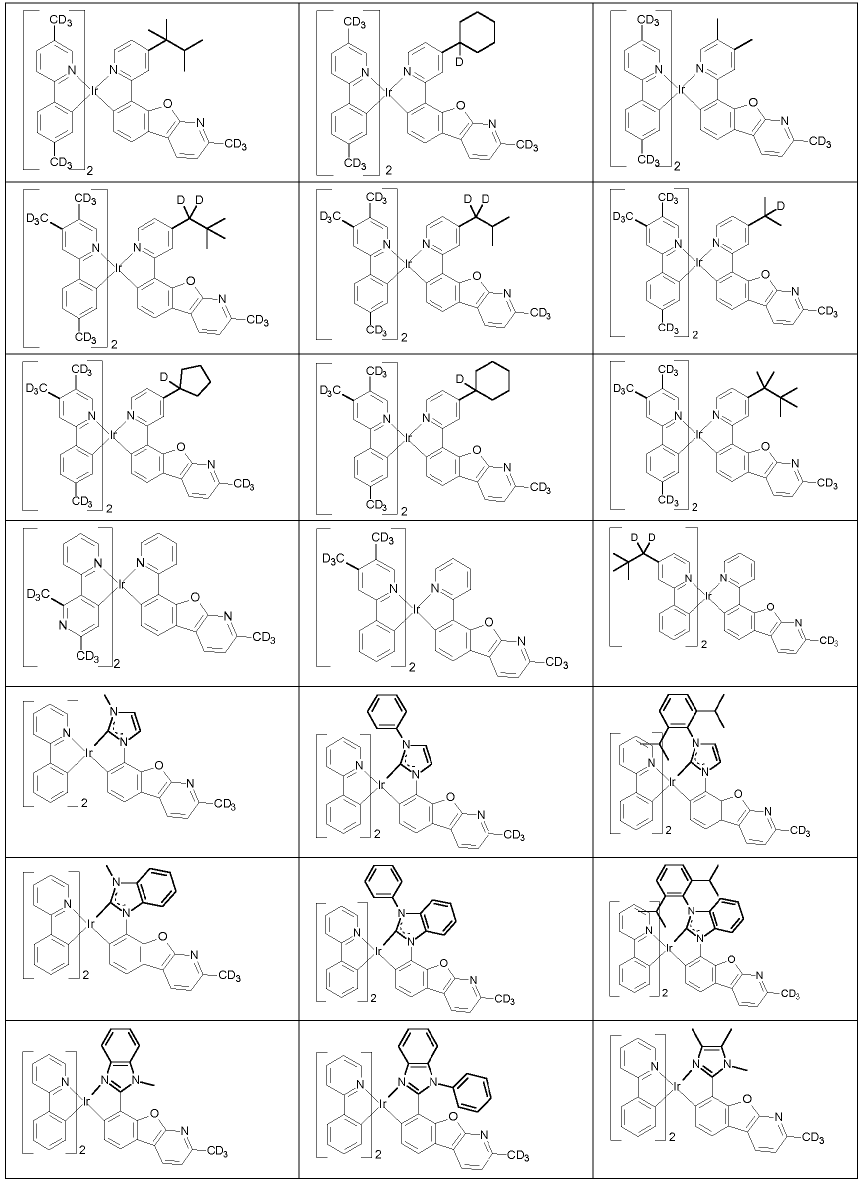

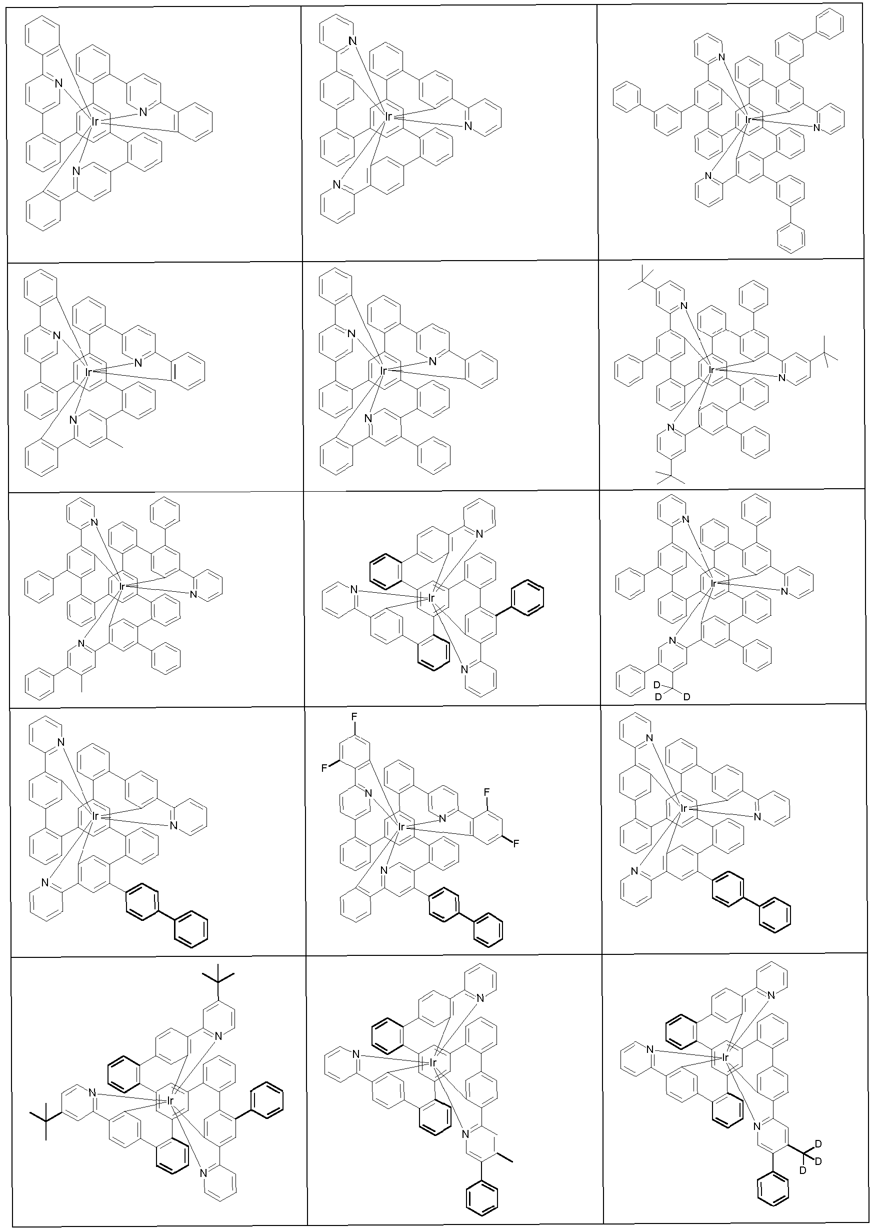

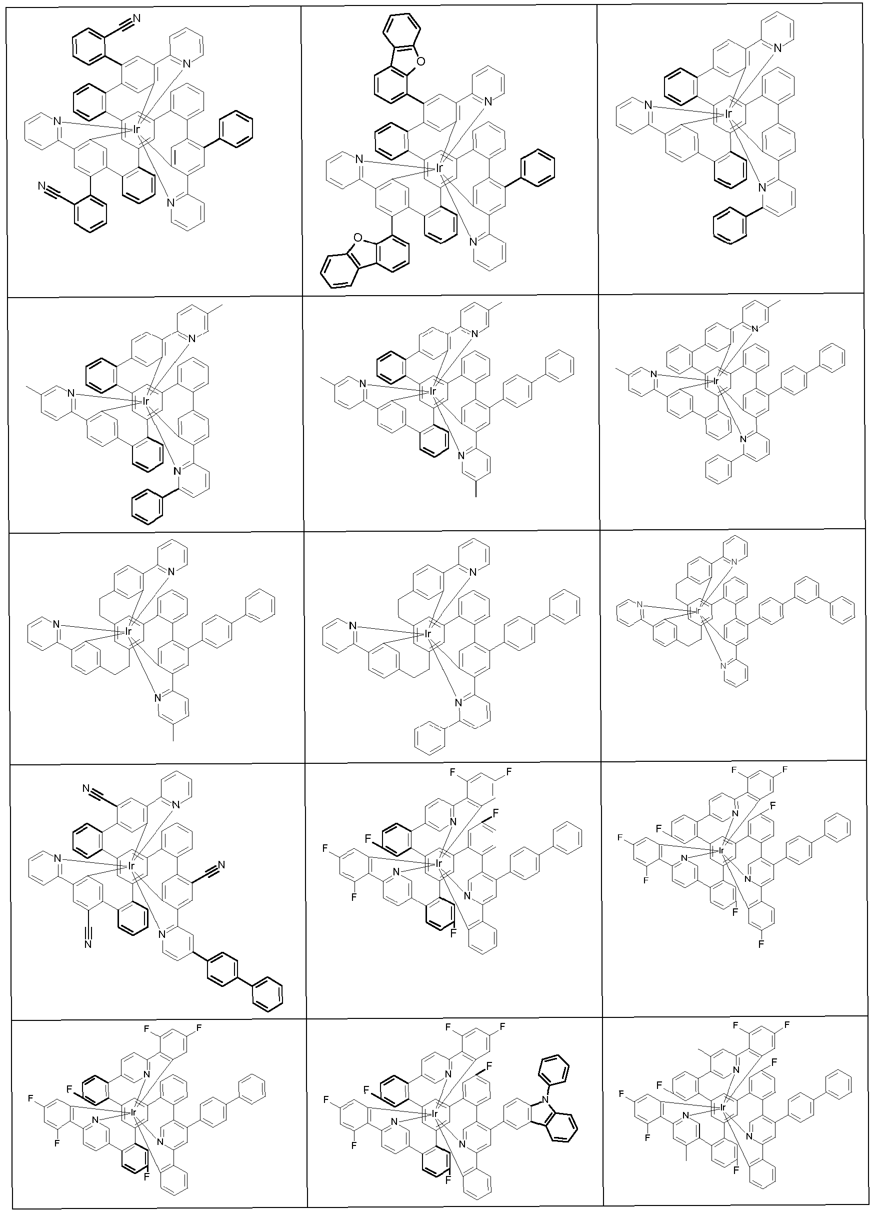

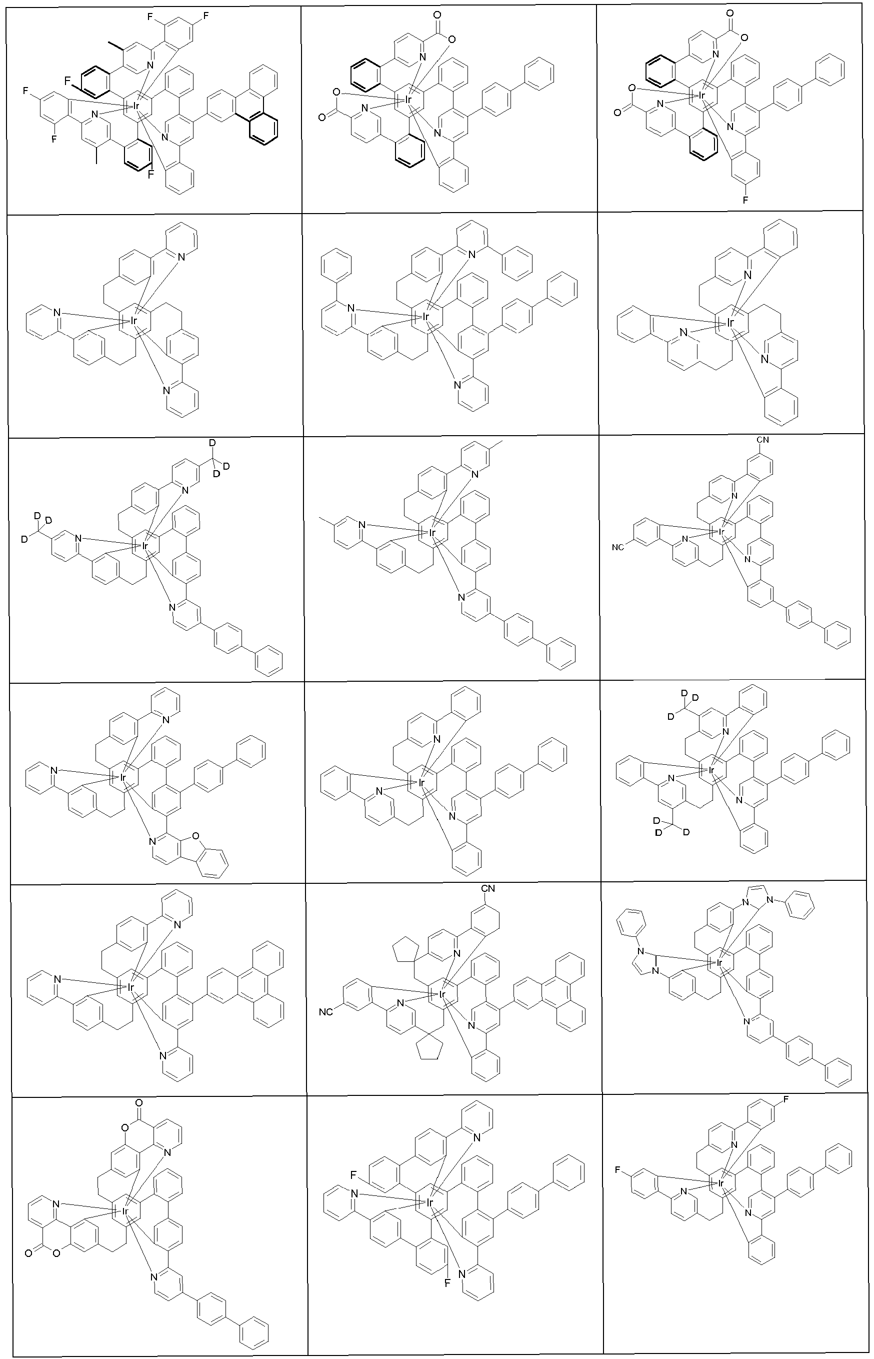

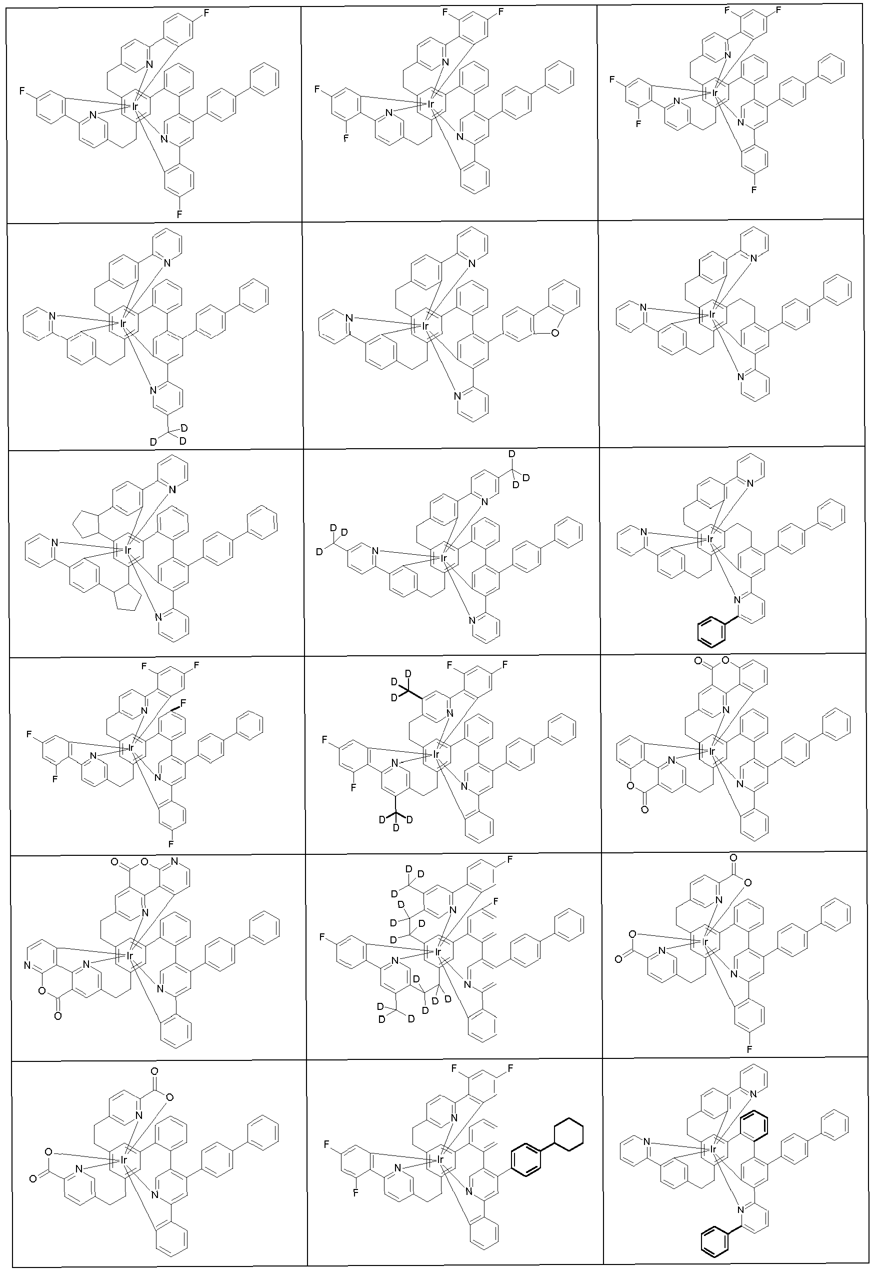

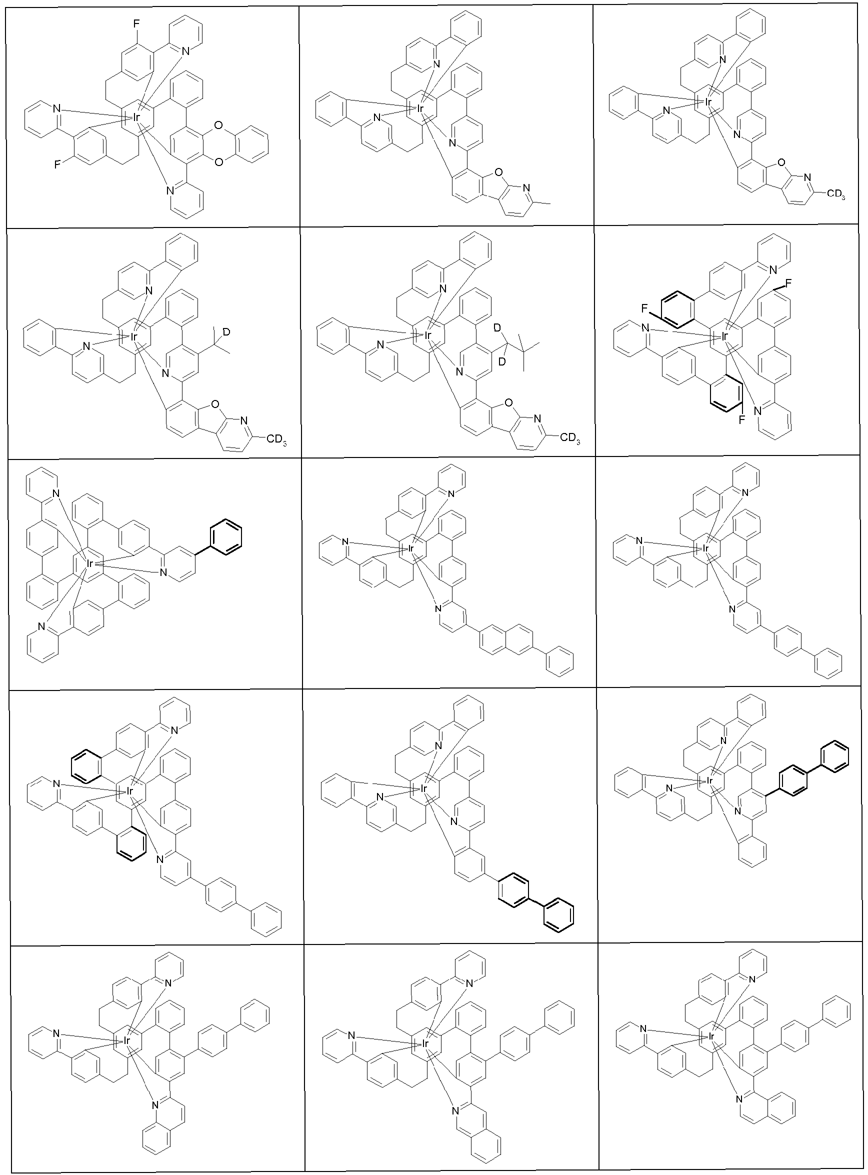

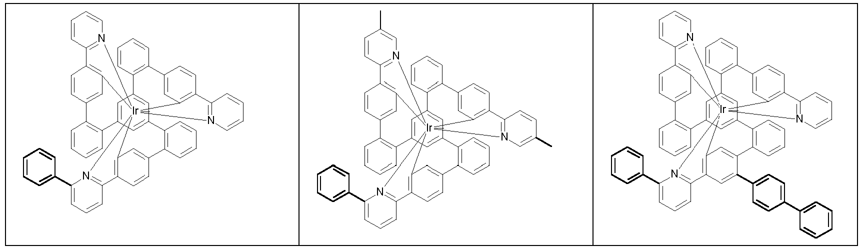

In einer weiteren Ausführungsform der Erfindung handelt es sich bei der organischen Elektrolumineszenzvorrichtung um eine Vorrichtung, wie sie z. B. in WO 98/24271, US 2011/0248247 und US 2012/0223633 beschrieben ist. In diesen mehrfarbigen Display-Bauteilen wird eine zusätzliche blaue Emissionsschicht vollflächig auf alle Pixel, auch diejenigen mit einer von Blau verschiedenen Farbe, aufgedampft.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den Verbindungen gemäß Formel (1) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen OLEDs weisen eine sehr gute Lebensdauer auf und insbesondere eine verbesserte Lebensdauer im Vergleich zu ähnlichen Verbindungen, welche jedoch statt des Benzindenocarbazol-Grundgerüsts ein Indenocarbazol-Grundgerüst aufweisen. Dabei bleiben die weiteren elektronischen Eigenschaften der OLED, wie Effizienz oder Betriebsspannung, mindestens gleich gut. Dies gilt insbesondere auch, wenn die Verbindungen der Formel (1) als einziges Hostmaterials ("single host") eingesetzt werden und nicht in Mischung mit einem oder mehreren weiteren Hostmaterialien. Dies ist ein überraschendes Ergebnis, da ähnliche Verbindungen bei Verwendung als einziges Hostmaterial zu schlechteren Ergebnissen führen als bei Verwendung als Mixed Host. Die Möglichkeit der Verwendung der Verbindung der Formel (1) als einziges Hostmaterial ohne eine Verschlechterung der Deviceergebnisse stellt einen signifikanten Vorteil bei der Herstellung der OLED dar.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Vorrichtungen herstellen.

### Beispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können von ALDRICH bzw. ABCR bezogen werden. Die bei den nicht kommerziell erhältlichen Edukten angegeben Nummern sind die entsprechenden CAS-Nummern.

### a) (2-Chlorphenyl)-(11,11-dimethyl-11H-benzo[a]fluoren-9-yl)-amin

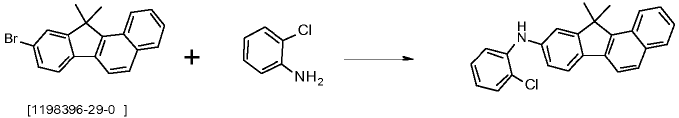

47 g (145 mmol) 9-Brom-11,11-dimethyl-11H-benzo[a]fluoren, 16.8 g (159 mmol) 2-Chloranilin, 41.9 g (436.2 mmol) Natrium-tert-butylat und 1.06 (1.45 mmol) Pd(dppf)Cl₂ werden in 500 mL Toluol gelöst und 5 h unter Rückfluss gerührt. Das Reaktionsgemisch wird auf Raumtemperatur abgekühlt, mit Toluol erweitert und über Celite filtriert. Das Filtrat wird im Vakuum eingeengt und der Rückstand aus Toluol/Heptan kristallisiert. Das Produkt wird als farbloser Feststoff isoliert. Ausbeute: 33 g (89 mmol), 70% d. Th..

Analog können folgende Verbindungen hergestellt werden:

| | **Edukt 1** | **Edukt 2** | **Produkt** | **Ausbeute** |
|---|---|---|---|---|
| 1a | 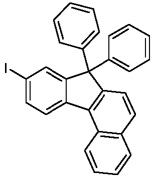 [1804905-31-4 ] | 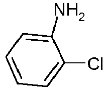 | 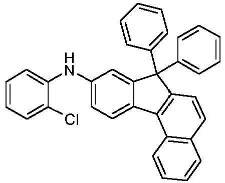 | 79% |
| 2a | 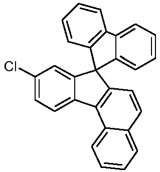 [1800333-59-8 ] | 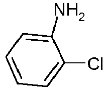 | 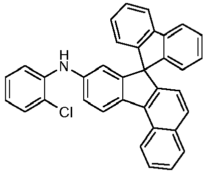 | 77% |
| 3a | 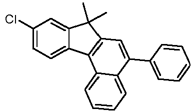 [1263204-40-5 ] | 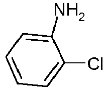 | 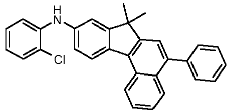 | 78% |
| 1d | 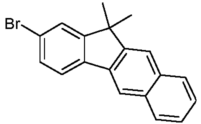 [1198396-39-2 ] | 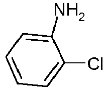 | 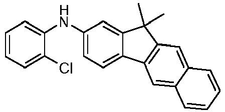 | 79% |
| 4a | 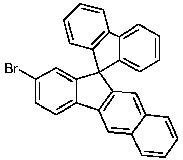 | 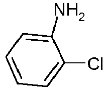 | 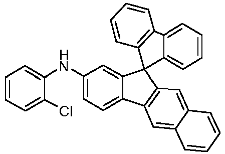 | 74% |
| 1f | 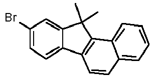 [1198396-29-0 ] | 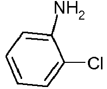 | 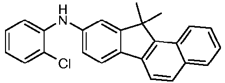 | 81% |
| 5a | 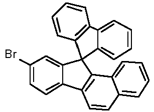 [1198396-35-8 ] | 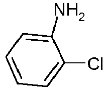 | 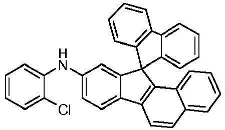 | 78% |
| 6a | 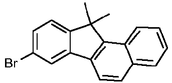 [1674335-13-7 ] | 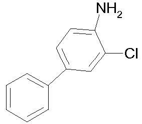 | 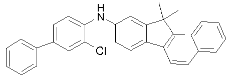 | 77% |

### b) Cyclisierung

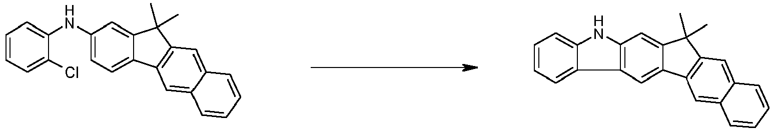

48 g (129 mmol) (2-Chlorphenyl)-(11,11-dimethyl-11H-benzo[a]fluoren-9-yl)-amin, 53 g (389 mmol) Kaliumcarbonat, 4.5 g (12 mmol) Tricyclohexylphosphintetrafluoroborat, 1.38 g (6 mmol) Palladium(II)acetat und 3.3 g (32 mmol) Pivalinsäure werden in 500 mL Dimethylacetamid suspendiert und 6 h unter Rückfluss gerührt. Nach Erkalten wird die Reaktionsmischung mit 300 mL Wasser und 400 mL Dichlormethan zugegeben. Man rührt 30 min., trennt die organische Phase ab, filtriert diese über ein kurzes Celite-Bett und entfernt dann das Lösungsmittel im Vakuum. Das Rohprodukt wird mit Toluol heiß extrahiert und aus Toluol umkristallisiert. Das Produkt wird als beigefarbener Feststoff isoliert. Ausbeute: 34 g (102 mmol), 78% d. Th..

Analog können folgende Verbindungen hergestellt werden:

| | **Edukt** | **Produkt** | **Ausbeute** |
|---|---|---|---|
| | | | |
| 1b | 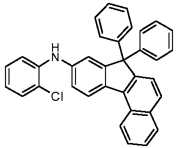 | 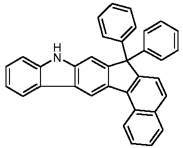 | 79% |
| 2b | 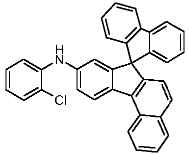 | 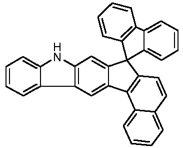 | 77% |
| 3b | 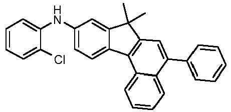 | 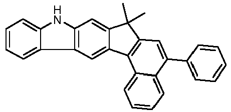 | 78% |
| 4b | 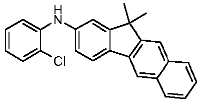 | 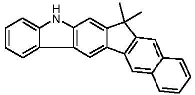 | 75% |
| 5b | 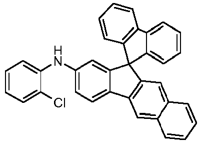 | 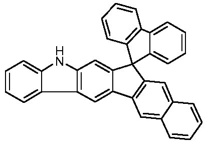 | 78% |
| 6b | 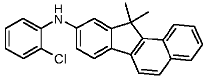 | 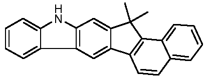 | 73% |
| 7b | 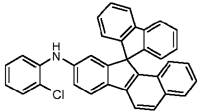 | 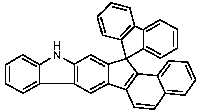 | 71% |
| 8b | 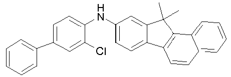 | 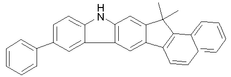 | 76% |

### c) 11,11-Dimethyl-3-(2-nitrophenyl)-11H-benzo[b]fluoren

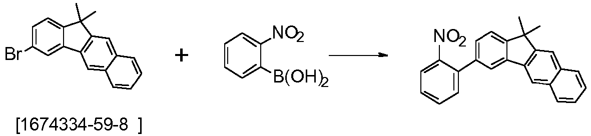

Eine gut gerührte, entgaste Suspension aus 59 g (183.8 mmol) 2-Nitrobenzolboronsäure, 54 g (184 mmol) 3-Brom-11,11-dimethyl-11H-benzo-[b]fluoren und 66.5 g (212.7 mmol) Kaliumcarbonat in einem Gemisch aus 250 mL Wasser und 250 mL THF wird mit 1.7 g (1.49 mmol) Pd(PPh₃)₄ versetzt und 17 h unter Rückfluss erhitzt. Nach Erkalten wird die organische Phase abgetrennt, dreimal mit 200 ml Wasser und einmal mit 200 ml gesättigter, wässriger Kochsalzlösung gewaschen, über Magnesiumsulfat getrocknet und zur Trockene einrotiert. Der graue Rückstand wird aus Hexan umkristallisiert. Die ausgefallenen Kristalle werden abgesaugt, mit etwas MeOH gewaschen und im Vakuum getrocknet. Ausbeute: 53 g (146 mmol), 80% d. Th..

Analog können folgende Verbindungen hergestellt werden:

| | **Edukt 1** | **Edukt 2** | **Produkt** | **Ausbeute** |
|---|---|---|---|---|
| 1c | 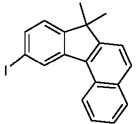 [1927921-26-3 ] | 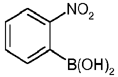 | 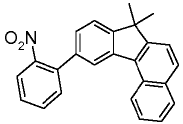 | 74% |
| 2c | 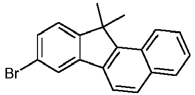 [1674335-13-7 ] | 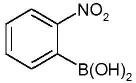 | 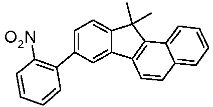 | 77% |

### d) Carbazolsynthese

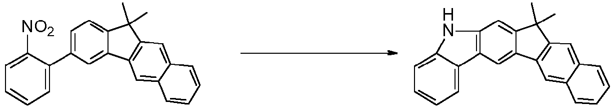

Eine Mischung aus 87 g (240 mmol) 11,11-Dimethyl-3-(2-nitrophenyl)-11H-benzo[b]fluoren und 290.3 ml (1669 mmol) Triethylphosphit wird 12 h unter Rückfluss erhitzt. Anschließend wird das restliche Triethylphosphit abdestilliert (72-76 °C / 9 mm Hg). Der Rückstand wird mit Wasser/MeOH (1:1) versetzt, der Feststoff abfiltriert und umkristallisiert. Ausbeute: 58 g (176 mmol), 74 % d. Th..

Analog können folgende Verbindungen hergestellt werden:

| | **Edukt** | **Produkt** | **Ausbeute** |
|---|---|---|---|
| 1d | 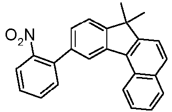 | 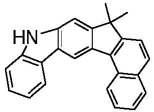 | 79% |
| 2d | 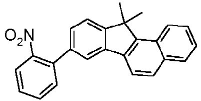 | 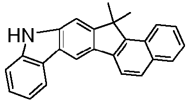 | 76% |

### e) Nukleophile Substitution

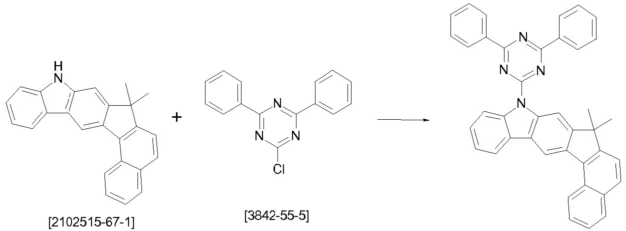

4.2 g (106 mmol) NaH (60%ig in Mineralöl) werden in 300 mL Dimethylformamid unter Schutzatmosphäre gelöst. 35 g (106 mmol) 7,9-Dihydro-7,7-dimethyl-benz[6,7]indeno[2,1-b]carbazol werden in 250 mL DMF gelöst und zu der Reaktionsmischung getropft. Nach 1 h bei Raumtemperatur wird eine Lösung von 2-Chlor-4,6-diphenyl-[1,3,5]triazin (34.5 g, 0.122 mol) in 200 mL THF zugetropft. Das Reaktionsgemisch wird 12 h bei Raumtemperatur gerührt und dann auf Eis gegossen. Der dabei ausgefallene Feststoff wird nach Erwärmen auf Raumtemperatur filtriert und mit Ethanol und Heptan gewaschen. Der Rückstand wird mit Toluol heiß extrahiert, aus Toluol/n-Heptan umkristallisiert und abschließend im Hochvakuum sublimiert. Die Reinheit beträgt 99.9%. Ausbeute 39 g (69 mmol), 66 % d. Th..

Analog können folgende Verbindungen hergestellt werden:

| | **Edukt 1** | **Edukt 2** | **Produkt** | **Ausbeute** |
|---|---|---|---|---|
| 1e | 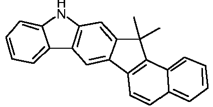 [213765-59-7] | 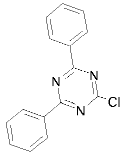 [3842-55-5] | 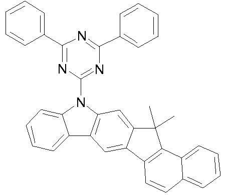 | 60% |
| 2e | 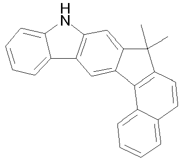 [2102515-67-1] | 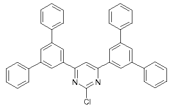 1384480-21-0 | 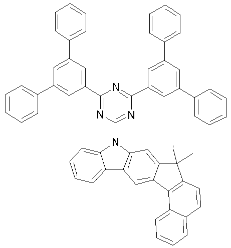 | 61% |
| 3e | 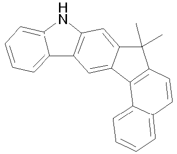 [2102515-67-1] | 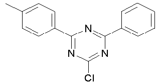 92853-85-5 | 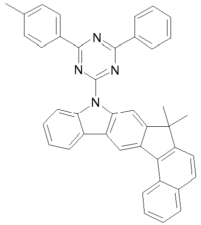 | 57% |
| 4e | 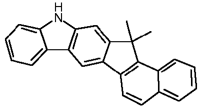 [213765-59-7] | 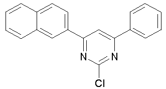 1260393-65-4 | 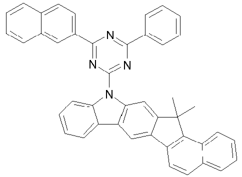 | 60% |
| | | | | |
| 6e | 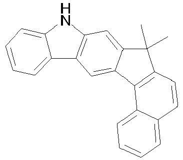 [2102515-67-1] | 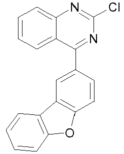 [1616499-38-7] | 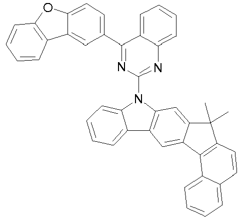 | 72% |
| 7e | 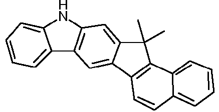 [213765-59-7] | 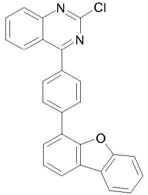 [1403252-58-3] | 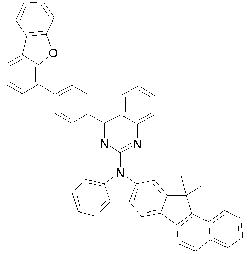 | 74% |
| 8e | 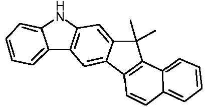 [213765-59-7] | 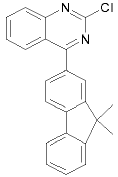 [1373265-66-7] | 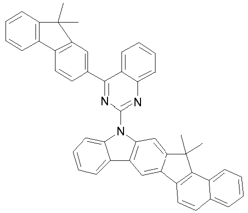 | 62% |
| 9e | 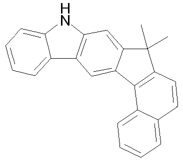 [2102515-67-1] | 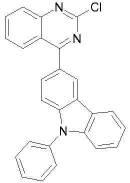 [1373317-91-9] | 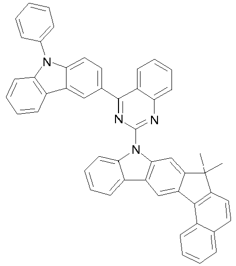 | 67% |
| 10e | 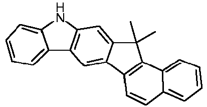 [213765-59-7] | 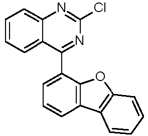 [14003252-55-0] | 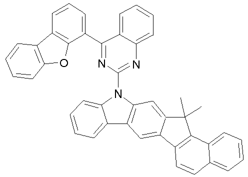 | 61% |
| 12e | 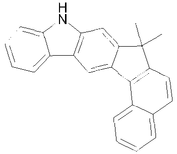 [2102515-67-1] | 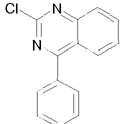 [29874-83-7] | 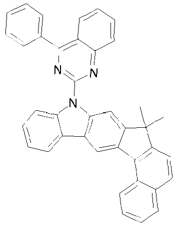 | 67% |
| 13e | 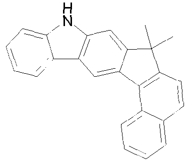 [2102515-67-1] | 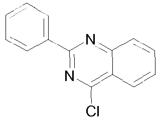 [6484-25-9] | 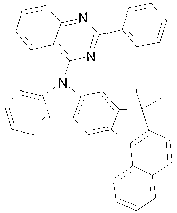 | 62% |
| 15e | 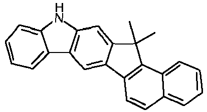 [213765-59-7] | 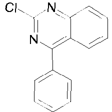 [29874-83-7] | 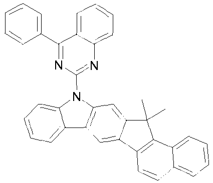 | 63% |
| 16e | 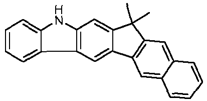 | 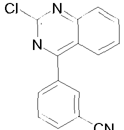 [1292317-90-8] | 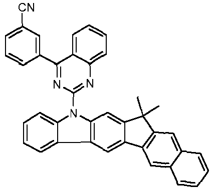 | 60% |
| 17e | 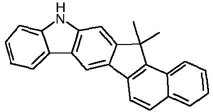 [213765-59-7] | 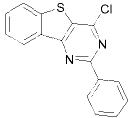 [900463-54-9 ] | 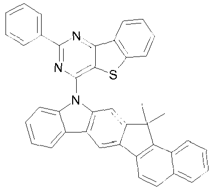 | 61% |
| 18e | 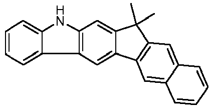 | 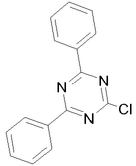 [3842-55-5] | 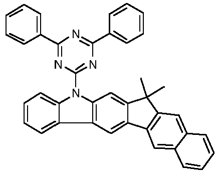 | 71% |

### f) Bromierung

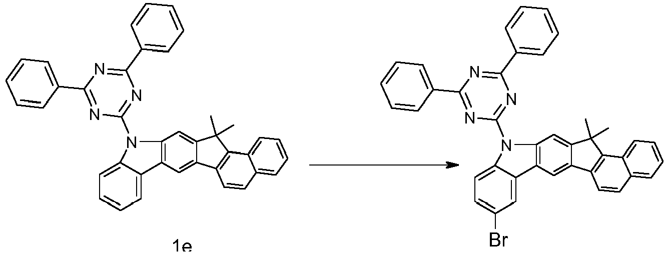

129 g (230 mmol) der Verbindung 1e werden in 1000 mL THF vorgelegt. Anschließend tropft man unter Lichtausschluss bei -15 °C eine Lösung aus 41.7 g (234.6 mmol) NBS in 500 ml THF zu, lässt auf RT kommen und rührt 4 h weiter bei dieser Temperatur. Anschließend wird die Mischung mit 150 mL Wasser versetzt und mit CH₂Cl₂ extrahiert. Die organische Phase wird über MgSO₄ getrocknet und die Lösungsmittel im Vakuum entfernt. Das Produkt wird mit Hexan heiß ausgerührt und abgesaugt. Ausbeute: 78.3 g (121 mmol), 53 % der Theorie; Reinheit nach ¹H-NMR ca. 97 %.

Analog können folgende Verbindungen hergestellt werden:

| | **Edukt** | **Produkt** | **Ausbeute** |
|---|---|---|---|
| 1f | 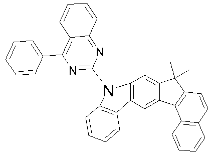 | 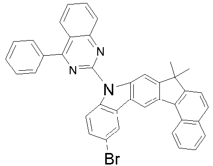 | 56% |
| 2f | 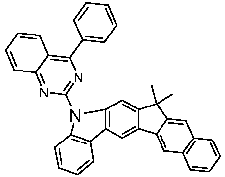 | 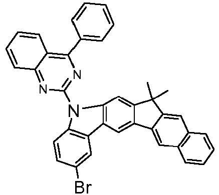 | 61% |

### g) Suzuki-Reaktion

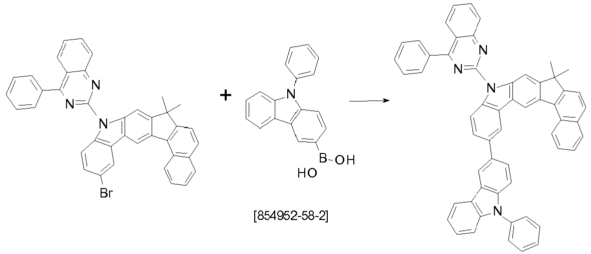

25.8 g (42.12 mmol) der Verbindung 1f, 13.4 g (47 mmol) 9-Phenyl-carbazol-3-boronsäure und 29.2 g Rb₂CO₃ werden in 250 mL p-Xylol suspendiert. Zu dieser Suspension werden 0.95 g (4.2 mmol) Pd(OAc)₂ und 12.6 ml einer 1M Tri-tert-butylphosphinlösung in Toluol gegeben. Die Reaktionsmischung wird 16 h unter Rückfluss erhitzt. Nach Erkalten wird die organische Phase abgetrennt, dreimal mit 200 mL Wasser gewaschen und anschließend zur Trockene eingeengt. Der Rückstand wird mit Toluol heiß extrahiert, aus Toluol umkristallisiert und abschließend im Hochvakuum sublimiert. Die Reinheit beträgt 99.9 %. Ausbeute: 24 g (31 mmol), 70 % d. Th..

Analog können folgende Verbindungen hergestellt werden:

| | **Edukt 1** | **Edukt 2** | **Produkt** | **Ausbeute** |
|---|---|---|---|---|
| 1g | 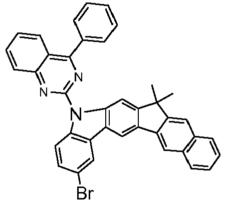 | 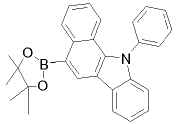 [1493715-37-9 ] | 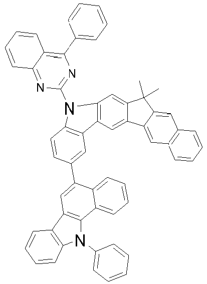 | 60% |
| 2g | 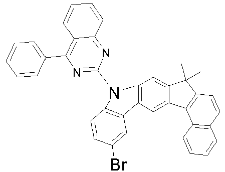 | 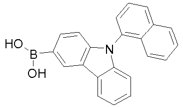 [1133057-97-2 ] | 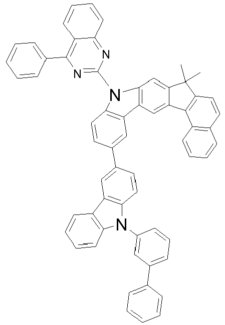 | 61% |

### Herstellung der OLEDs

In den folgenden Beispielen E1 bis E9 (siehe Tabelle 1) wird der Einsatz des erfindungsgemäßen Materials in OLEDs vorgestellt.

**Vorbehandlung für die Beispiele E1-E9:** Glasplättchen, die mit strukturiertem ITO (Indium-Zinn-Oxid) der Dicke 50 nm beschichtet sind, werden vor der Beschichtung mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 2 gezeigt. Die Daten der OLEDs sind in Tabelle 3 aufgelistet.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie EG1:IC2:TER5 (55%:35%:10%) bedeutet hierbei, dass das Material EG1 in einem Volumenanteil von 55%, IC2 in einem Volumenanteil von 35% und TER5 in einem Volumenanteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (SE, gemessen in cd/A) und die externe Quanteneffizienz (EQE, gemessen in %) in Abhängigkeit der Leuchtdichte, berechnet aus Strom-Spannungs-Leuchtdichte-Kennlinien unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 3 bezeichnet die Spannung, die für eine Leuchtdichte von 1000 cd/m² benötigt wird. SE1000 und EQE1000 bezeichnen die Stromeffizienz bzw. die externe Quanteneffizienz, die bei 1000 cd/m² erreicht werden. Als Lebensdauer LD wird die Zeit definiert, nach der die Leuchtdichte bei Betrieb mit konstanter Stromdichte j₀ von der Startleuchtdichte auf einen gewissen Anteil L1 absinkt. Eine Angabe L1=95% in Tabelle 3 bedeutet, dass die in Spalte LD angegebene Lebensdauer der Zeit entspricht, nach der die Leuchtdichte auf 95% ihres Anfangswertes absinkt.

### Verwendung der Verbindungen der Formel (1) als Matrixmaterial in OLEDs

Üblicherweise kommt in der Emissionsschicht von OLEDs eine Mischung aus zwei Hostmaterialien (Matrixmaterialien) zum Einsatz, um eine optimale Ladungsbalance und damit sehr gute Leistungsdaten der OLED zu erreichen. Hinsichtlich einer vereinfachten Herstellung von OLEDs ist eine Reduzierung der unterschiedlichen verwendeten Materialien erstrebenswert. In der Emissionsschicht ist also der Einsatz von lediglich einem Hostmaterial statt einer Mischung aus zwei Hostmaterialien vorteilhaft.

Mit dem Einsatz der erfindungsgemäßen Verbindungen EG1-EG4, EG6, EG7 in den Beispielen E1-E4, E6-E9 als Matrixmaterial in der Emissionsschicht phosphoreszierender roter OLEDs kann gezeigt werden, dass die Verwendung als Einzelmaterial mindestens gleich gute oder verbesserte Leistungsdaten der OLEDs gegenüber einer Mischung mit einem zweiten Hostmaterial IC2 (E2 und E4) liefert. Das stellt aus produktiontechnischer Sicht einen klaren Vorteil dar. Das Beispiel E5 ist ein Referenzbeispiel, das nicht Teil der vorliegenden Erfindung ist.

**Tabelle 1: Aufbau der OLEDs**

| Bsp. | HIL Dicke | HTL Dicke | EBL Dicke | EML Dicke | HBL Dicke | ETL Dicke | EIL Dicke |
|---|---|---|---|---|---|---|---|
| E1 | HATCN | SpMA1 | SpMA3 | EG1:TER | ST2 | ST2:LiQ (50%:50%) | LiQ |
| | 5nm | 125nm | 10nm | (97%:3%) 35nm | 10nm | 30nm | 1nm |
| E2 | HATCN | SpMA1 | SpMA3 | EG1:IC2:TER | ST2 | ST2:LiQ (50%:50%) | LiQ |
| | 5nm | 125nm | 10nm | (72%:25%:3%) 35nm | 10nm | 30nm | 1nm |
| E3 | HATCN | SpMA1 | SpMA3 | EG2:TER | ST2 | ST2:LiQ (50%:50%) | LiQ |
| | 5nm | 125nm | 10nm | (97%:3%) 35nm | 10nm | 30nm | 1nm |
| E4 | HATCN | SpMA1 | SpMA3 | EG2:IC2:TER | ST2 | ST2:LiQ (50%:50%) | LiQ |
| | 5nm | 125nm | 10nm | (32%:65%:3%) 35nm | 10nm | 30nm | 1nm |
| E5 | HATCN | SpMA1 | SpMA3 | EG3:TER | ST2 | ST2:LiQ (50%:50%) | LiQ |
| | 5nm | 125nm | 10nm | (97%:3%) 35nm | 10nm | 30nm | 1nm |
| E6 | HATCN | SpMA1 | SpMA3 | EG4:TER | ST2 | ST2:LiQ (50%:50%) | LiQ |
| | 5nm | 125nm | 10nm | (97%:3%) 35nm | 10nm | 30nm | 1nm |
| E7 | HATCN | SpMA1 | SpMA3 | EG5:TER | ST2 | ST2:LiQ (50%:50%) | LiQ |
| | 5nm | 125nm | 10nm | (97%:3%) 35nm | 10nm | 30nm | 1nm |
| E8 | HATCN | SpMA1 | SpMA3 | EG6:TER | ST2 | ST2:LiQ (50%:50%) | LiQ |
| | 5nm | 125nm | 10nm | (97%:3%) 35nm | 10nm | 30nm | 1nm |
| E9 | HATCN | SpMA1 | SpMA3 | EG7:TER | ST2 | ST2:LiQ (50%:50%) | LiQ |
| | 5nm | 125nm | 10nm | (97%:3%) 35nm | 10nm | 30nm | 1nm |

**Tabelle 2: Strukturformeln der Materialien für die OLEDs**

| | |
|---|---|
| 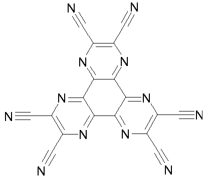 | 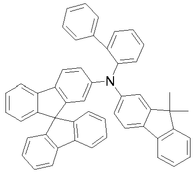 |
| HATCN | SpMA1 |
| 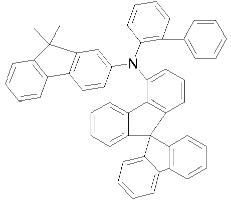 | 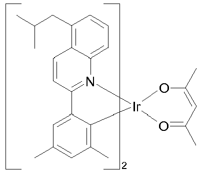 |
| SpMA3 | TER |
| 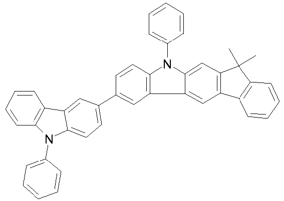 | 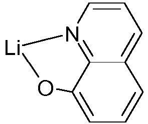 |
| IC2 | LiQ |
| 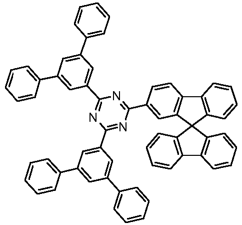 | 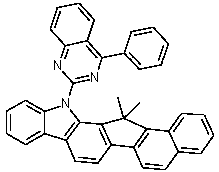 |
| ST2 | EG1 |
| 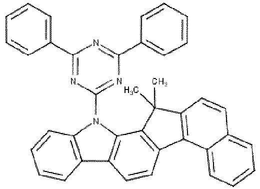 | 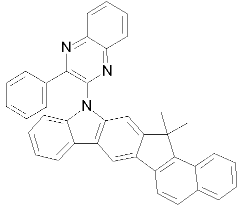 |
| EG2 | EG3 |
| 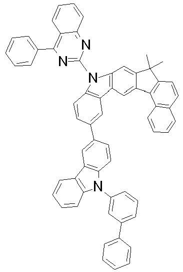 | 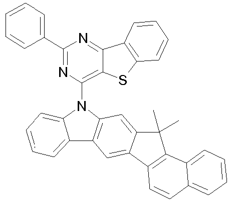 |
| EG4 | EG5 |
| 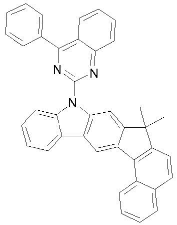 | 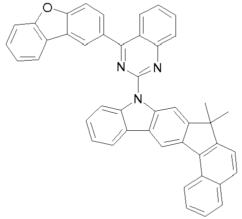 |
| EG6 | EG7 |

**Tabelle 3: Daten der OLEDs**

| Bsp. | U1000 (V) | SE1000 (cd/A) | EQE 1000 (%) | CIE x/y bei 1000 cd/m² | j₀ (mA/cm²) | L1 (%) | LD (h) |
|---|---|---|---|---|---|---|---|
| E1 | 3.9 | 23 | 21 | 0.67/0.33 | 20 | 95 | 1310 |
| E2 | 3.8 | 23 | 20 | 0.66/0.34 | 20 | 95 | 1160 |
| E3 | 3.4 | 23 | 21 | 0.67/0.33 | 20 | 95 | 110 |
| E4 | 3.8 | 24 | 21 | 0.67/0.33 | 20 | 95 | 70 |
| E5 | 3.8 | 22 | 20 | 0.66/0.34 | | | |
| E6 | 3.5 | 23 | 22 | 0.66/0.34 | | | |
| E7 | 3.7 | 22 | 20 | 0.67/0.33 | | | |
| E8 | 3.9 | 23 | 21 | 0.66/0.34 | | | |
| E9 | 3.9 | 22 | 20 | 0.67/0.34 | | | |

## Patentansprüche

1. Organische Elektrolumineszenzvorrichtung enthaltend Anode, Kathode und mindestens eine emittierende Schicht, die mindestens eine phosphoreszierende Verbindung enthält, **dadurch gekennzeichnet, dass** die emittierende Schicht mindestens eine Verbindung gemäß Formel (1) enthält, 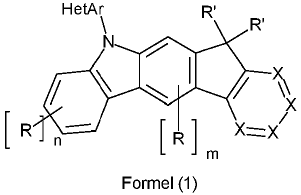 wobei für die verwendeten Symbole und Indizes gilt:
X zwei benachbarte X stehen für eine Gruppe der folgenden Formel (2), und die beiden anderen X stehen für CR, 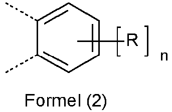 wobei die beiden gestrichelten Bindungen die Verknüpfung dieser Gruppe darstellen;
HetAr ist eine Gruppe gemäß einer der Formeln (HetAr-1d), (HetAr-2a), (HetAr-3a), (HetAr-4a), (HetAr-5a), (HetAr-6a), (HetAr-7a) und (HetAr-8c), 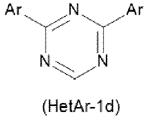 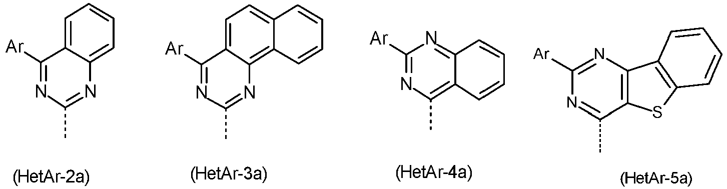 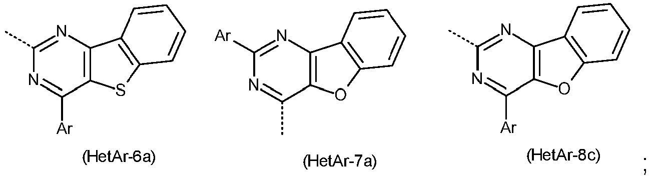
Ar ist gleich oder verschieden bei jedem Auftreten ein aromatisches oder heteroaromatisches Ringsystem mit 5 bis 40 aromatischen Ringatomen, welches mit einem oder mehreren Resten R¹ substituiert sein kann;
R ist bei jedem Auftreten gleich oder verschieden H, D, F, Cl, Br, I, N(R¹)₂, N(Ar')₂, CN, NO₂, OR¹, SR¹, COOR¹, C(=O)N(R¹)₂, Si(R¹)₃, B(OR¹)₂, C(=O)R¹, P(=O)(R¹)₂, S(=O)R¹, S(=O)₂R¹, OSO₂R¹, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹ substituiert sein kann und wobei eine oder mehrere nicht benachbarte CH₂-Gruppen durch Si(R¹)₂, C=O, NR¹, O, S oder CONR¹ ersetzt sein können, oder ein aromatisches oder heteroaromatisches Ringsystem mit 5 bis 60 aromatischen Ringatomen, das jeweils durch einen oder mehrere Reste R¹ substituiert sein kann;
R' ist bei jedem Auftreten gleich oder verschieden eine geradkettige Alkylgruppe mit 1 bis 20 C-Atomen oder eine verzweigte oder cyclische Alkylgruppe mit 3 bis 20 C-Atomen, wobei die geradkettige, verzweigte oder cyclische Alkylgruppe jeweils mit einem oder mehreren Resten R¹ substituiert sein kann und wobei eine oder mehrere nicht benachbarte CH₂-Gruppen durch O ersetzt sein können, oder ein aromatisches oder heteroaromatisches Ringsystem mit 5 bis 40 aromatischen Ringatomen, das jeweils durch einen oder mehrere Reste R¹ substituiert sein kann; dabei können zwei Reste R' auch miteinander ein aromatisches, heteroaromatisches, aliphatisches oder heteroaliphatisches Ringsystem bilden;
Ar' ist bei jedem Auftreten gleich oder verschieden ein aromatisches oder heteroaromatisches Ringsystem mit 5 bis 40 aromatischen Ringatomen, welches mit einem oder mehreren Resten R¹ substituiert sein kann;
R¹ ist bei jedem Auftreten gleich oder verschieden H, D, F, Cl, Br, I, N(R²)₂, CN, NO₂, OR², SR², Si(R²)₃, B(OR²)₂, C(=O)R², P(=O)(R²)₂, S(=O)R², S(=O)₂R², OSO₂R², eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R² substituiert sein kann, wobei eine oder mehrere nicht benachbarte CH₂-Gruppen durch Si(R²)₂, C=O, NR², O, S oder CONR² ersetzt sein können, oder ein aromatisches oder heteroaromatisches Ringsystem mit 5 bis 40 aromatischen Ringatomen, das jeweils durch einen oder mehrere Reste R² substituiert sein kann;
R² ist bei jedem Auftreten gleich oder verschieden H, D, F oder ein aliphatischer, aromatischer oder heteroaromatischer organischer Rest, insbesondere ein Kohlenwasserstoffrest, mit 1 bis 20 C-Atomen, in dem auch ein oder mehrere H-Atome durch F ersetzt sein können;
m ist 0, 1 oder 2;
n ist bei jedem Auftreten gleich oder verschieden 0 oder 1.

2. Organische Elektrolumineszenzvorrichtung nach Anspruch 1, **dadurch gekennzeichnet, dass** die Verbindung der Formel (1) ausgewählt ist aus den Verbindungen gemäß den Formeln (3), (4) oder (5), 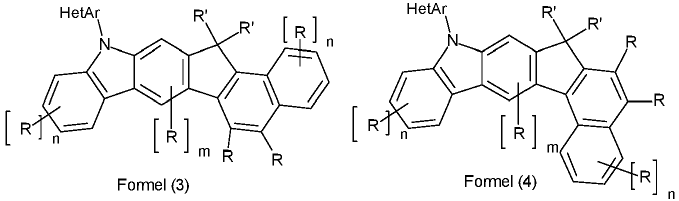 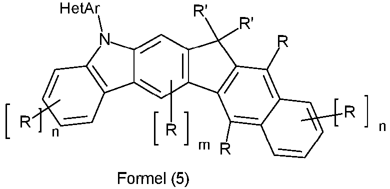 wobei die Symbole und Indizes die in Anspruch 1 genannten Bedeutungen aufweisen.

3. Organische Elektrolumineszenzvorrichtung nach Anspruch 1 oder 2, **dadurch gekennzeichnet, dass** die Verbindung der Formel (1) ausgewählt ist aus den Verbindungen der Formeln (3a-1), (3a-2), (4a-1), (4a-2), (5a-1) und (5a-2), 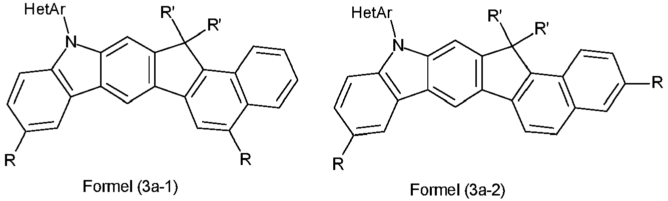 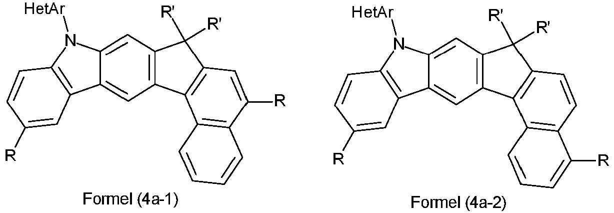 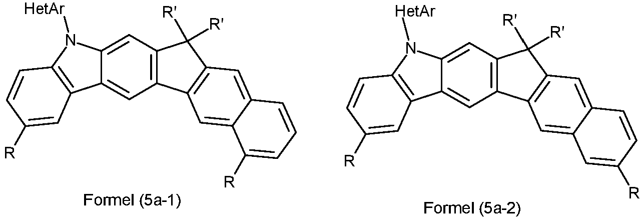 wobei HetAr, R und R' die in Anspruch 1 genannten Bedeutungen aufweisen.

4. Organische Elektrolumineszenzvorrichtung nach einem oder mehreren der Ansprüche 1 bis 3, **dadurch gekennzeichnet, dass** die Verbindung der Formel (1) ausgewählt ist aus den Verbindungen der Formeln (3b), (4b) und (5b), 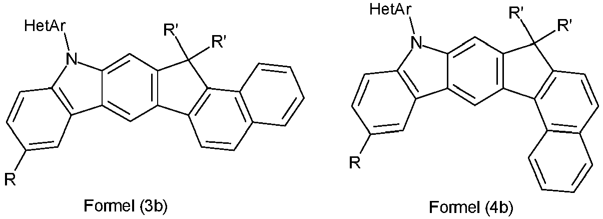 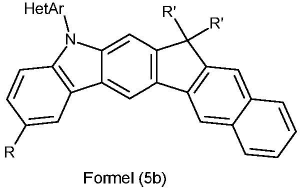 wobei HetAr, R und R' die in Anspruch 1 genannten Bedeutungen aufweisen.

5. Organische Elektrolumineszenzvorrichtung nach einem oder mehreren der Ansprüche 1 bis 4, **dadurch gekennzeichnet, dass** Ar ausgewählt ist aus Phenyl, Biphenyl, insbesondere ortho-, meta- oder para-Biphenyl, Terphenyl, Quaterphenyl, Fluoren, welches über die 1-, 2-, 3- oder 4-Position verknüpft ist, Spirobifluoren, welches über die 1-, 2-, 3- oder 4-Position verknüpft ist, Naphthalin, Indol, Benzofuran, Benzothiophen, Carbazol, welches über die 1-, 2-, 3- oder 4-Position verknüpft ist, Dibenzofuran, welches über die 1-, 2-, 3- oder 4-Position verknüpft sein ist, Dibenzothiophen, welches über die 1-, 2-, 3- oder 4-Position verknüpft ist, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R¹ substituiert sein können, oder eine Kombination aus zwei oder drei dieser Gruppen.

6. Organische Elektrolumineszenzvorrichtung nach einem oder mehreren der Ansprüche 1 bis 5, **dadurch gekennzeichnet, dass** gilt:
R ist gleich oder verschieden bei jedem Auftreten ausgewählt aus der Gruppe bestehend aus H, D, einem aromatischen oder heteroaromatischen Ringsystem mit 6 bis 30 aromatischen Ringatomen, welches mit einem oder mehreren Resten R¹ substituiert sein kann, oder einer Gruppe N(Ar')₂;
R' ist bei jedem Auftreten gleich oder verschieden ausgewählt aus der Gruppe bestehend aus einer geradkettigen Alkylgruppe mit 1 bis 6 C-Atomen oder einer cyclischen Alkylgruppe mit 3 bis 6 C-Atomen, wobei die Alkylgruppe jeweils mit einem oder mehreren Resten R¹ substituiert sein kann, oder einem aromatischen oder heteroaromatischen Ringsystem mit 6 bis 24 aromatischen Ringatomen, das jeweils durch einen oder mehrere Reste R¹ substituiert sein kann; dabei können zwei Reste R' auch miteinander ein Ringsystem bilden, wodurch ein Spirosystem entsteht;
R¹ ist gleich oder verschieden bei jedem Auftreten ausgewählt aus der Gruppe bestehend aus H, D, F, CN, einer geradkettigen Alkylgruppe mit 1 bis 10 C-Atomen oder einer verzweigten oder cyclischen Alkylgruppe mit 3 bis 10 C-Atomen, wobei die Alkylgruppe jeweils mit einem oder mehreren Resten R² substituiert sein kann, oder einem aromatischen oder heteroaromatischen Ringsystem mit 6 bis 24 aromatischen Ringatomen, das jeweils durch einen oder mehrere Reste R² substituiert sein kann;
R² ist gleich oder verschieden bei jedem Auftreten H, eine Alkylgruppe mit 1 bis 4 C-Atomen oder eine Arylgruppe mit 6 bis 10 C-Atomen, welche mit einer Alkylgruppe mit 1 bis 4 C-Atomen substituiert sein kann, bevorzugt aber unsubstituiert ist.

7. Organische Elektrolumineszenzvorrichtung nach einem oder mehreren der Ansprüche 1 bis 6, **dadurch gekennzeichnet, dass** als phosphoreszierende Verbindung eine rot, orange oder gelb phosphoreszierende Verbindung eingesetzt wird.

8. Organische Elektrolumineszenzvorrichtung nach einem oder mehreren der Ansprüche 1 bis 7, **dadurch gekennzeichnet, dass** die emittierende Schicht aus genau einer Verbindung der Formel (1) und einer oder mehreren phosphoreszierenden Verbindungen besteht.

9. Organische Elektrolumineszenzvorrichtung nach einem oder mehreren der Ansprüche 1 bis 7, **dadurch gekennzeichnet, dass** die emittierende Schicht außer der Verbindung der Formel (1) und der phosphoreszierenden Verbindung mindestens ein weiteres Matrixmaterial enthält, welches ausgewählt ist aus der Gruppe bestehend aus Carbazol-derivaten, Biscarbazolderivaten, Indolocarbazolderivaten, Indenocarbazolderivaten, verbrückten Carbazol-Derivaten oder Triarylaminen.

10. Formulierung enthaltend mindestens eine Verbindung gemäß Formel (1) gemäß einem oder mehreren der Ansprüche 1 bis 6, mindestens eine phosphoreszierende Verbindung und mindestens ein Lösemittel.

11. Verwendung einer Formulierung nach Anspruch 10 zur Herstellung einer organischen Elektrolumineszenzvorrichtung.

## Claims

1. Organic electroluminescent device comprising anode, cathode and at least one emitting layer containing at least one phosphorescent compound, **characterized in that** the emitting layer contains at least one compound of formula (1) 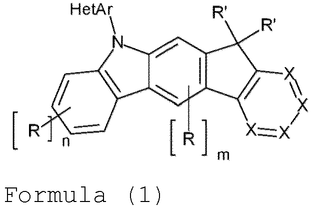 where the symbols and indices used are as follows:
X two adjacent X are a group of the following formula (2), and the two other X are CR, 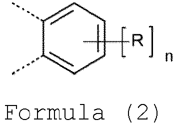
where the two dotted bonds represent the linkage of this group;
HetAr is a group of one of the formulae (HetAr-1d), (HetAr-2a), (HetAr-3a), (HetAr-4a), (HetAr-5a), (HetAr-6a), (HetAr-7a) and (HetAr-8c): 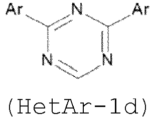 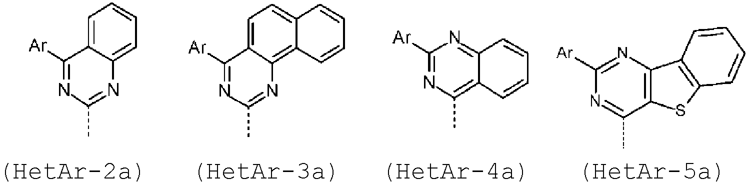 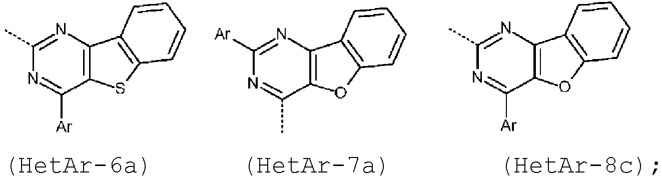
Ar is the same or different at each instance and is an aromatic or heteroaromatic ring system which has 5 to 40 aromatic ring atoms and may be substituted by one or more R¹ radicals;
R is the same or different at each instance and is H, D, F, Cl, Br, I, N(R¹)₂, N(Ar')₂, CN, NO₂, OR¹, SR¹, COOR¹, C(=O)N(R¹)₂, Si(R¹)₃, B(OR¹)₂, C(=O)R¹, P(=O)(R¹)₂, S(=O)R¹, S(=O)₂R¹, OSO₂R¹, a straight-chain alkyl group having 1 to 20 carbon atoms or an alkenyl or alkynyl group having 2 to 20 carbon atoms or a branched or cyclic alkyl group having 3 to 20 carbon atoms, where the alkyl, alkenyl or alkynyl group may in each case be substituted by one or more R¹ radicals and where one or more nonadjacent CH₂ groups may be replaced by Si(R¹)₂, C=O, NR¹, O, S or CONR¹, or an aromatic or heteroaromatic ring system which has 5 to 60 aromatic ring atoms and may be substituted in each case by one or more R¹ radicals;
R' is the same or different at each instance and is a straight-chain alkyl group having 1 to 20 carbon atoms or a branched or cyclic alkyl group having 3 to 20 carbon atoms, where the straight-chain, branched or cyclic alkyl group may in each case be substituted by one or more R¹ radicals and where one or more nonadjacent CH₂ groups may be replaced by O, or an aromatic or heteroaromatic ring system which has 5 to 40 aromatic ring atoms and may be substituted in each case by one or more R¹ radicals; it is also possible here for two R' radicals together to form an aromatic, heteroaromatic, aliphatic or heteroaliphatic ring system;
Ar' is the same or different at each instance and is an aromatic or heteroaromatic ring system which has 5 to 40 aromatic ring atoms and may be substituted by one or more R¹ radicals;
R¹ is the same or different at each instance and is H, D, F, Cl, Br, I, N (R²)₂, CN, NO₂, OR², SR², Si (R²)₃, B (OR²)₂, C(=O)R², P(=O)(R²)₂, S(=O)R², S(=O)₂R², OSO₂R², a straight-chain alkyl group having 1 to 20 carbon atoms or an alkenyl or alkynyl group having 2 to 20 carbon atoms or a branched or cyclic alkyl group having 3 to 20 carbon atoms, where the alkyl, alkenyl or alkynyl group may in each case be substituted by one or more R² radicals, where one or more nonadjacent CH₂ groups may be replaced by Si(R²)₂, C=O, NR², O, S or CONR², or an aromatic or heteroaromatic ring system which has 5 to 40 aromatic ring atoms and may be substituted in each case by one or more R² radicals;
R² is the same or different at each instance and is H, D, F or an aliphatic, aromatic or heteroaromatic organic radical, especially a hydrocarbyl radical, having 1 to 20 carbon atoms, in which it is also possible for one or more hydrogen atoms to be replaced by F;
m is 0, 1 or 2;
n is the same or different at each instance and is 0 or 1.

2. Organic electroluminescent device according to Claim 1, **characterized in that** the compound of the formula (1) is selected from the compounds of the formulae (3), (4) and (5): 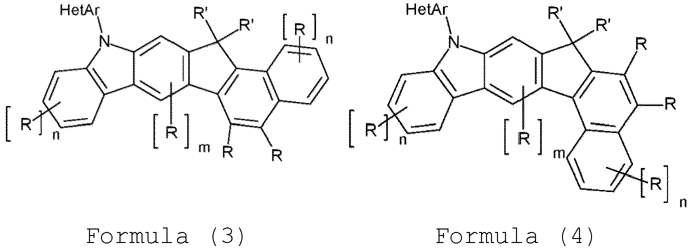 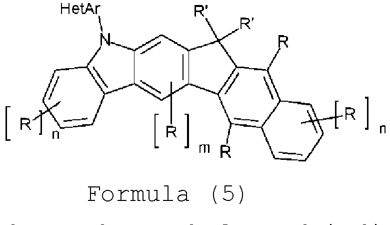 where the symbols and indices have the definitions given in Claim 1.

3. Organic electroluminescent device according to Claim 1 or 2, **characterized in that** the compound of the formula (1) is selected from the compounds of the formulae (3a-1), (3a-2), (4a-1), (4a-2), (5a-1) and (5a-2): 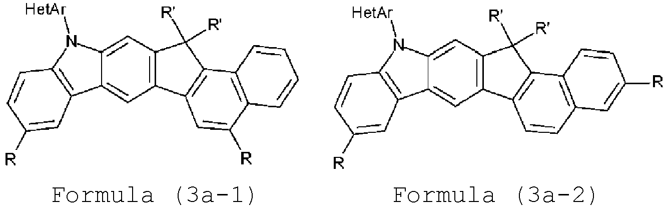 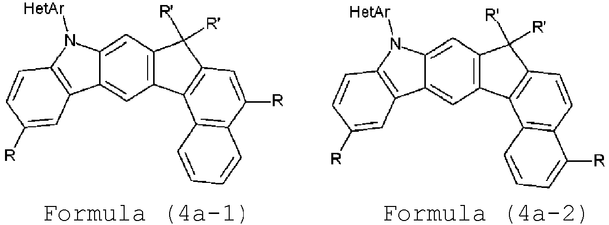 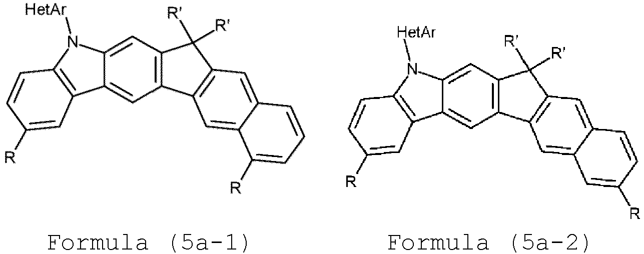 where HetAr, R and R' have the meanings given in Claim 1.

4. Organic electroluminescent device according to one or more of Claims 1 to 3, **characterized in that** the compound of the formula (1) is selected from the compounds of the formulae (3b), (4b) and (5b): 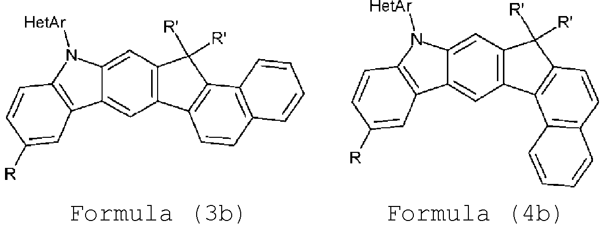 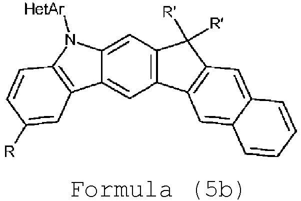 where HetAr, R and R' have the meanings given in Claim 1.

5. Organic electroluminescent device according to one or more of Claims 1 to 4, **characterized in that** Ar is selected from phenyl, biphenyl, especially ortho-, meta- or para-biphenyl, terphenyl, quaterphenyl, fluorene joined via the 1, 2, 3 or 4 position, spirobifluorene joined via the 1, 2, 3 or 4 position, naphthalene, indole, benzofuran, benzothiophene, carbazole joined via the 1, 2, 3 or 4 position, dibenzofuran joined via the 1, 2, 3 or 4 position, dibenzothiophene joined via the 1, 2, 3 or 4 position, indenocarbazole, indolocarbazole, pyridine, pyrimidine, pyrazine, pyridazine, triazine, quinoline, isoquinoline, quinazoline, quinoxaline, phenanthrene or triphenylene, each of which may be substituted by one or more R¹ radicals, or a combination of two or three of these groups.

6. Organic electroluminescent device according to one or more of Claims 1 to 5, **characterized in that**:
R is the same or different at each instance and is selected from the group consisting of H, D, an aromatic or heteroaromatic ring system which has 6 to 30 aromatic ring atoms and may be substituted by one or more R¹ radicals, or an N(Ar')₂ group;
R' is the same or different at each instance and is selected from the group consisting of a straight-chain alkyl group having 1 to 6 carbon atoms or a cyclic alkyl group having 3 to 6 carbon atoms, where the alkyl group may be substituted in each case by one or more R¹ radicals, or an aromatic or heteroaromatic ring system which has 6 to 24 aromatic ring atoms and may be substituted in each case by one or more R¹ radicals; it is also possible here for two R' radicals together to form a ring system, giving rise to a spiro system;
R¹ is the same or different at each instance and is selected from the group consisting of H, D, F, CN, a straight-chain alkyl group having 1 to 10 carbon atoms or a branched or cyclic alkyl group having 3 to 10 carbon atoms, where the alkyl group may be substituted in each case by one or more R² radicals, or an aromatic or heteroaromatic ring system which has 6 to 24 aromatic ring atoms and may be substituted in each case by one or more R² radicals;
R² is the same or different at each instance and is H, an alkyl group having 1 to 4 carbon atoms or an aryl group which has 6 to 10 carbon atoms and may be substituted by an alkyl group having 1 to 4 carbon atoms, but is preferably unsubstituted.

7. Organic electroluminescent device according to one or more of Claims 1 to 6, **characterized in that** the phosphorescent compound used is a red-, orange- or yellow-phosphorescing compound.

8. Organic electroluminescent device according to one or more of Claims 1 to 7, **characterized in that** the emitting layer consists of exactly one compound of the formula (1) and one or more phosphorescent compounds.

9. Organic electroluminescent device according to one or more of Claims 1 to 7, **characterized in that** the emitting layer, apart from the compound of the formula (1) and the phosphorescent compound, contains at least one further matrix material selected from the group consisting of carbazole derivatives, biscarbazole derivatives, indolocarbazole derivatives, indenocarbazole derivatives, bridged carbazole derivatives or triarylamines.

10. Formulation comprising at least one compound of formula (1) according to one or more of Claims 1 to 6, at least one phosphorescent compound and at least one solvent.

11. Use of a formulation according to Claim 10 for production of an organic electroluminescent device.

## Revendications

1. Dispositif électroluminescent organique contenant une anode, une cathode et au moins une couche émettrice, qui contient au moins un composé phosphorescent, **caractérisé en ce que** la couche émettrice contient au moins un composé selon la formule (1), 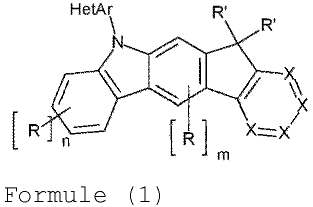 pour les symboles et indices utilisés, ce qui suit étant d'application :
X deux X voisins représentent un groupe de formule (2) suivante, et les deux autres X représentent CR, 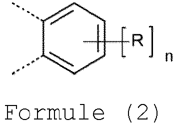
les deux liaisons en pointillés représentant le lien de ce groupe ;
HetAr est un groupe selon l'une des formules (HetAr-1d), (HetAr-2a), (HetAr-3a), (HetAr-4a), (HetAr-5a), (HetAr-6a), (HetAr-7a) et (HetAr-8c), 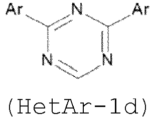 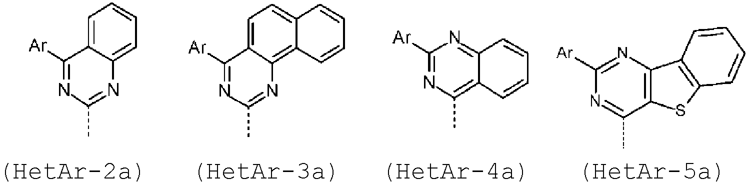 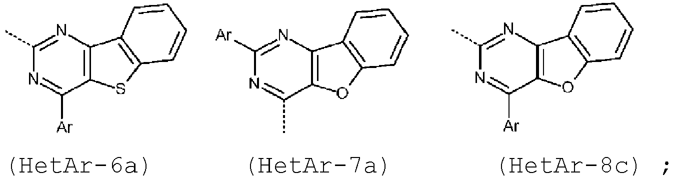
Ar représente, de manière identique ou différente, en chaque occurrence, un système cyclique aromatique ou hétéroaromatique comprenant 5 à 40 atomes de cycle aromatique, qui peut être substitué par un ou plusieurs radicaux R¹ ;
R représente, en chaque occurrence, de manière identique ou différente, H, D, F, Cl, Br, I, N(R¹)₂, N(Ar')₂, CN, NO₂, OR¹, SR¹, COOR¹, C(=O)N(R¹)₂, Si(R¹)₃, B(OR¹)₂, C(=O)R¹, P(=O)(R¹)₂, S(=O)R¹, S(=O)₂R¹, OSO₂R¹, un groupe alkyle linéaire comprenant 1 à 20 atomes C ou un groupe alcényle ou alcynyle comprenant 2 à 20 atomes C ou un groupe alkyle ramifié ou cyclique comprenant 3 à 20 atomes C, le groupe alkyle, alcényle ou alcynyle pouvant dans chaque cas être substitué par un ou plusieurs radicaux R¹ et un ou plusieurs groupes CH₂ non voisins pouvant être remplacés par Si(R¹)₂, C=O, NR¹, O, S ou CONR¹, ou un système cyclique aromatique ou hétéroaromatique comportant 5 à 60 atomes de cycle aromatique, qui peut dans chaque cas être substitué par un ou plusieurs radicaux R¹ ;
R' représente, en chaque occurrence, de manière identique ou différente, un groupe alkyle linéaire comprenant 1 à 20 atomes C ou un groupe alkyle ramifié ou cyclique comprenant 3 à 20 atomes C, le groupe alkyle linéaire, ramifié ou cyclique pouvant dans chaque cas être substitué par un ou plusieurs radicaux R¹ et un ou plusieurs groupes CH₂ non voisins pouvant être remplacés par O, ou un système cyclique aromatique ou hétéroaromatique comprenant 5 à 40 atomes de cycle aromatique, qui peut dans chaque cas être substitué par un ou plusieurs radicaux R¹ ; deux radicaux R' pouvant également former ensemble un système cyclique aromatique, hétéroaromatique, aliphatique ou hétéroaliphatique ;
Ar' représente, en chaque occurrence, de manière identique ou différente, un système cyclique aromatique ou hétéroaromatique comprenant 5 à 40 atomes de cycle aromatique, qui peut être substitué par un ou plusieurs radicaux R¹ ;
R¹ représente, en chaque occurrence, de manière identique ou différente, H, D, F, Cl, Br, I, N(R²)₂, CN, NO₂, OR², SR², Si(R²)₃, B(OR²)₂, C(=O)R², P(=O)(R²)₂, S(=O)R², S(=O)₂R², OSO₂R², un groupe alkyle linéaire comprenant 1 à 20 atomes C ou un groupe alcényle ou alcynyle comprenant 2 à 20 atomes C ou un groupe alkyle ramifié ou cyclique comprenant 3 à 20 atomes C, le groupe alkyle, alcényle ou alcynyle pouvant dans chaque cas être substitué par un ou plusieurs radicaux R², un ou plusieurs groupes CH₂ non voisins pouvant être remplacés par Si(R²)₂, C=O, NR², O, S ou CONR², ou un système cyclique aromatique ou hétéroaromatique comportant 5 à 40 atomes de cycle aromatique, qui peut dans chaque cas être substitué par un ou plusieurs radicaux R² ;
R² représente, en chaque occurrence, de manière identique ou différente, H, D, F ou un radical organique aliphatique, aromatique ou hétéroaromatique, notamment un radical hydrocarboné, comprenant 1 à 20 atomes C, un ou plusieurs atomes H pouvant également être remplacés par F ;
m représente 0, 1 ou 2 ;
n représente, en chaque occurrence, de manière identique ou différente, 0 ou 1.

2. Dispositif électroluminescent organique selon la revendication 1, **caractérisé en ce que** le composé de formule (1) est choisi parmi les composés selon les formules (3), (4) ou (5), 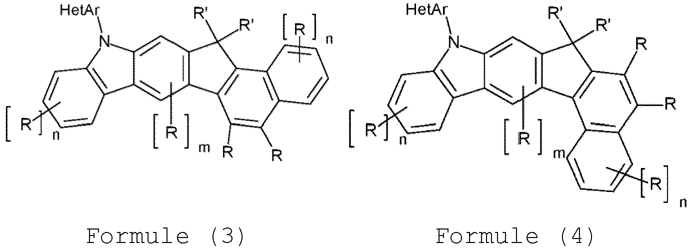 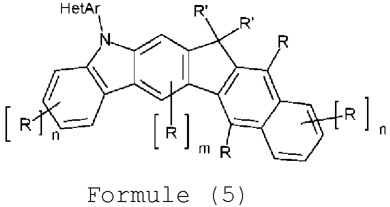 les symboles et les indices présentant les significations données dans la revendication 1.

3. Dispositif électroluminescent organique selon la revendication 1 ou 2, **caractérisé en ce que** le composé de formule (1) est choisi parmi les composés de formules (3a-1), (3a-2), (4a-1), (4a-2), (5a-1) et (5a-2), 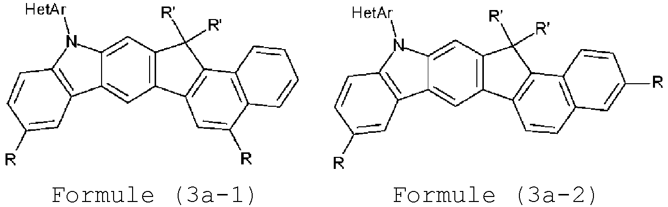 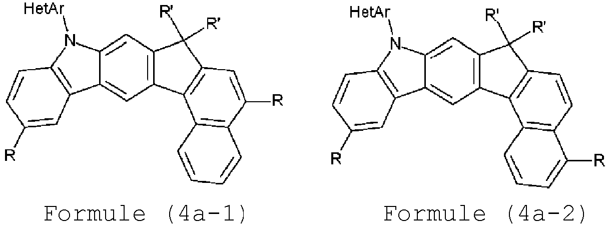 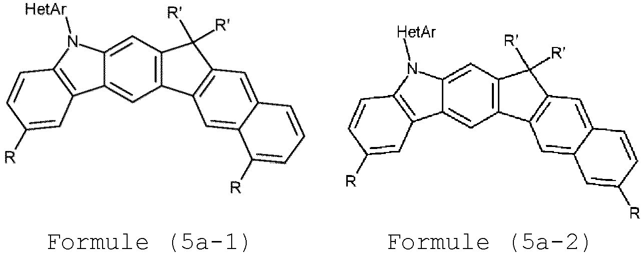 HetAr, R et R' présentant les significations données dans la revendication 1.

4. Dispositif électroluminescent organique selon une ou plusieurs des revendications 1 à 3, **caractérisé en ce que** le composé de formule (1) est choisi parmi les composés de formules (3b), (4b) et (5b), 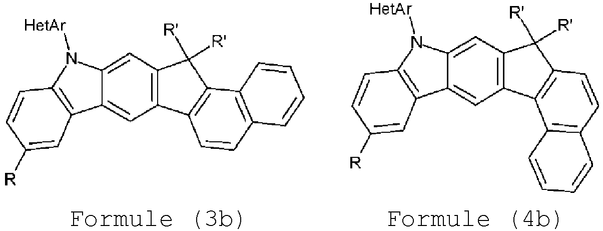 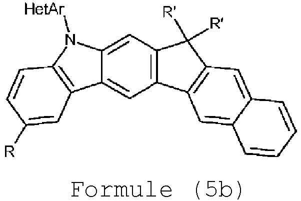 HetAr, R et R' présentant les significations données dans la revendication 1.

5. Dispositif électroluminescent organique selon une ou plusieurs des revendications 1 à 4, **caractérisé en ce qu'**Ar est choisi parmi phényle, biphényle, notamment ortho-, méta- ou para-biphényle, terphényle, quaterphényle, fluorène qui est lié par la position 1, 2, 3 ou 4, spirobifluorène qui est lié par la position 1, 2, 3 ou 4, naphtalène, indole, benzofurane, benzothiophène, carbazole qui est lié par la position 1, 2, 3 ou 4, dibenzofurane qui est lié par la position 1, 2, 3 ou 4, dibenzothiophène qui est lié par la position 1, 2, 3 ou 4, indénocarbazole, indolocarbazole, pyridine, pyrimidine, pyrazine, pyridazine, triazine, quinoléine, isoquinoléine, quinazoline, quinoxaline, phénanthrène ou triphénylène, qui peuvent dans chaque cas être substitué par un ou plusieurs radicaux R¹, ou une combinaison de deux ou trois de ces groupes.

6. Dispositif électroluminescent organique selon une ou plusieurs des revendications 1 à 5, **caractérisé en ce que** ce qui suit est d'application :
R est choisi, de manière identique ou différente, en chaque occurrence, dans le groupe constitué par H, D, un système cyclique aromatique ou hétéroaromatique comprenant 6 à 30 atomes de cycle aromatique, qui peut être substitué par un ou plusieurs radicaux R¹, ou un groupe N(Ar')₂ ;
R' est choisi, en chaque occurrence, de manière identique ou différente, dans le groupe constitué par un groupe alkyle linéaire comprenant 1 à 6 atomes C ou un groupe alkyle cyclique comprenant 3 à 6 atomes C, le groupe alkyle pouvant dans chaque cas être substitué par un ou plusieurs radicaux R¹, ou un système cyclique aromatique ou hétéroaromatique comprenant 6 à 24 atomes de cycle aromatique, qui peut dans chaque cas être substitué par un ou plusieurs radicaux R¹ ; deux radicaux R' pouvant également former ensemble un système cyclique, ce qui donne un système spiro ;
R¹ est choisi, de manière identique ou différente, en chaque occurrence, dans le groupe constitué par H, D, F, CN, un groupe alkyle linéaire comprenant 1 à 10 atomes C ou un groupe alkyle ramifié ou cyclique comprenant 3 à 10 atomes C, le groupe alkyle pouvant dans chaque cas être substitué par un ou plusieurs radicaux R², ou un système cyclique aromatique ou hétéroaromatique comprenant 6 à 24 atomes de cycle aromatique, qui peut dans chaque cas être substitué par un ou plusieurs radicaux R² ;
R² représente, de manière identique ou différente, en chaque occurrence, H, un groupe alkyle comprenant 1 à 4 atomes C ou un groupe aryle comprenant 6 à 10 atomes C, qui peut être substitué par un groupe alkyle comprenant 1 à 4 atomes C, mais qui est de préférence non substitué.

7. Dispositif électroluminescent organique selon une ou plusieurs des revendications 1 à 6, **caractérisé en ce qu'**un composé phosphorescent rouge, orange ou jaune est utilisé en tant que composé phosphorescent.

8. Dispositif électroluminescent organique selon une ou plusieurs des revendications 1 à 7, **caractérisé en ce que** la couche émettrice est constituée d'exactement un composé de formule (1) et d'un ou plusieurs composés phosphorescents.

9. Dispositif électroluminescent organique selon une ou plusieurs des revendications 1 à 7, **caractérisé en ce que** la couche émettrice contient, outre le composé de formule (1) et le composé phosphorescent, au moins un autre matériau de matrice choisi dans le groupe constitué par les dérivés de carbazole, les dérivés de biscarbazole, les dérivés d'indolocarbazole, les dérivés d'indénocarbazole, les dérivés de carbazole pontés ou les triarylamines.

10. Formulation contenant au moins un composé de formule (1) selon une ou plusieurs des revendications 1 à 6, au moins un composé phosphorescent et au moins un solvant.

11. Utilisation d'une formulation selon la revendication 10 pour la fabrication d'un dispositif électroluminescent organique.
